# EUROPEAN PATENT APPLICATION

(11) **EP 4 732 851 A1**
(43) Date of publication of application: **29.04.2026**
(21) Application number: 25781875.7
(22) Date of filing: 01.04.2025
(51) Int. Cl.: A61K 47/68, A61K 39/395, A61K 31/4184, C07K 16/28, A61P 35/00

(54) **LINKER, ANTIBODY-DRUG CONJUGATE, AND PREPARATION METHOD THEREFOR**

(30) Priority: 03.04.2024 CN 202410403435; 27.09.2024 CN 202411363352
(71) Applicant: Fudan University, Shanghai 200433 (CN)
(72) Inventor: YU, Ke, Shanghai 200433 (CN); WANG, Yue, Shanghai 200433 (CN); LIU, Liang, Shanghai 200433 (CN)
(74) Representative: Mewburn Ellis LLP
(86) International application number: PCT/CN2025/086645
(87) International publication number: WO 2025/209482

(57) **Abstract**

The present invention provides a linker, an antibody-drug conjugate, and a preparation method therefor. The drug linker of the present invention has good solubility, and can greatly improve the water solubility of existing small molecule compounds and improve the stability and uniformity of the conjugate after being conjugated to an antibody.

## Description

### Technical Field

This application relates to the field of biomedicine, specifically to a linker, antibody-drug conjugate, and preparation method therefor.

### Background

Antibody-drug conjugate (ADC) utilizes the ability of monoclonal antibodies to specifically recognize specific antigens on the surface of tumor cells, thereby enabling the precise delivery and release of antitumor agents (such as small molecule chemotherapeutic drugs) to target tumor cells, achieving the goal of precise killing of tumors. ADC is also considered the most promising anti-tumor drug due to its appropriate molecular weight, high stability, strong targeting ability, and low toxicity. However, there are also many issues that must be considered and addressed in the successful development of ADCs, such as the need for antibodies to specifically recognize diseased sites, low immunogenicity, and undergo efficient and rapid internalization; the antibody-drug linker should have high stability in blood and the ability to be specifically activated to efficiently release small molecule drugs in targeted cells; and the conjugated small molecule drugs should have strong cell killing ability. Nanobody-drug conjugate (NDC) developed using nanobodies not only retains the advantages of traditional ADCs, but also has high vascular permeability, good blood-brain barrier penetration, strong tumor penetration, and fast reaching of target cells, which can improve the accumulation of drugs in tumors and moderately control the plasma exposure and half-life of drugs, improving the therapeutic effect and overall treatment window of solid tumors, and is expected to become the most promising new class of anti-tumor drugs.

When traditional linkers are used, ADCs may exhibit instability and aggregation. In addition, high lipophilicity can lead to rapid metabolic elimination in metabolic organs such as the liver, resulting in poor PK properties. Therefore, it is necessary to explore linker structures that can ensure high drug loading while optimizing the physicochemical and pharmacokinetic (PK) properties of ADC.

Therefore, there is still a need in this field to develop novel linkers, antibody-drug conjugates, and preparation methods therefor to further advance ADC drugs with improved therapeutic effects.

### Summary of Invention

The purpose of the present invention is to provide a novel linker, antibody-drug conjugate, and preparation method therefor.

In the first aspect of the present invention, a compound, or stereoisomer thereof, or pharmaceutically acceptable salt thereof is provided, and the compound has a structure as shown in Formula A: 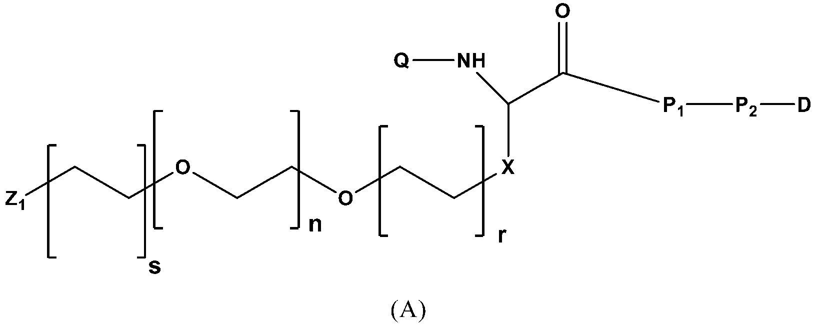 wherein,
Q is a linker group for connecting to an antibody;
Z1 comprises a glucosyl group containing Y hydroxyl groups;
s is an integer from 0 to 10;
n is an integer from 1 to 24;
r is an integer from 0 to 10;
X is a linking group;
P1 is a polypeptide residue;
P2 is a chemical bond or AA-PAB structure; wherein AA is a dipeptide, tripeptide, or tetrapeptide fragment (i.e. a fragment formed by 2-4 amino acids linked through peptide bonds), and PAB is p-aminobenzylcarbamoyl;
D is a drug.

In another preferred embodiment, the glucosyl group comprises a cyclic structure and an open-chain structure.

In another preferred embodiment, the glucosyl group comprises precursor or derivative thereof.

In another preferred embodiment, in Z1, Y is a positive integer.

In another preferred embodiment, Y is 4-50, preferably 5-30, and more preferably 6-25.

In another preferred embodiment, in Z1, Y≥5, preferably ≥ 8.

In another preferred embodiment, Z1 further comprises an amino group.

In another preferred embodiment, the structure of Z1 is selected from the group consisting of:

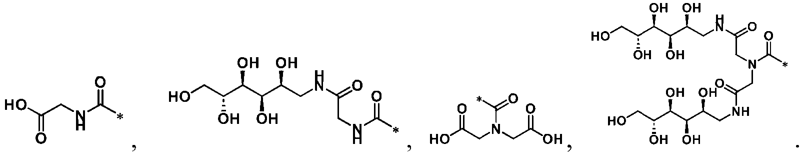

In another preferred embodiment, the structure of Z1 is selected from the group consisting of:

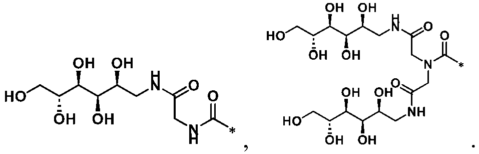

In another preferred embodiment, the linker group Q is selected from:

| | | |
|---|---|---|
| 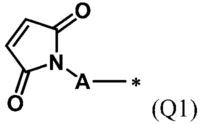 | 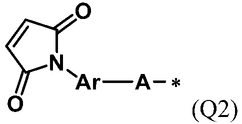 | 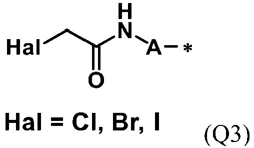 |
| 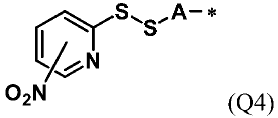 wherein NO₂ is optionally substituted | 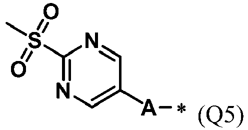 | 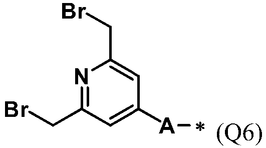 |
| 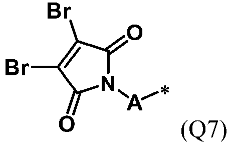 | 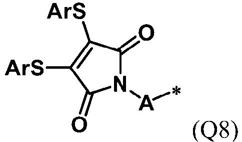 | 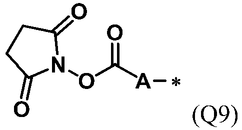 |

wherein A represents an optionally substituted C3-C8 alkylene, C3-C8 alkene, C3-C8 alkynyl, C3-C6 cycloalkene, C3-C8 cycloalkyl, and optionally substituted diglycolyl to octaglycolyl group; Ar represents an optionally substituted C5-6 aryl or heteroaryl group; and "*" represents -C=O- forming an amide bond with an amino group.

In another preferred embodiment, the optional substitution refers to the substitution at any available connecting site on the aryl group.

In another preferred embodiment, P1 is selected from the group consisting of:
^{NH}-Val-Cit-^{C=O}, ^{NH}-Val-Ala-^{C=O}, ^{NH}-Ala-Ala-Ala-^{C=O}, ^{NH}-Ala-Ala-^{C=O}, ^{NH}-Gly-Gly-Phe-Gly-^{C=O}, ^{NH}-Val-Lys-^{C=O}.

In another preferred embodiment, X is selected from

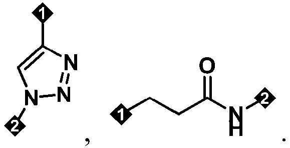

In another preferred embodiment, in X, 1 and 2 represent connection sites respectively; for example, 1 is the site connecting to the upper part of Formula A, and 2 is the site connecting to the lower part of Formula A.

In another preferred embodiment, D is a cytotoxic small molecule drug selected from the group consisting of: a STING agonist, KRAS-G12D inhibitor, tubulin inhibitor, topoisomerase inhibitor, and DNA binding agent.

In another preferred embodiment, the STING agonist is selected from the group consisting of: a diABZI analog.

In another preferred embodiment, the KRAS-G12D inhibitor is selected from a MRTX1133 analog.

In another preferred embodiment, the tubulin inhibitor is selected from the group consisting of: a maytansine derivative, Monomethyl Auristatin E (MMAE), Monomethyl Auristatin F (MMAF), Monomethyl Dolastatin 10 (MMAD), Tubulysin derivative, Cryptophycin derivative, Taltobulin; preferably MMAE or MMAF.

In another preferred embodiment, the topoisomerase inhibitor is selected from the group consisting of: SN38, DXd, Doxorubicin metabolite PNU-159682 derivative, Exatecan (DX8951), irinotecan (CPT-11) metabolite SN38 derivative; preferably, a topoisomerase 1 (Topo1) inhibitor such as SN38, DXd, or Exatecan.

In another preferred embodiment, the DNA binding agent is selected from the group consisting of: a PBD derivative and duocarmycin derivative.

In another preferred embodiment, the structure of Formula A is selected from the group consisting of:

| | |
|---|---|
| 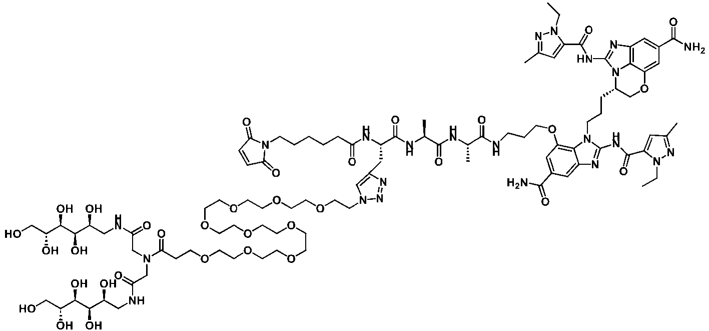 | A1 |
| 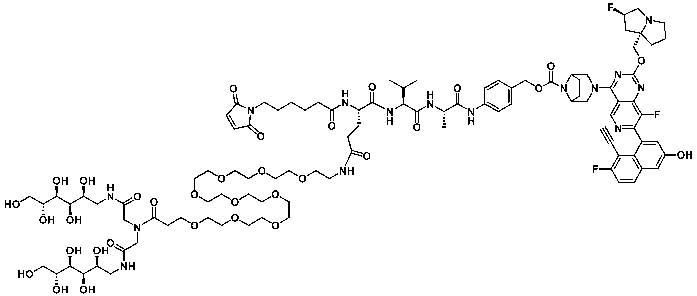 | A2 |
| 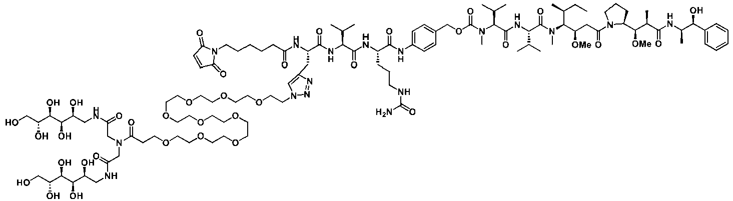 | A3 |
| 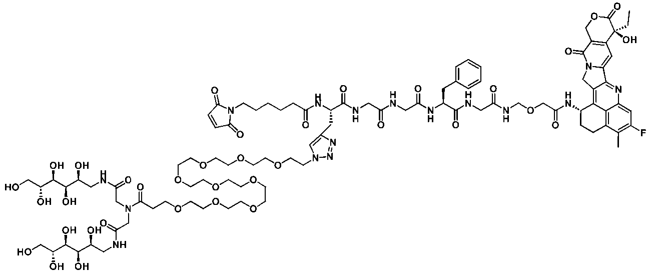 | A4 |
| 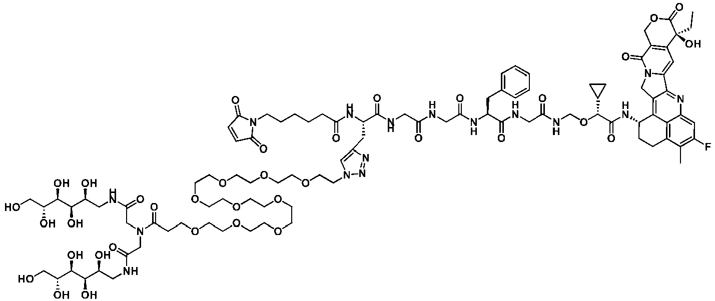 | A5 |
| 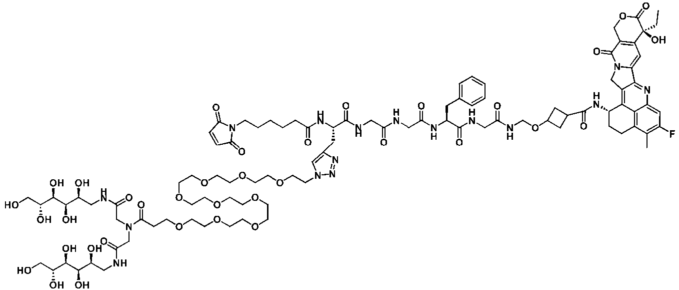 | A6 |
| 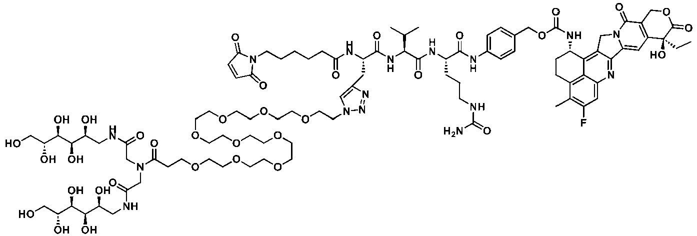 | A7 |
| 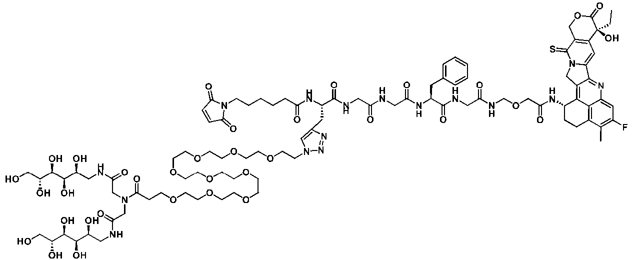 | A8 |
| 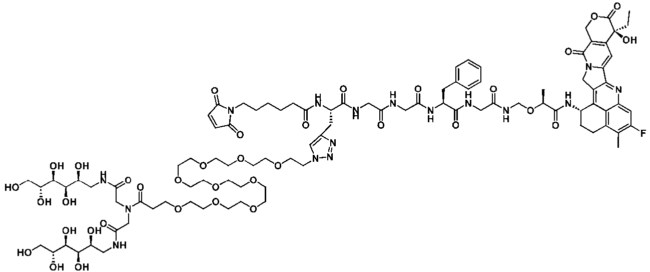 | A9 |
| 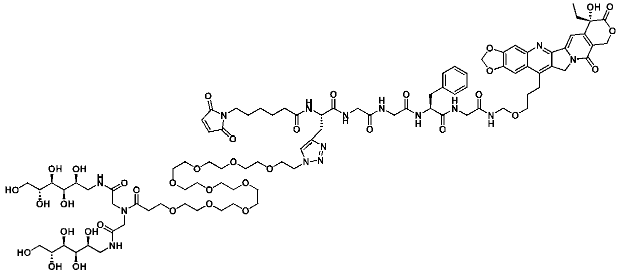 | A10 |

In the second aspect of the present invention, an Antibody-drug conjugate (ADC) is provided, wherein the ADC is formed by conjugating the compound of Formula A of the first aspect of the present invention with an antibody.

In another preferred embodiment, the conjugate is shown as Formula B: 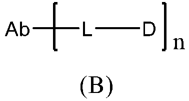 wherein:
Ab is an antibody;
L is a linker;
D is a drug;
N is an integer or decimal from 1 to 10.

In another preferred embodiment, the antibody comprises an antigen binding fragment, a nanobody, a chimeric antibody, a bivalent antibody, and/or a multivalent antibody.

In another preferred embodiment, the antibody is an animal-derived antibody, a humanized antibody, a chimeric antibody, or a chimeric antigen receptor antibody (CAR).

In another preferred embodiment, the CDR region of the humanized antibody comprises 1, 2, or 3 amino acid changes.

In another preferred embodiment, the animal is a non-human mammal, preferably a mouse, sheep, rabbit, or camel.

In another preferred embodiment, the antibody is a double-chain antibody or a single-chain antibody.

In another preferred embodiment, the antibody is a nanobody or a monoclonal antibody.

In another preferred embodiment, the antibody is a partially or fully humanized monoclonal antibody.

In another preferred embodiment, the antibody or nanobody or fusion protein thereof targets a target selected from the group consisting of: TF, EGFR, HER2, HER3, BCMA, B7-H3, CD73, AXL, DLL3, CD38, CD123, CD19, CD20, CD22, B7-H6, GPC3, PMSA, CD28, 4-1BB, OX40, CD40, CD27, CD3, CTLA4, PD1, PDL1, BCMA, Trop2, TIGIT, LAG-3, TLR7, or a combination thereof.

In another preferred embodiment, the antigen binding fragment comprises: (i) Fab fragment; (ii) F (ab') 2 fragment; (iii) Fd fragment; (iv) Fv fragment; (v) single-chain Fv (scFv) molecule; (vi) dAb fragment.

In another preferred embodiment, the antibody is an antibody or nanobody targeting TF and/or HER2 (anti-TF and/or HER2 antibody, anti-TF and/or HER2 nanobody, or a fusion protein thereof).

In another preferred embodiment, the antigen binding fragment targeting TF nanobody has CDR1 as shown in SEQ ID NO. 1, CDR2 as shown in SEQ ID NO. 2, and CDR3 as shown in SEQ ID NO. 3.

In another preferred embodiment, the nanobody targeting TF has a heavy chain variable region as shown in SEQ ID NO. 4.

In another preferred embodiment, the immunoconjugate comprises: a multivalent (e.g. divalent) nanobody targeting TF and/or HER2 or antibody targeting TF and/or HER2.

In another preferred embodiment, the multivalent refers to that the amino acid sequence of the immunoconjugate comprises multiple repeats of the nanobody or antibody targeting TF and/or HER2.

In another preferred embodiment, the antibody-drug conjugate ADC is a monomer, dimer, or polymer.

In another preferred embodiment, the antibody comprises functional domains capable of enhancing the physicochemical properties or druggability of the protein, such as an Fc region, an anti-human serum albumin nanobody (HLE), or an albumin-binding domain (ABD).

In another preferred embodiment, the nanobody targeting TF comprises an Fc segment, preferably the nanobody targeting TF is as shown in SEQ ID NO. 5.

In another preferred embodiment, the nanobody targeting HER2 comprises an Fc segment, preferably the nanobody targeting HER2 is as shown in any one of SEQ ID NO. 7-9.

In another preferred embodiment, the antibody-drug conjugate comprises the structure as shown in Formula (B): 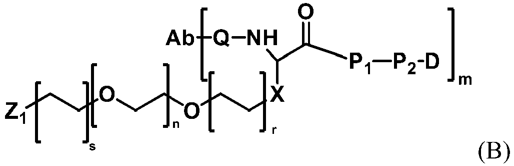 wherein: Q is a linker group the nanobody targeting HER2; Z1 is a hydrophilic group, including a hydroxyl and a glucosyl group containing an amino group; X is a linking group; P1 is a polypeptide residue; P2 is a direct bond or a p-aminobenzyloxycarbonyl (PABC) group; D is an anti-tumor drug; n is an integer from 1 to 24; Ab is an antibody or nanobody fusion protein; m=1-8.

In another preferred embodiment, the antibody-drug conjugate (ADC) is selected from the group consisting of:
The ADC conjugate structures are as follows:

| | |
|---|---|
| 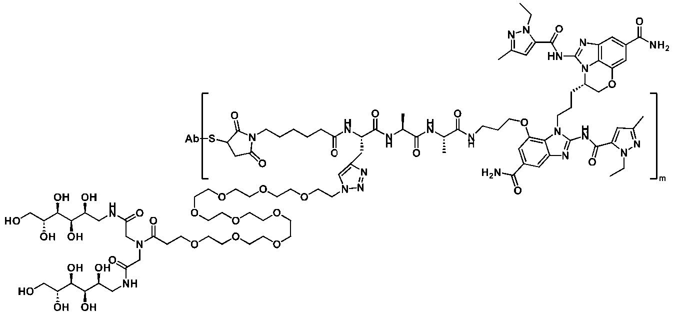 | B1 |
| 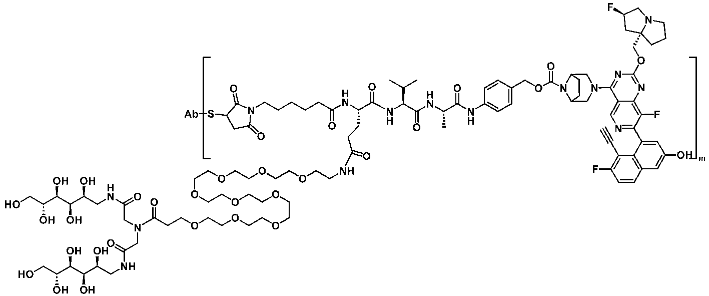 | B2 |
| 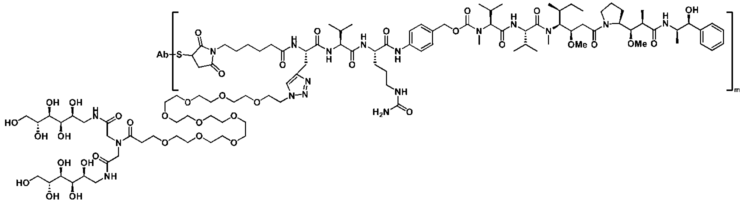 | B3 |
| 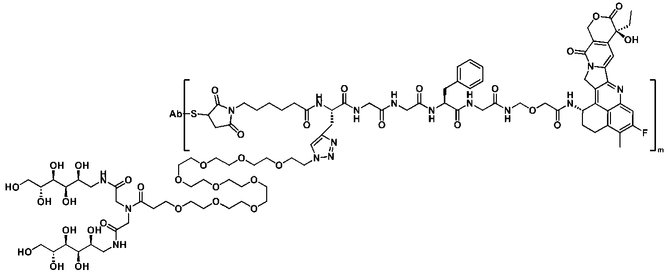 | B4 |
| 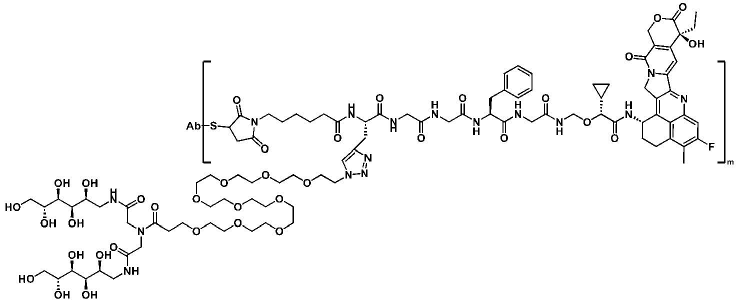 | B5 |
| 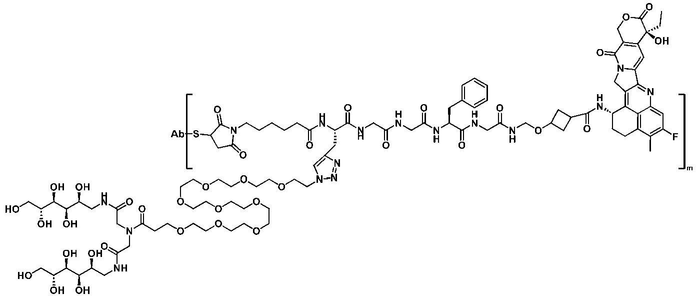 | B6 |
| 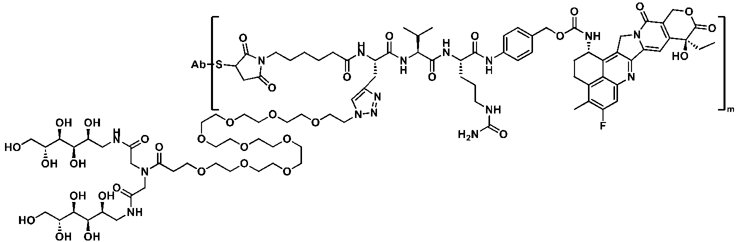 | B7 |
| 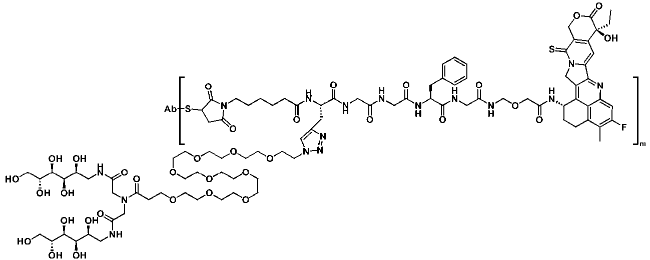 | B8 |
| 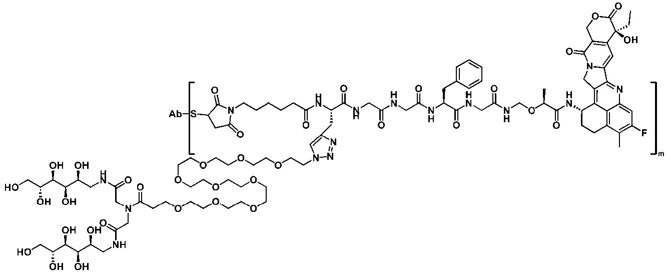 | B9 |
| 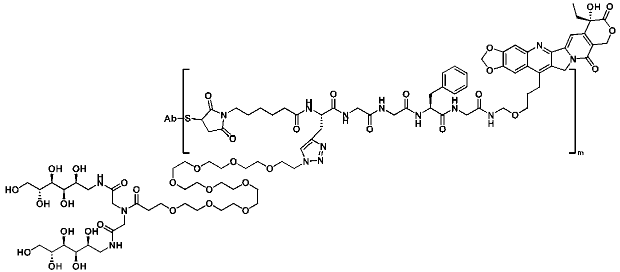 | B10 |

wherein: Ab is a ligand, m=1-8.

In the third aspect of the present invention, a pharmaceutical composition is provided, comprising (a) the antibody-drug conjugate of the second aspect of the present invention or a pharmaceutically acceptable salt thereof, and (b) a pharmaceutically acceptable carrier or excipient.

In the fourth aspect of the present invention, a use of the antibody-drug conjugate f the second aspect of the present invention or a pharmaceutically acceptable salt thereof, or a pharmaceutical composition comprising the conjugate or a pharmaceutically acceptable salt thereof, in the manufacture of a medicament for treating a tumor or cancer is provided.

In another preferred embodiment, the cancer is selected from the group consisting of: lung cancer, liver cancer, breast cancer (triple-negative breast cancer), ovarian cancer, non-Hodgkin lymphoma, Hodgkin lymphoma, acute lymphoblastic leukemia, anaplastic large cell lymphoma, multiple myeloma, prostate cancer, non-small cell lung cancer, small cell lung cancer, malignant melanoma, squamous cell cancer, glioblastoma, renal cell cancer, gastrointestinal cancer, pancreatic cancer, colorectal cancer, gastric cancer, glioma, mesothelioma.

In the fifth aspect of the present invention, a method for preparing the antibody-drug conjugate of the second aspect of the present invention is provided, comprising the steps of:
(1) reacting an antibody with a reducing reagent in a buffer to obtain a reduced antibody;
(2) crosslinking (conjugating) the compound shown in Formula A with the reduced antibody obtained in step (1) in a mixture of buffer and organic solvent to obtain the antibody-drug conjugate B.

It should be understood that within the scope of the present invention, the various technical features of the present invention described above and the various technical features specifically described below (e.g., in the examples) can be combined with each other to form new or preferred technical solutions. Due to space limitations, these are not exhaustively detailed here.

### Brief Description of the Drawings

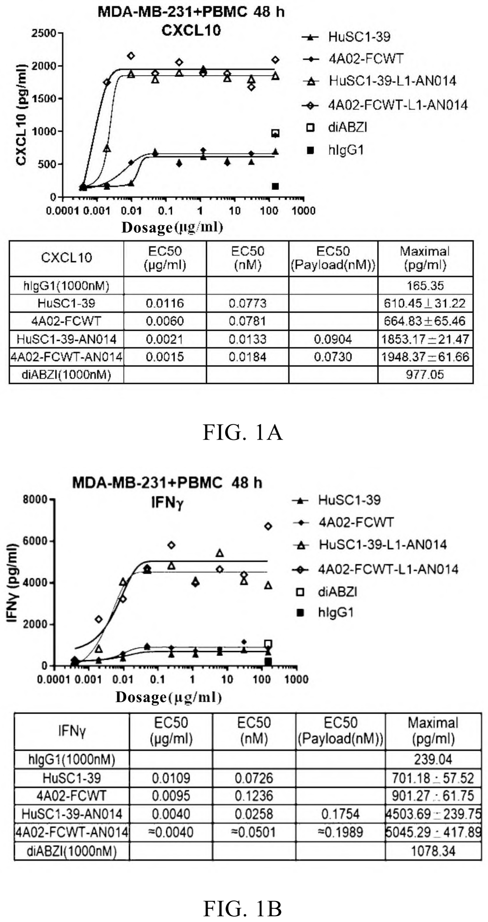

**Figure 1** shows the effect of 4A02-FCWT, 4A02-FCWT-L1-AN014, HuSC1-39, and HuSC1-39-L1-AN014 on inducing cytokines after 48 hours of co-culture with MDA-MB-231/PBMC. 1000 nM diABZI was used as the positive control, and 150 µg/mL hIgG1 was used as the negative control. **Figure 1A** shows the detection results of the induced chemokine CXCL10; **Figure 1B** shows the detection results of the induced inflammatory factor cytokine Interferon-γ.

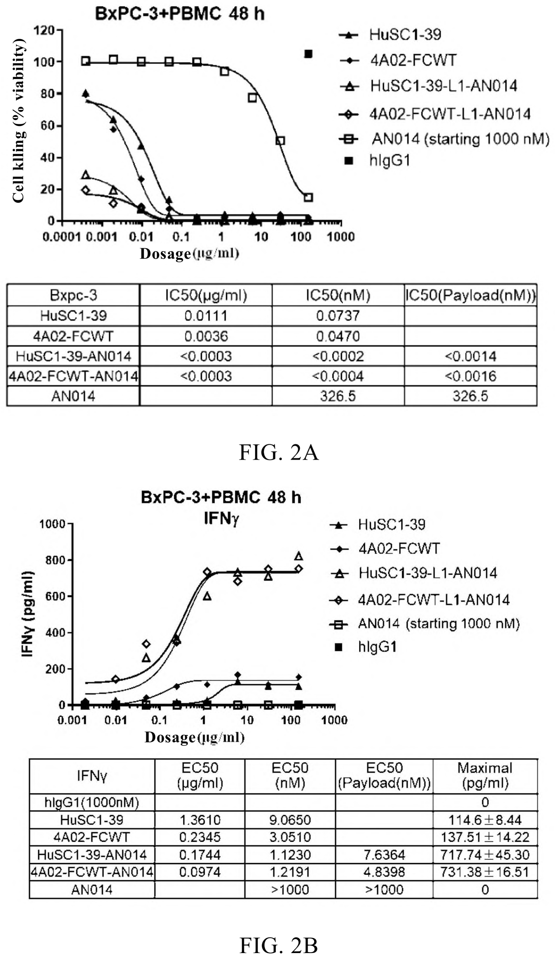

**Figure 2** shows the experimental results of 4A02-FCWT, 4A02-FCWT-L1-AN014, HuSC1-39, and HuSC1-39-L1-AN014 after 48 hours of co-culture with BxPC3-Luc/PBMC. **Figure 2A** shows the survival rate of BxPC3-Luc cells, where the starting concentration of the synchronously diluted small molecule payload AN014 was 1000 nM, and 150 µg/mL hIgG1 was used as a negative control; **Figure 2B** shows the effect of the drugs on promoting the expression and secretion of the inflammatory cytokine Interferon-γ.

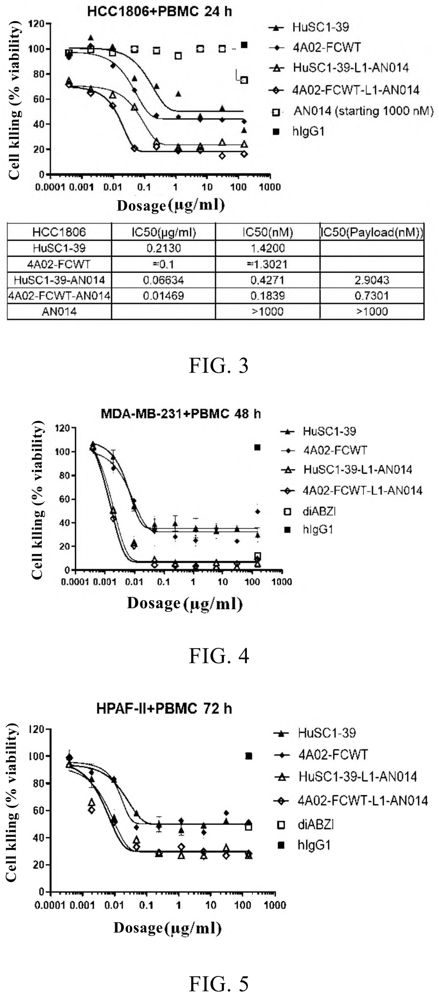

**Figure 3** shows the survival rate of HCC1806-Luc cells after 24 hours of co-culture with 4A02-FCWT, 4A02-FCWT-L1-AN014, HuSC1-39, and HuSC1-39-L1-AN014 with HCC 1806-Luc/PBMC, where the starting concentration of the synchronously diluted small molecule payload AN014 was 1000 nM, and 150 µg/mL hIgG1 was used as a negative control.
**Figure 4** shows the survival rate of MDA-MB-231-Luc cells after 48 hours of co-culture of 4A02-FCWT, 4A02-FCWT-L1-AN014, HuSC1-39, and HuSC1-39-L1-AN014 with MDA-MB-231-Luc/PBMC. The starting concentration of the synchronously diluted small molecule payload AN014 was 1000 nM, and 150 µg/mL hIgG1 was used as a negative control.
**Figure 5** shows the survival rate of HPAF-II-Luc cells detected after 72 hours of co-culture of 4A02-FCWT, 4A02-FCWT-L1-AN014, HuSC-39, HuSC1-39-L1-AN014 with HPAF-II-Luc/PBMC. The starting concentration of the synchronously diluted small molecule payload AN014 was 1000 nM, and 150 µg/mL hIgG1 was used as a negative control.

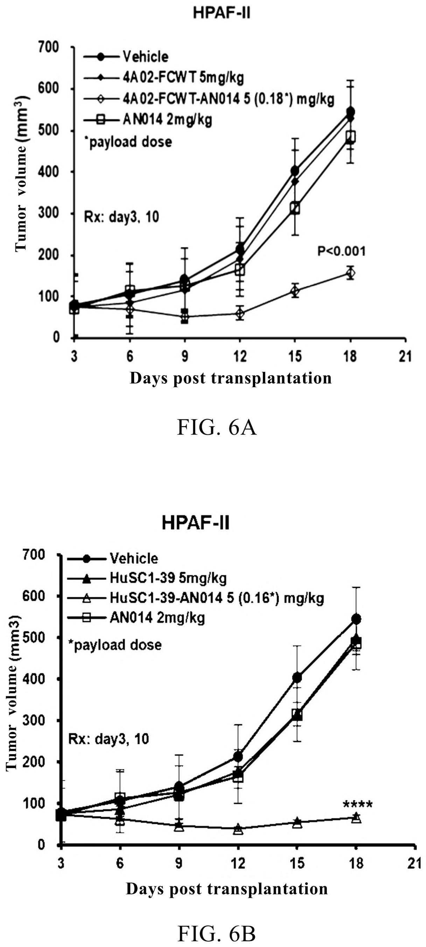

**Figure 6** shows the therapeutic effect of 4A02-FCWT-L1-AN014 **(****Figure 6A****)** and HuSC1-39-L1-AN014 **(****Figure 6B****)** in a pancreatic cancer HPAF-II nude mouse xenograft model. On the third day after cell inoculation, when the tumor volume reached ~100 mm³, tumor-bearing mice were randomly grouped for administration. The doses for 4A02-FCWT-L1-AN014 and HuSC1-39-L1-AN014 were 5 mg/kg, the doses for the naked antibody groups 4A02-FCWT and HuSC1-39 were 5 mg/kg, and the dose for the free small molecule AN014 was 2 mg/kg, administered once weekly for a total of 2 doses.

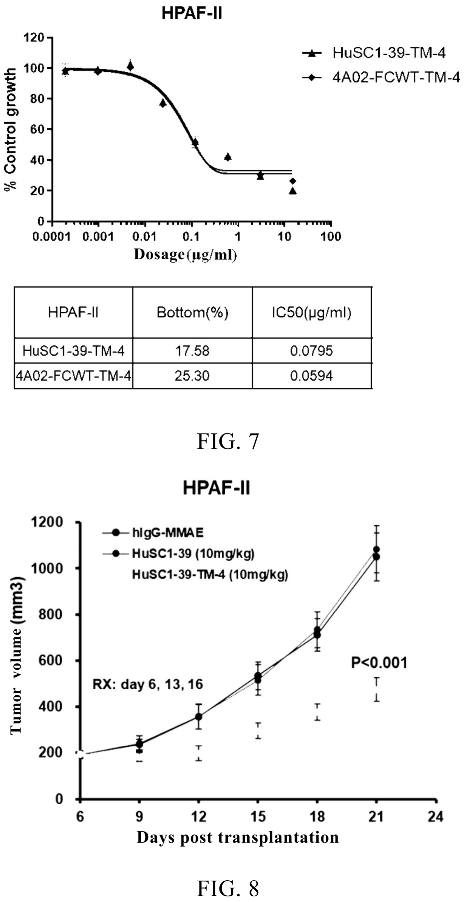

**Figure 7** shows the effect of concentration-gradient diluted 4A02-FCWT-TM-4 and HuSC1-39-TM-4 on the *in vitro* proliferation inhibition of KRAS^{G12D} mutant HPAF-II cells, with the inhibition curve and IC₅₀ value 3 days after drug treatment presented.
**Figure 8** shows the therapeutic effect of TF-KRAS^{G12D}-I ADC HuSC1-39-TM-4 in the HPAF-II nude mouse xenograft tumor model. Tumor-bearing mice were intravenously injected with 10 mg/kg naked antibody HuSC1-39 and HuSC1-39-TM-4 once a week, for a total of 3 administrations.

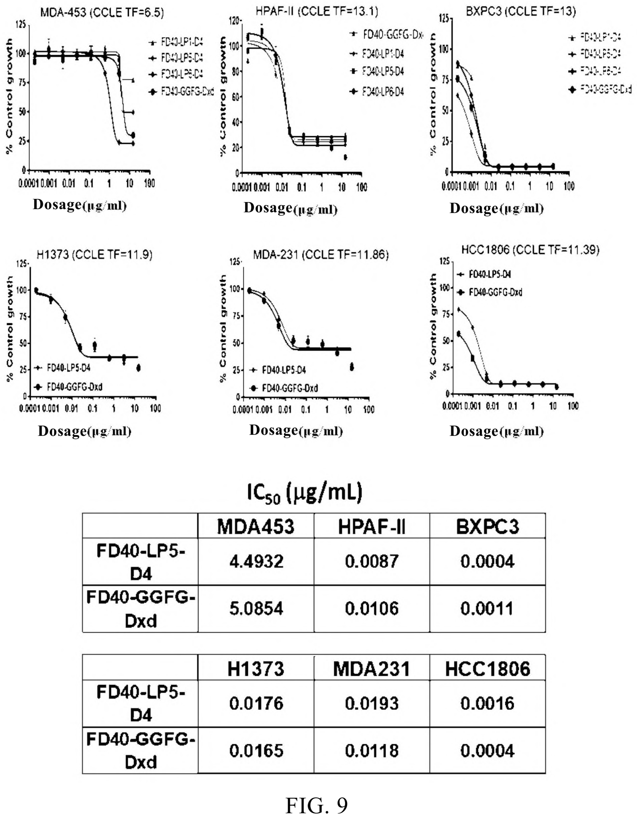

**Figure 9** shows the *in vitro* anti-tumor activity of the humanized antibody 4A02-HM8 (FD40)-Topo1 inhibitor conjugate. The *in vitro* proliferation activity of FD40-LP1-D4, FD40-LP5-D4, and FD40-LP6-D4 against TF-negative breast cancer MDA-453 cells, and TF-high expressing pancreatic cancer HPAF-II, BxPC3, lung cancer NCI-H1373, triple negative breast cancer MDA-231, HCC1806 cells is shown, and the dose efficacy curve and a summary table of IC₅₀ values are shown.
**Figure 10** shows the therapeutic effects of FD40-GFG-Dxd, FD40-LP1-D4, FD40-LP5-D4, and FD40-LP6-D4 on lung cancer NCI-H1373 nude mouse xenograft model. On the 8th day after cell inoculation, when the tumor volume reached ~200 mm³, tumor-bearing mice were randomly grouped for administration. TF-NDC was intravenously injected at 10 mg/kg, once weekly for a total of 2 doses.
**Figure 11** shows the therapeutic effect of FD40-LP1-D4, FD40-LP5-D4 and FD40-LP6-D4 on a pancreatic cancer HPAF-II nude mouse xenograft model. On the 7th day after cell inoculation, when the tumor volume reached ~150 mm³, tumor-bearing mice were randomly grouped for administration. TF-NDC was intravenously injected at 10 mg/kg, for a total of 1 administration.
**Figure 12** shows the therapeutic effect of FD40-GGFG-Dxd (10 mg/kg) and FD40-LP5-D4 (10 mg/kg, 5 mg/kg, 2.5 mg/kg) on a pancreatic cancer HPAF-II nude mouse xenograft model. On the 6th day after cell inoculation, when the tumor volume reached ~200 mm³, tumor-bearing mice were randomly grouped for administration, for a total of 1 administration.
**Figure 13** shows the therapeutic effect of FD40-LP5-D4 (10 mg/kg, 5 mg/kg, 2.5 mg/kg) on a triple-negative breast cancer HCC1806 nude mouse xenograft model. On the 10th day after cell inoculation, when the tumor volume reached ~200 mm³, tumor-bearing mice were randomly grouped for administration, for a total of 1 administration.

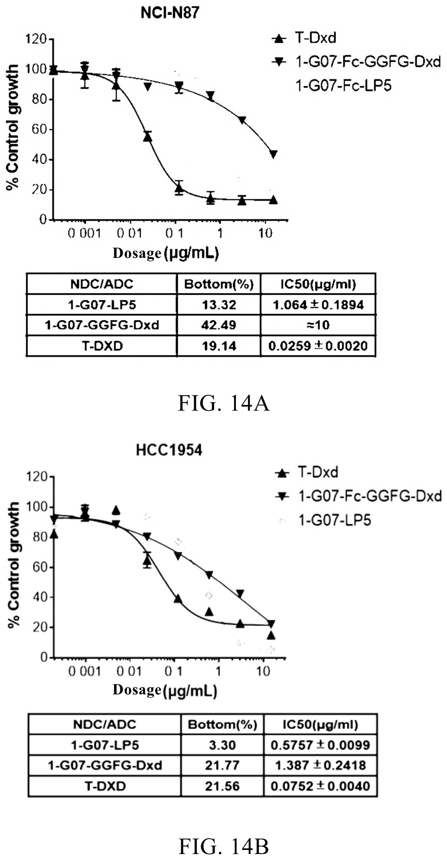

**Figure 14** shows the *in vitro* anti-tumor activity of HER2-NDC 1-G07-LP5, 1-G07-GGFG Dxd, and T-Dxd. **Figure 14A** shows the anti-proliferative activity of the drugs against gastric cancer NCI-N87 cells. **Figure 14B** shows the anti-proliferative activity of drugs against breast cancer HCC1954 cells. The dose efficacy curve and IC₅₀ values are shown.

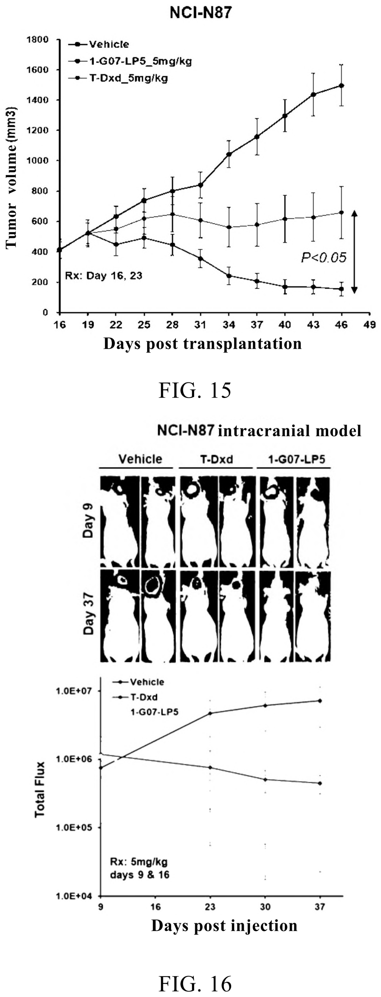

**Figure 15** shows the therapeutic effects of HER2-NDC 1-G07-LP5 and T-Dxd on an NCI-N87 nude mouse xenograft model. On the 16th day after cell inoculation, when the tumor volume reached ~400 mm³, tumor-bearing mice were randomly grouped for administration at a dose of 5 mg/kg, once weekly for a total of 2 doses.
**Figure 16** shows the therapeutic effects of HER2-NDC 1-G07-LP5 and T-Dxd on the NCI-N87-Luc intracranial tumor model. On the 9th day after cell inoculation, tumor growth was monitored by *in vivo* live imaging, and intravenous administration was administered in groups at a dose of 5 mg/kg, once weekly for a total of 2 doses.
**Figure 17** shows the therapeutic effect of TF-NDC FD40-LP5-D4 on HCC1806 Luc intracranial tumor model. On the 7th day after intracranial inoculation of cells, tumor growth was monitored by *in vivo* live imaging, and intravenous administration was administered in groups at a dose of 5 mg/kg, for a total of 1 administration.

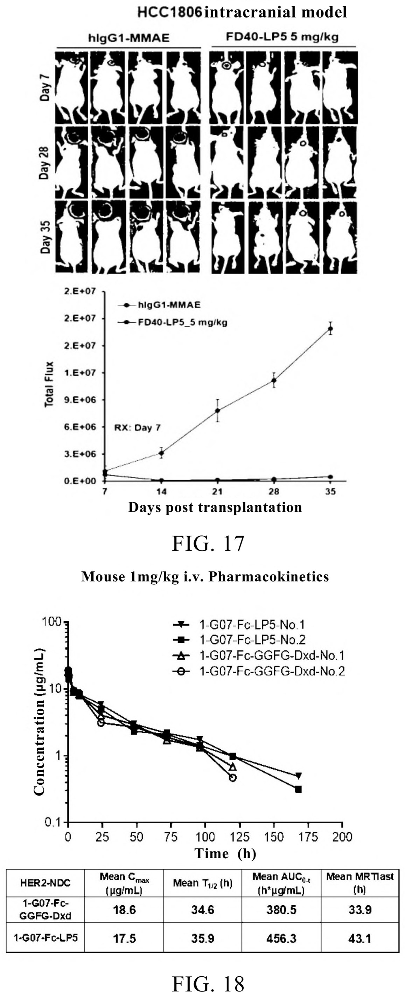

**Figure 18** shows the pharmacokinetic results of HER2-NDC 1-G07-LP5 and 1-G07-GGFG Dxd administered intravenously in mice at a dose of 1 mg/kg.

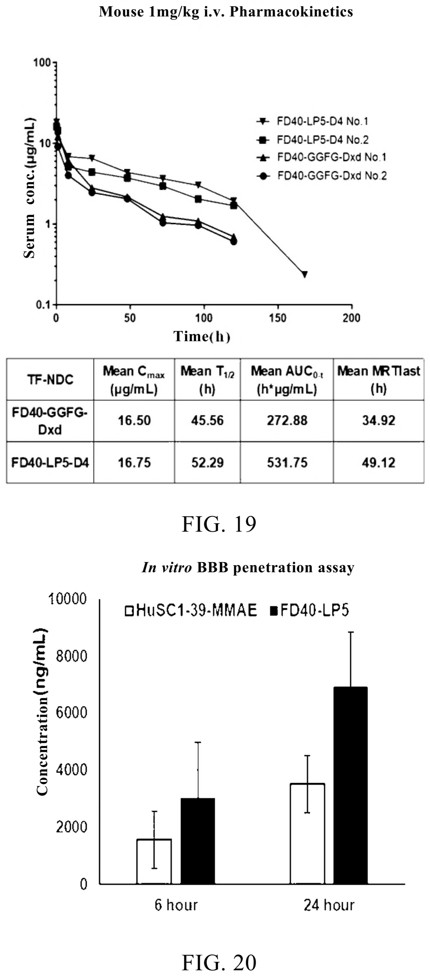

**Figure 19** shows the pharmacokinetic results of TF-NDC FD40-LP5 and FD40-GFG-Dxd administered intravenously in mice at a dose of 1 mg/kg.
**Figure 20** shows the detection results of TF-NDC FD40-LP5 and TF-ADC HuSC1-39-MMAE in an *in vitro* blood-brain barrier penetration model.

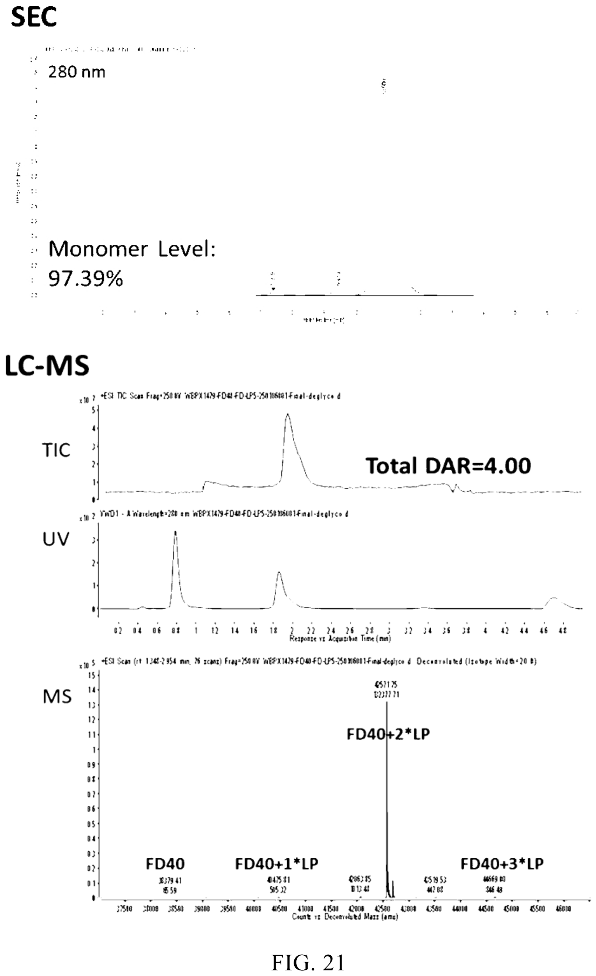

**Figure 21** shows the drug analysis results of gram-scale batch conjugate of FD40-LP5.

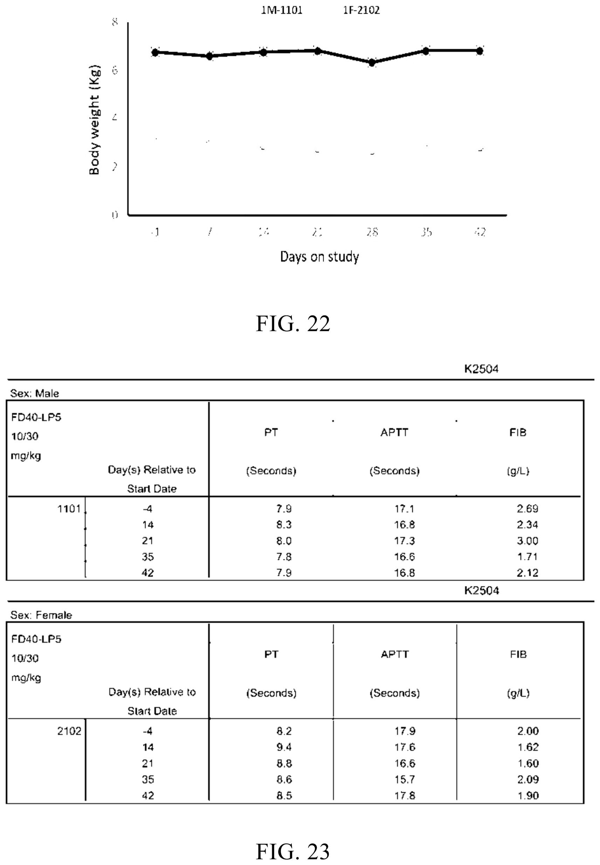

**Figure 22** shows the body weight change results in the exploratory safety assessment study of FD40-LP5 in cynomolgus monkeys.
**Figure 23** shows the blood coagulation index detection results in the exploratory safety assessment study of FD40-LP5 in cynomolgus monkeys.

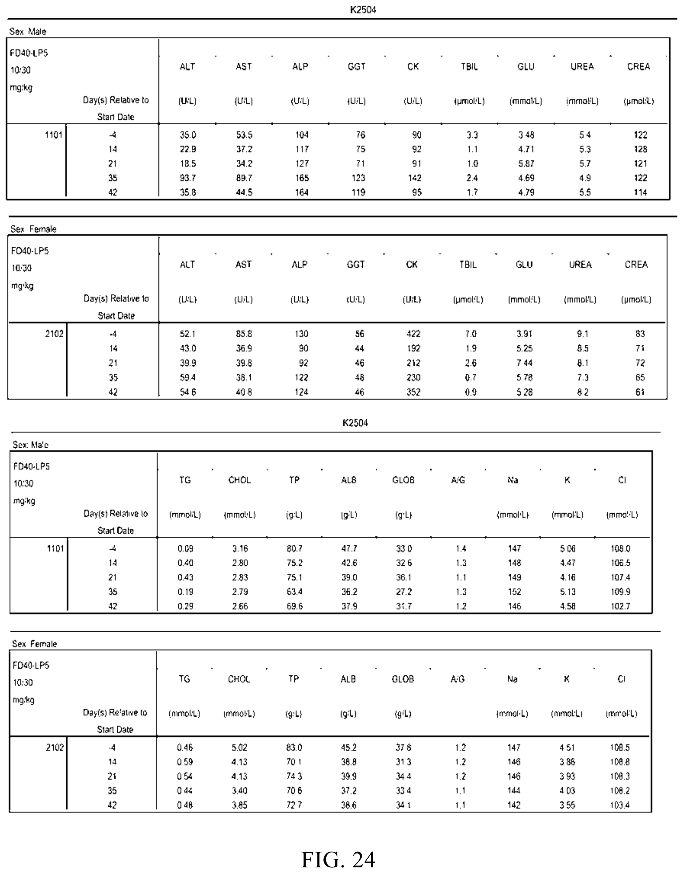

**Figure 24** shows the blood biochemical index detection results in the exploratory safety assessment study of FD40-LP5 in cynomolgus monkeys.

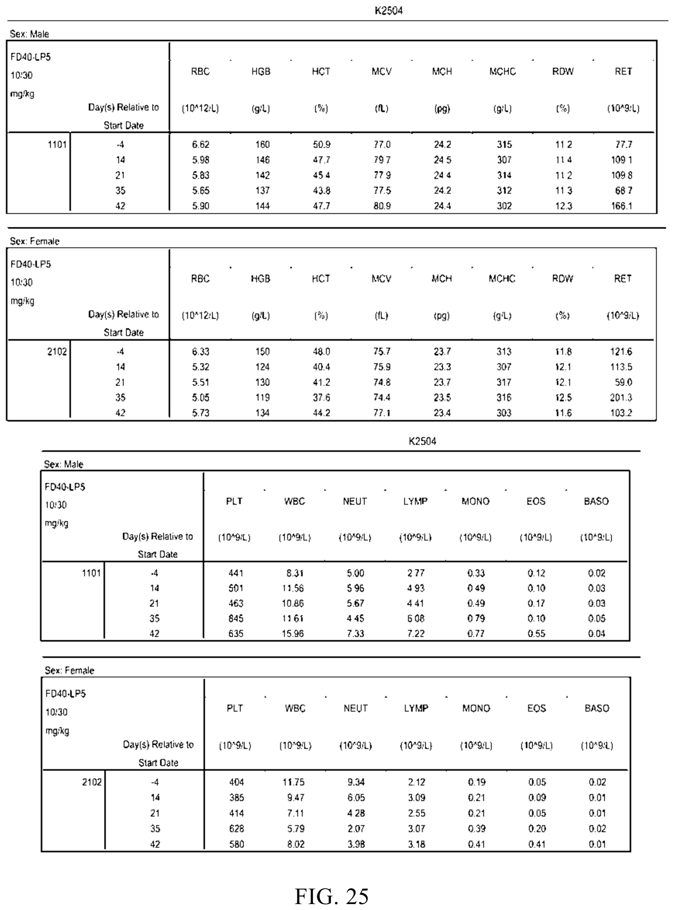

**Figure 25** shows the hematology index detection results in the exploratory safety assessment study of FD40-LP5 in cynomolgus monkeys.

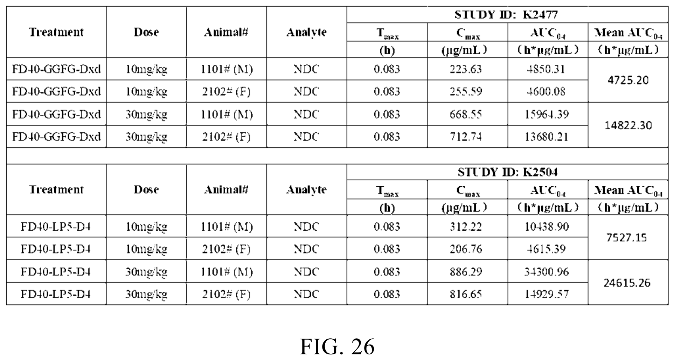

**Figure 26** shows the toxicokinetic (TK) detection results in the exploratory safety assessment study of FD40-GGFG-Dxd and FD40-LP5 in cynomolgus monkeys.

### Detailed Description

After extensive and in-depth research, the inventor has constructed a novel linker for the first time. The antibody-drug conjugate constructed using the linker of the present invention exhibits significant tumor inhibitory effects on cell lines derived from various cancers or tumors, superior to positive controls. This indicates that the antibody-drug conjugate of the present invention can be used as a therapeutic drug for various solid tumors and hematological tumors, used for the treatment of tumors or cancers. The present invention was completed on this basis.

### Terms

Unless otherwise defined, all technical and scientific terms used herein have the same meanings as commonly understood by those skilled in the art to which the present invention belongs.

The term "about" may refer to a value or composition within the acceptable error range for a given value or composition as determined by one of ordinary skill in the art, which will depend in part on how the value or composition is measured or determined.

As used herein, the terms "comprising" or "including" can be open, semi-closed, or closed. In other words, the terms also include "consisting essentially of" or "consisting of". Unless the context clearly requires otherwise, throughout the specification and the claims, the words "comprise", "have", "include", and the like are to be construed in an inclusive sense as opposed to an exclusive or exhaustive sense; that is to say, in the sense of "including, but not limited to". Unless stated otherwise, "comprising" includes "consisting of".

The term "alkyl" refers to a saturated straight or branched aliphatic hydrocarbon group having 1 to 20 (e.g. 1, 2, 3, 4, 5, 6, 7, 8, 9, 10, 11, 12, 13, 14, 15, 16, 17, 18, 19, or 20) carbon atoms (i.e., C₁₋₂₀ alkyl). The preferred alkyl group has 1 to 12 carbon atoms (i.e., C₁₋₁₂ alkyl), more preferably 1 to 6 carbon atoms (i.e., C₁₋₆ alkyl). Non-limiting examples include: methyl, ethyl, n-propyl, isopropyl, n-butyl, isobutyl, tert-butyl, sec-butyl, n-pentyl, 1,1-dimethylpropyl, 1,2-dimethylpropyl, 2,2-dimethylpropyl, 1-ethylpropyl, 2-methylbutyl, 3-methylbutyl, n-hexyl, 1-ethyl-2-methylpropyl, 1,1,2-trimethylpropyl, 1,1-dimethylbutyl, 1,2-dimethylbutyl, 2,2-dimethylbutyl, 1,3-dimethylbutyl, 2-ethylbutyl, 2-methylpentyl, 3-methylpentyl, 4-methylpentyl, 2,3-dimethylbutyl, n-heptyl, 2-methylhexyl, 3-methylhexyl, 4-methylhexyl, 5-methylhexyl, 2,3-dimethylpentyl, 2,4-dimethylpentyl, 2,2-dimethylpentyl, 3,3-dimethylpentyl, 2-ethylpentyl, 3-ethylpentyl, n-octyl, 2,3-dimethylhexyl, 2,4-dimethylhexyl, 2,5-dimethylhexyl, 2,2-dimethylhexyl, 3,3-dimethylhexyl, 4,4-dimethylhexyl, 2-ethylhexyl, 3-ethylhexyl, 4-ethylhexyl, 2-methyl-2-ethylpentyl, 2-methyl-3-ethylpentyl, n-nonyl, 2-methyl-2-ethylhexyl, 2-methyl-3-ethylhexyl, 2,2-diethylpentyl, n-decyl, 3,3-diethylhexyl, 2,2-diethylhexyl, and various branched isomers thereof, etc. The alkyl group may be substituted or unsubstituted. When substituted, it can be substituted at any available connecting point, and the substituent is preferably selected from one or more of D atom, halogen, alkoxy, haloalkyl, haloalkoxy, cycloalkyloxy, heterocyclyloxy, hydroxyl, hydroxyalkyl, cyano, amino, nitro, cycloalkyl, heterocyclyl, aryl, and heteroaryl.

The term "alkylene" refers to a divalent alkyl group, wherein alkyl is as defined above, having 1 to 20 (e.g., 1, 2, 3, 4, 5, 6, 7, 8, 9, 10, 11, 12, 13, 14, 15, 16, 17, 18, 19, or 20) carbon atoms (i.e., C₁₋₂₀ alkylene). The alkylene group preferably has 1 to 12 carbon atoms (i.e., C₁₋₁₂ alkylene), more preferably 1 to 6 carbon atoms (i.e., C₁₋₆ alkylene). Non-limiting examples include: -CH₂-, -CH(CH₃)-, -C(CH₃)₂-, -CH₂CH₂-, -CH(CH₂CH₃)-, -CH₂CH(CH₃)-, -CH₂C(CH₃)₂-, -CH₂CH₂CH₂-, -CH₂CH₂CH₂CH₂-, etc. The alkylene group may be substituted or unsubstituted. When substituted, it can be substituted at any available connecting point, and the substituent is preferably selected from one or more of D atom, halogen, alkoxy, haloalkyl, haloalkoxy, cycloalkyloxy, heterocyclyloxy, hydroxyl, hydroxyalkyl, cyano, amino, nitro, cycloalkyl, heterocyclyl, aryl, and heteroaryl.

The term "alkoxy" refers to -O-(alkyl), wherein alkyl is as defined above. Non-limiting examples include: methoxy, ethoxy, propoxy, and butoxy, etc. The alkoxy group may be substituted or unsubstituted. When substituted, it can be substituted at any available connecting point, and the substituent is preferably selected from one or more of D atom, halogen, alkoxy, haloalkyl, haloalkoxy, cycloalkyloxy, heterocyclyloxy, hydroxyl, hydroxyalkyl, cyano, amino, nitro, cycloalkyl, heterocyclyl, aryl, and heteroaryl.

The term "cycloalkyl" refers to a saturated or partially unsaturated monocyclic all-carbon ring (i.e., monocyclic cycloalkyl) or polycyclic ring system (i.e., polycyclic cycloalkyl) having 3 to 20 (e.g., 3, 4, 5, 6, 7, 8, 9, 10, 11, 12, 13, 14, 15, 16, 17, 18, 19, or 20) ring atoms (i.e., 3 to 20-membered cycloalkyl). The cycloalkyl group preferably has 3 to 12 ring atoms (i.e., 3 to 12-membered cycloalkyl), more preferably 3 to 8 ring atoms (i.e., 3 to 8-membered cycloalkyl, e.g., C₃₋₇ cycloalkyl), and most preferably 3 to 6 ring atoms (i.e., 3 to 6-membered cycloalkyl, e.g., C₃₋₆ cycloalkyl).

The term "aryl" refers to a monocyclic all-carbon aromatic ring (i.e., monocyclic aryl) or polycyclic aromatic ring system (i.e., polycyclic aryl) with a conjugated π-electron system, having 6 to 14 (e.g., 6, 7, 8, 9, 10, 11, 12, 13, or 14) ring atoms (i.e., 6 to 14-membered aryl). The aryl group preferably has 6 to 10 ring atoms (i.e., 6 to 10-membered aryl). The monocyclic aryl is, for example, phenyl. Non-limiting examples of the polycyclic aryl include: naphthyl, anthracenyl, phenanthrenyl, etc. The polycyclic aryl also includes phenyl fused with one or more heterocyclyl or cycloalkyl, or naphthyl fused with one or more heterocyclyl or cycloalkyl, wherein the connection point is on the phenyl or naphthyl ring, and in this case, the number of ring atoms continues to refer to the number of ring atoms in the polycyclic aromatic ring system. Non-limiting examples include: 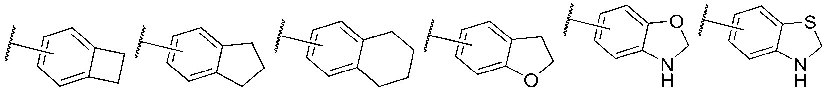 etc.

The aryl group may be substituted or unsubstituted. When substituted, it can be substituted at any available connecting point, and the substituent is preferably selected from one or more of D atom, halogen, alkyl, alkoxy, haloalkyl, haloalkoxy, cycloalkyloxy, heterocyclyloxy, hydroxyl, hydroxyalkyl, oxo, cyano, amino, nitro, cycloalkyl, heterocyclyl, aryl, and heteroaryl.

The term "heteroaryl" refers to a monocyclic heteroaromatic ring (i.e., monocyclic heteroaryl) or polycyclic heteroaromatic ring system (i.e., polycyclic heteroaryl) with a conjugated π-electron system, containing at least one (e.g., 1, 2, 3, or 4) heteroatom selected from nitrogen, oxygen, and sulfur in the ring (said nitrogen may optionally be oxidized, i.e., forming N-oxide; said sulfur may optionally be oxo-substituted, i.e., forming sulfoxide or sulfone, but excluding -O-O-, -O-S-, or -S-S-), having 5 to 14 (e.g., 5, 6, 7, 8, 9, 10, 11, 12, 13, or 14) ring atoms (i.e., 5 to 14-membered heteroaryl). The heteroaryl group preferably has 5 to 10 ring atoms (i.e., 5 to 10-membered heteroaryl), more preferably 5 or 6 ring atoms (i.e., 5 or 6-membered heteroaryl).

The term "cycloalkyloxy" refers to cycloalkyl-O-, wherein cycloalkyl is as defined above. The term "heterocyclyloxy" refers to heterocyclyl-O-, wherein heterocyclyl is as defined above. The term "aryloxy" refers to aryl-O-, wherein aryl is as defined above. The term "heteroaryloxy" refers to heteroaryl-O-, wherein heteroaryl is as defined above. The term "alkylthio" refers to alkyl-S-, wherein alkyl is as defined above. The term "haloalkyl" refers to alkyl substituted by one or more halogen atoms, wherein alkyl is as defined above. The term "deuteroalkyl" refers to alkyl substituted by one or more deuterium atoms, wherein alkyl is as defined above. The term "haloalkoxy" refers to alkoxy substituted by one or more halogen atoms, wherein alkoxy is as defined above.

The term "hydroxyalkyl" refers to alkyl substituted by one or more hydroxyl groups, wherein alkyl is as defined above. The term "halogen" refers to fluorine, chlorine, bromine, or iodine. The term "hydroxyl" refers to -OH. The term "amino" refers to -NH₂. The term "cyano" refers to -CN. N-Ethyldiisopropylamine is abbreviated as DIEA. N,N-Dimethylformamide is abbreviated as DMF. O-(7-Azabenzotriazol-1-yl)-N,N,N',N'-tetramethyluronium hexafluorophosphate is abbreviated as HATU. 1-Hydroxybenzotriazole is abbreviated as HOBt.

"Substituted" means that one or more hydrogen atoms, preferably 1 to 6, more preferably 1 to 3 hydrogen atoms, in a group are independently replaced by a corresponding number of substituents. Those skilled in the art can determine possible or impossible substitutions without undue effort (experimentally or theoretically). For example, an amino or hydroxyl group with a free hydrogen attached to a carbon atom having an unsaturated (e.g., olefinic) bond may be unstable.

As used herein, the term "amino acid residue" refers to the group formed by the removal of one H from the N-terminal -NH₂ and the removal of -OH from the C-terminal -COOH of an amino acid. Generally, the chain segment of an amino acid (residue) including the N-terminus and C-terminus is referred to as the backbone, while the part that determines the specific type of amino acid is called the side chain. Typically, an amino acid residue is represented as -NH-CH(R)-CO-, where R is the side chain (amino acid side chain). Unless otherwise defined, amino acids herein include natural or unnatural amino acids, including D- and/or L-amino acids. Examples of amino acids include but are not limited to: Ala (A), Arg (R), Asn (N), Asp (D), Cys (C), Gln (Q), Glu (E), Gly (G), His (H), Ile (I), Leu (L), Lys (K), Met (M), Phe (F), Pro (P), Ser (S), Thr (T), Trp (W), Tyr (Y), Val (V). Preferably, the amino acid herein is selected from the group consisting of: L-glycine (L-Gly), L-alanine (L-Ala), β-alanine (β-Ala), L-glutamic acid (L-Glu), L-aspartic acid (L-Asp), L-histidine (L-His), L-arginine (L-Arg), L-lysine (L-Lys), L-valine (L-Val), L-serine (L-Ser), L-threonine (L-Thr). Furthermore, when an amino acid contains two or more amino groups and/or two or more carboxyl groups, the term also includes the divalent group formed by the removal of one H from a -NH₂ and the removal of -OH from a -COOH that are not on the same carbon atom. For example, the divalent group -C(O)-(CH₂)₂-C(COOH)-NH- formed by the removal of one H from the -NH₂ and the non-α-position -COOH of glutamic acid.

As used herein, the term "pharmaceutically acceptable salt" refers to salts formed by the compounds of the present invention with acids or bases that are suitable for pharmaceutical use. Pharmaceutically acceptable salts include inorganic salts and organic salts. A preferred class of salts is those formed by the compounds of the present invention with acids. Acids suitable for salt formation include, but are not limited to: inorganic acids such as hydrochloric acid, hydrobromic acid, hydrofluoric acid, sulfuric acid, nitric acid, phosphoric acid, etc.; organic acids such as formic acid, acetic acid, propionic acid, oxalic acid, malonic acid, succinic acid, fumaric acid, maleic acid, lactic acid, malic acid, tartaric acid, citric acid, picric acid, methanesulfonic acid, benzenesulfonic acid, p-toluenesulfonic acid, etc.; and acidic amino acids such as aspartic acid, glutamic acid. 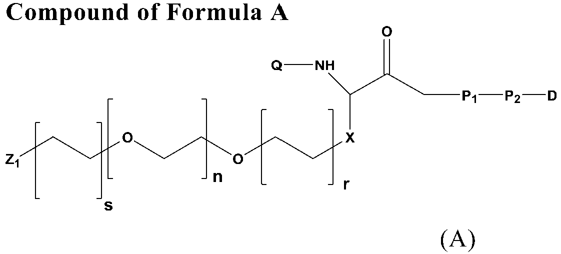 wherein:
Q is a linker group for connecting to an antibody;
Z1 comprises a glucosyl group containing Y hydroxyl groups;
s is an integer from 0 to 10;
n is an integer from 1 to 24;
r is an integer from 0 to 10;
X is a linking group;
P1 is a polypeptide residue;
P2 is a chemical bond or an AA-PAB structure; wherein AA is a dipeptide, tripeptide, or tetrapeptide fragment (i.e. a fragment formed by 2-4 amino acids linked through peptide bonds), and PAB is p-aminobenzylaminocarbonyl group;
D is a drug.

### Preparation Method

The following provides a more specific description of the preparation method of the compound of Formula A of the present invention, but these specific methods do not constitute any limitation on the present invention. The compounds of the present invention can also be conveniently prepared by combining various synthetic methods described in this specification or known in the art, and such combinations can be easily carried out by those skilled in the art to which the present invention belongs.

Typically, in the preparation process, each reaction is generally carried out in an inert solvent at room temperature to reflux temperature (such as 0 °C to 80 °C, preferably 0 °C to 50 °C). The reaction time is usually 0.1-60 hours, preferably 0.5-48 hours.

The general preparation routes below can be used to synthesize the compounds of Formula A of the present invention.

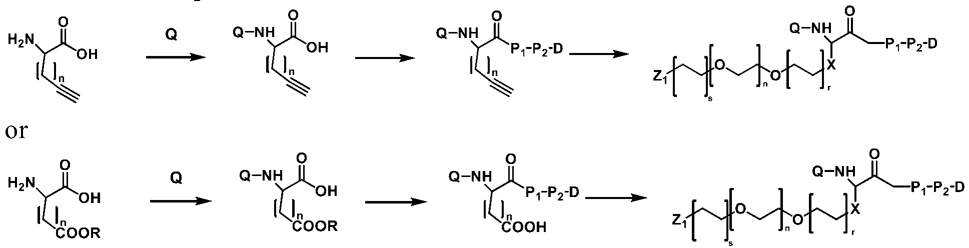

Furthermore, the preparation route for the antibody-drug conjugate is shown below. The interchain disulfide bonds of the antibody are reduced, generating 2n (e.g., 4) thiol groups. The substituted maleimide linker-drug conjugate of the present invention is crosslinked with the thiol groups of the reduced antibody to generate the corresponding antibody-drug conjugate.

### Antibody

As used herein, the terms "antibody" or "immunoglobulin" refer to heterotetrameric glycoproteins of approximately 150,000 daltons, sharing the same structural characteristics, composed of two identical light chains (L) and two identical heavy chains (H). Each light chain is linked to a heavy chain by a covalent disulfide bond, with the number of disulfide bonds between the heavy chains varying among different immunoglobulin isotypes. Each heavy and light chain also has regularly spaced intrachain disulfide bonds. Each heavy chain has a variable region (VH) at one end, followed by multiple constant regions. Each light chain has a variable region (VL) at one end and a constant region at the other end; the constant region of the light chain is aligned with the first constant region of the heavy chain, and the variable region of the light chain is aligned with the variable region of the heavy chain. Specific amino acid residues form an interface between the variable regions of the light and heavy chains.

As used herein, the term "variable" indicates that certain portions of the variable regions of antibodies differ in sequence, which confers the binding and specificity of various specific antibodies for their specific antigens. However, variability is not distributed evenly throughout the antibody variable regions. It is concentrated in three segments called complementarity determining regions (CDRs) or hypervariable regions in the variable regions of both the light and heavy chains. The more conserved portions of the variable regions are called framework regions (FR). The variable regions of natural heavy and light chains each comprise four FR regions, adopting a predominantly β-sheet configuration, connected by three CDRs forming loop structures, and in some cases may form part of the β-sheet structure. The CDRs in each chain are held in close proximity by the FR regions and, together with the CDRs from the other chain, contribute to the formation of the antigen-binding site of the antibody (see Kabat et al., NIH Publ. No. 91-3242, Vol. I, pages 647-669 (1991)). The constant regions are not directly involved in binding the antibody to the antigen but exhibit various effector functions, such as participation in antibody-dependent cellular cytotoxicity.

The "light chains" of vertebrate antibodies (immunoglobulins) can be assigned to one of two distinct types (called kappa and lambda) based on the amino acid sequences of their constant regions. Immunoglobulins can be divided into different classes based on the amino acid sequences of their heavy chain constant regions. There are five major classes of immunoglobulins: IgA, IgD, IgE, IgG, and IgM, some of which can be further divided into subclasses (isotypes), such as IgG1, IgG2, IgG3, IgG4, IgA1, and IgA2. The heavy chain constant regions corresponding to the different classes of immunoglobulins are called α, δ, ε, γ, and µ, respectively. The subunit structures and three-dimensional configurations of the different classes of immunoglobulins are well known to those skilled in the art.

Generally, the antigen-binding properties of an antibody can be described by three specific regions located in the variable regions of the heavy and light chains, called complementarity determining regions (CDRs), which are interspersed by four framework regions (FRs). The amino acid sequences of the four FRs are relatively conserved and not directly involved in the binding reaction. These CDRs form loop structures that are brought into close proximity in three-dimensional space by the β-sheets formed by the intervening FRs. The CDRs on the heavy chain and the corresponding CDRs on the light chain constitute the antigen-binding site of the antibody. The amino acids constituting the FR or CDR regions can be determined by comparing the amino acid sequences of antibodies of the same type.

The present invention includes not only intact antibodies but also fragments of antibodies having immunologic activity or fusion proteins formed by antibodies and other sequences. Therefore, the present invention also includes fragments, derivatives, and analogs of said antibodies.

In the present invention, antibodies include murine, chimeric, humanized, or fully human antibodies prepared using techniques well known to those skilled in the art. Recombinant antibodies, such as chimeric and humanized monoclonal antibodies, comprising both human and non-human portions, can be obtained by standard DNA recombination techniques and are all useful antibodies. A chimeric antibody is a molecule in which different portions derive from different animal species, for example, a chimeric antibody having the variable region from a murine monoclonal antibody and the constant region from a human immunoglobulin (see, for example, U.S. Patent No. 4,816,567 and 4,816,397, incorporated herein by reference in their entirety). A humanized antibody refers to an antibody molecule derived from a non-human species, having one or more complementarity determining regions (CDRs) derived from a non-human species and framework regions derived from a human immunoglobulin molecule (see U.S. Patent No. 5,585,089, incorporated herein by reference in its entirety). These chimeric and humanized monoclonal antibodies can be prepared using DNA recombination techniques well known in the art.

In the present invention, the antibody can be monospecific, bispecific, trispecific, or of higher multispecificity.

In the present invention, the antibodies of the present invention also include conservative variants thereof, which refer to polypeptides wherein, compared to the amino acid sequence of the antibody of the present invention, up to 10, preferably up to 8, more preferably up to 5, and most preferably up to 3 amino acids are replaced by amino acids with similar or related properties. These conservative variant polypeptides are preferably produced by amino acid substitutions according to Table A.

**Table A**

| Original residue | Representative substitution | Preferred Substitution |
|---|---|---|
| Ala (A) | Val; Leu; Ile | Val |
| Arg (R) | Lys; Gln; Asn | Lys |
| Asn (N) | Gln; His; Lys; Arg | Gln |
| Asp (D) | Glu | Glu |
| Cys (C) | Ser | Ser |
| Gln (Q) | Asn | Asn |
| Glu (E) | Asp | Asp |
| Gly (G) | Pro; Ala | Ala |
| His (H) | Asn; Gln; Lys; Arg | Arg |
| Ile (I) | Leu; Val; Met; Ala; Phe | Leu |
| Leu (L) | Ile; Val; Met; Ala; Phe | Ile |
| Lys (K) | Arg; Gln; Asn | Arg |
| Met (M) | Leu; Phe; Ile | Leu |
| Phe (F) | Leu; Val; Ile; Ala; Tyr | Leu |
| Pro (P) | Ala | Ala |
| Ser (S) | Thr | Thr |
| Thr (T) | Ser | Ser |
| Trp (W) | Tyr; Phe | Tyr |
| Tyr (Y) | Trp; Phe; Thr; Ser | Phe |
| Val (V) | Ile; Leu; Met; Phe; Ala | Leu |

### Preparation of Antibodies

The DNA sequences encoding the antibody or fragment thereof of the present invention can be obtained by conventional techniques, such as PCR amplification or genomic library screening. Furthermore, the coding sequences for the light and heavy chains can be fused together to form a single-chain antibody.

Once the relevant sequences are obtained, they can be produced in large quantities using recombinant methods. This typically involves cloning the sequences into a vector, introducing the vector into cells, and then isolating the relevant sequences from the propagated host cells using conventional methods.

Additionally, relevant sequences can be synthesized artificially, especially when the fragment length is short. Usually, sequences of considerable length can be obtained by first synthesizing multiple small fragments and then ligating them.

Currently, DNA sequences encoding the antibody (or fragment or derivative thereof) of the present invention can be obtained entirely by chemical synthesis. This DNA sequence can then be introduced into various existing DNA molecules (such as vectors) and cells known in the art. Furthermore, mutations can be introduced into the protein sequence of the present invention by chemical synthesis.

The present invention also relates to vectors comprising the aforementioned appropriate DNA sequences and appropriate promoters or control sequences. These vectors can be used to transform appropriate host cells to enable them to express the protein.

The host cell can be a prokaryotic cell, such as a bacterial cell; a lower eukaryotic cell, such as a yeast cell; or a higher eukaryotic cell, such as a mammalian cell. Preferred animal cells include (but are not limited to): CHO-S, HEK-293 cells.

Typically, the transformed host cells are cultured under conditions suitable for the expression of the antibody of the present invention. The antibody of the present invention is then purified using conventional immunoglobulin purification steps, such as protein A-Sepharose, hydroxyapatite chromatography, gel electrophoresis, dialysis, ion-exchange chromatography, hydrophobic chromatography, size-exclusion chromatography, or affinity chromatography, and other conventional separation and purification methods well known to those skilled in the art.

The obtained monoclonal antibodies can be identified using conventional methods. For example, the binding specificity of monoclonal antibodies can be determined by immunoprecipitation or *in vitro* binding assays (such as radioimmunoassay (RIA) or enzyme-linked immunosorbent assay (ELISA)). The binding affinity of monoclonal antibody can be determined, for example, by Scatchard analysis by Munson et al., Anal. Biochem., 107:220 (1980).

The antibody of the present invention can be expressed intracellularly, on cell membranes, or secreted extracellularly. If needed, recombinant proteins can be separated and purified through various separation methods using their physical, chemical, and other properties. These methods are well-known to those skilled in the art. Examples of these methods include but are not limited to: conventional renaturation treatment, protein precipitation treatment (salting-out method), centrifugation, osmotic bacteria lysis, ultrasonic treatment, ultracentrifugation, molecular sieve chromatography (gel filtration), adsorption chromatography, ion-exchange chromatography, high-performance liquid chromatography (HPLC) and other various liquid chromatography technologies and combinations thereof.

### Antibody-Drug Conjugate (ADC)

The present invention also provides an antibody-drug conjugate (ADC) based on the antibody of the present invention.

Typically, the antibody-drug conjugate comprises the antibody and an effector molecule, wherein the antibody is conjugated to the effector molecule, and preferably chemically conjugated. The preferred effector molecule is a drug with therapeutic activity. In addition, the effector molecule may be one or more of toxic proteins, chemotherapeutic drugs, small molecule drugs, or radionuclides.

The antibody of the present invention can be conjugated with the effector molecule through a conjugating agent. Examples of the conjugating agent can be any one or several of non-selective conjugating agents, conjugating agents utilizing carboxyl groups, peptide chains, and conjugating agents utilizing disulfide bonds. The non-selective conjugating agent refers to a compound that forms a covalent bond between the effector molecule and the antibody, such as glutaraldehyde. The conjugating agent utilizing carboxyl groups can be any one or several of the cis-aconitic anhydride based conjugating agents (such as cis-aconitic anhydride) and acyl hydrazone based conjugating agents (with acyl hydrazone as the conjugating site).

Some residues on antibodies, such as Cys or Lys, are used to link to various functional groups, including imaging reagents (such as chromophores and fluorescent groups), diagnostic reagents (such as MRI contrast agents and radioactive isotopes), stabilizers (such as ethylene glycol polymers), and therapeutic agents. Antibodies can be conjugated to functional agents to form antibody-functional agent conjugates. Functional agents (such as drugs, detection reagents, stabilizers) are covalently linked to antibodies. Functional agents can be directly or indirectly linked to antibodies through a linker.

Typical conjugating methods applicable to the present invention include K-Lock and C-Lock conjugating methods. In the K-Lock conjugating method, the drug molecule is conjugated to the lysine (K) residue in the antibody sequence, while in the C-Lock conjugating method, the drug molecule is conjugated to the cysteine (C) residue in the antibody sequence.

Antibodies can be conjugated to drugs to form antibody-drug conjugates (ADCs). Typically, ADC comprises a linker located between the drug and the antibody. The joint can be degradable or non degradable. Degradable linkers are typically prone to degradation in the intracellular environment, such as at the target site, leading to the release of drugs from antibodies. Suitable degradable linkers include, for example, enzyme degradable linkers, which include peptide based linkers that can be degraded by intracellular proteases (such as lysosomal proteases or endosomal proteases), or sugar linkers, such as glucuronide containing linkers that can be degraded by glucosidase. Peptide based linkers can include, for example, dipeptides such as valine-citrulline, phenylalanine-lysine, or valine-alanine. Other suitable degradable linkers include, for example, pH sensitive linkers (such as linkers that hydrolyze at pH less than 5.5, such as hydrazone linkers) and linkers that degrade under reducing conditions (such as disulfide bond linkers). Non-degradable linkers typically release drugs under conditions where antibodies are hydrolyzed by proteases.

Before linked to the antibody, the linker has reactive groups that can react with certain amino acid residues, and the linking is achieved through reactive groups. Thiol specific reactive groups are preferred and include, for example, maleimide compounds, haloacetamides (such as iodine, bromine, or chlorine); haloacetates (such as iodine, bromine, or chlorinated); halomethyl ketones (such as iodine, bromine, or chlorine), benzyl halides (such as iodine, bromine, or chlorine); vinyl sulfone, pyridyl disulfide; mercury derivatives such as 3,6-bis(mercurymethyl)dioxane, with acetate, chloride, or nitrate ions as the counter ions; and polymethylene dimethyl sulfide thiosulfates. The linker may include, for example, a maleimide linked to the antibody via a thiosuccinimide.

The drug can be any cytotoxic, cytostatic, or immunosuppressive drug. In embodiments, the linker connects the antibody and the drug, and the drug has a functional group capable of bonding with the linker. For example, the drug can have an amino, carboxyl, thiol, hydroxyl, or ketone group that can bond with the linker. In cases where the drug is directly connected to the linker, the drug possesses a reactive group before attachment to the antibody.

Useful classes of drugs include, for example, anti-tubulin agents, DNA minor groove binders, DNA replication inhibitors, alkylating agents, antibiotics, antifolates, antimetabolites, chemosensitizers, topoisomerase inhibitors, vinca alkaloids, etc. Examples of particularly useful classes of cytotoxic drugs include, for example, DNA minor groove binders, DNA alkylating agents, and tubulin inhibitors. Typical cytotoxic drugs include, for example, auristatins, camptothecins, duocarmycins, etoposides, maytansines and maytansinoids (e.g., DM1 and DM4), taxanes, benzodiazepines or benzodiazepine-containing drugs (e.g., pyrrolo[1,4]benzodiazepines (PBDs), indolinobenzodiazepines, and oxazolidinobenzodiazepines), and vinca alkaloids.

In the present invention, drug-linker constructs can be used to form ADCs in a single step. In other embodiments, bifunctional linker compounds can be used to form ADCs in a two-step or multi-step method. For example, cysteine residues react with the reactive moiety of the linker in a first step, and in a subsequent step, a functional group on the linker reacts with the drug to form the ADC.

Typically, the functional groups on the linker are selected to facilitate specific reaction with suitable reactive groups on the drug moiety. As a non-limiting example, azide-based moieties can be used to specifically react with reactive alkyne groups on the drug moiety. The drug is covalently attached to the linker via 1,3-dipolar cycloaddition between the azide and the alkyne. Other useful functional groups include, for example, ketones and aldehydes (suitable for reaction with hydrazides and alkoxyamines), phosphines (suitable for reaction with azides); isocyanates and isothiocyanates (suitable for reaction with amines and alcohols); and activated esters, such as N-hydroxysuccinimide esters (suitable for reaction with amines and alcohols). These and other conjugation strategies, such as those described in Bioconjugate Techniques, Second Edition (Elsevier), are well known to those skilled in the art. Those skilled in the art will appreciate that for selective reactions between the drug moiety and the linker, when a complementary pair of reactive functional groups is selected, each member of the complementary pair can be used on either the linker or the drug.

The present invention also provides methods for preparing ADCs, which can further comprise: combining the antibody with a drug-linker compound under conditions sufficient to form the antibody conjugate (ADC).

In some embodiments, the method of the present invention comprises: combining the antibody with a bifunctional linker compound under conditions sufficient to form an antibody-linker conjugate. In these embodiments, the method of the present invention further comprises: combining the antibody-linker conjugate with a drug moiety under conditions sufficient to covalently attach the drug moiety to the antibody via the linker.

In some embodiments, the antibody-drug conjugate ADC has the following molecular formula: 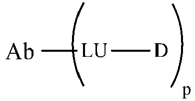 wherein:
Ab is an antibody,
LU is a linker;
D is a drug;
and the subscript p is a value selected from 1-10, preferably 1 to 8.

### Drug

As used herein, "drug" generally refers to any compound with expected biological activity and reactive functional groups for the preparation of the conjugate of the present invention. The expected biological activity includes diagnosing, curing, alleviating, treating, and preventing diseases in humans or other animals. Therefore, as long as it has the necessary reactive functional groups, the term "drug" refers to compounds that are officially recognized in national pharmacopoeias, as well as in official pharmacopoeias such as the United States Pharmacopeia, the National Formulary, or any supplements thereof. Typical drugs are listed in the Physician's Desk Medication Reference (PDR) and the Orange Book of the US Food and Drug Administration (FDA). It should be understood that with the continuous discovery and development of new drugs, these drugs should also be included as "drugs" in the conjugates of the present invention.

Drugs useful for constructing the ADCs of the present invention include, but are not limited to: cytotoxic agents (e.g., cytotoxic small molecule drugs).

The term "cytotoxic agent" refers to a substance that inhibits or prevents cellular expression activity, cellular function, and/or causes cellular damage. The term includes radioactive isotopes, chemotherapeutic agents, and toxins such as small molecule toxins or enzyme active toxins of bacterial, fungal, plant, or animal origin, including their fragments and/or variants. Examples of cytotoxic agents include but are not limited to: auristatins (e.g., auristatin E, auristatin F, MMAE, and MMAF), actinomycin, maytansinoids, ricin, ricin A-chain, combretastatin, duocarmycin, dolastatin, doxorubicin, daunorubicin, paclitaxel, cisplatin, CC-1065, ethidium bromide, mitomycin, etoposide, tenoposide, vincristine, vinblastine, colchicine, dihydroxyanthracenedione, dactinomycin, diphtheria toxin, Pseudomonas exotoxin (PE) A, PE40, abrin, abrin A chain, modeccin A chain, alpha-sarcin, gelonin, mitogellin, restrictocin, phenomycin, enomycin, curicin, crotin, calicheamicin, saporin, glucocorticoids, and other chemotherapeutic agents, as well as radioactive isotopes such as At211, I131, I125, Y90, Re186, Re188, Sm153, Bi212 or 213, P32, and radioactive isotopes of Lu including Lu177. Antibodies can also be conjugated to enzymes capable of converting prodrugs into their active forms.

Preferred small molecule drugs are compounds with high cytotoxicity, preferably monomethyl auristatin, calicheamicin, maytansinoids, or combinations thereof; more preferably selected from: monomethyl auristatin E (MMAE), monomethyl auristatin D (MMAD), monomethyl auristatin F (MMAF), or combinations thereof.

Preferably, the "drug" refers to: a cytotoxic drug for cancer treatment, or a protein or polypeptide with a desired biological activity, such as a toxin like abrin, ricin A, Pseudomonas exotoxin, and diphtheria toxin; other suitable proteins include tumor necrosis factor, alpha-interferon, beta-interferon, nerve growth factor, platelet-derived growth factor, tissue plasminogen activator, and biological response modifiers such as lymphokines, interleukin-1 (IL-1), interleukin-2 (IL-2), interleukin-6 (IL-6), granulocyte-macrophage colony-stimulating factor (GM-CSF), granulocyte colony-stimulating factor, or other growth factors.

A preferred drug of the present invention is maytansine or a maytansinoid. Maytansine compounds inhibit cell proliferation by inhibiting tubulin polymerization. Maytansinoids are derivatives of maytansine. Both maytansine and maytansinoids possess high cytotoxicity, but their clinical application in cancer therapy is largely limited by their low selectivity for tumors. However, this high cytotoxicity makes them prime candidates as drug components for antibody-drug conjugates. The structure of desacetylmaytansine is listed below.

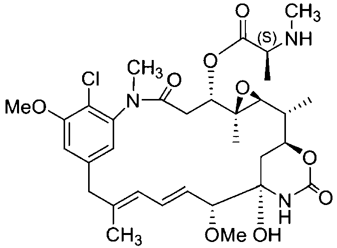

### Desacetylmaytansine

Another preferred drug of the present invention is the auristatin class of peptides. Auristatin peptides are analogs of dolastatin 10, a biologically active polypeptide isolated from the marine mollusk Dolabella auricularia. Dolastatin 10 inhibits tubulin polymerization by binding to tubulin (at the same binding site as vincristine). Dolastatin 10, auristatin PE, and auristatin E are linear peptides containing four amino acids (three of which are unique to the dolastatin class) and a C-terminal amide group. Two representative auristatin compounds, monomethyl auristatin E (MMAE) and monomethyl auristatin F (MMAF), are both preferred drugs for antibody-drug conjugates.

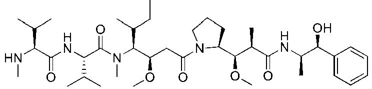

### Monomethyl Auristatin E(MMAE)

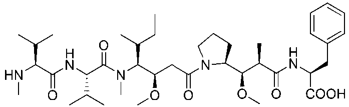

### Monomethyl Auristatin F(MMAF)

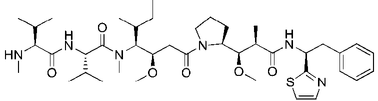

### Monomethyl Dolastatin 10(MMAD)

Another preferred drug of the present invention is pyrrolo[2,1-c][1,4]benzodiazepines (PBDs) or PBD dimers. PBDs are a class of natural products produced by Streptomyces, uniquely characterized by their ability to form non-distorting covalent adducts in the minor groove of DNA, specifically at purine-guanine-purine sequences. There is growing interest in using PBDs as part of small molecule strategies to target specific DNA sequences and as novel anticancer and antibacterial agents. Dimers formed by linking two PBD units at their C8/C8' hydroxyl groups via a flexible carbon chain exhibit enhanced biological activity. PBD dimers are believed to produce sequence-selective DNA damage, such as reversed 5'-Pu-GATC-Py-3' interstrand cross-links, leading to their biological activity. These compounds have been shown to be highly potent cytotoxic agents, making them candidate drugs for antibody-drug conjugates. 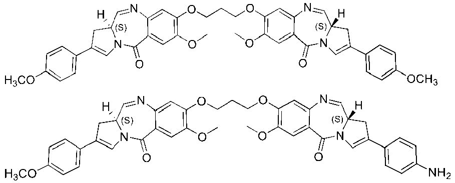 PBD dimer

Another preferred drug of the present invention is PNU-159682 derivatives. PNU-159682 is the major active metabolite of Nemorubicin in human liver microsomes, exhibiting 3000-fold higher activity compared to MMDX and doxorubicin.

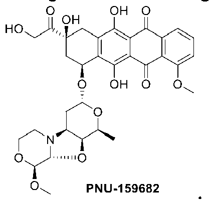

On the other hand, drugs are not limited to the categories mentioned above and include all drugs suitable for use in antibody-drug conjugates. Particularly, those cytotoxins that can coordinate via amide bonds with the linker, such as through a basic amine group (primary or secondary amine).

The term "antibody-drug conjugate" (ADC) refers to a monoclonal antibody or antibody fragment linked to a biologically active toxic drug via a connecting unit. The antibodies or antibody fragments described in this disclosure can be conjugated to effector molecules by any means. For example, antibodies or antibody fragments can be attached to toxic drugs chemically or recombinantly. Chemical methods for preparing fusions or conjugates are known in the art. The methods used for conjugating antibodies or antibody fragments and drugs must be capable of linking the antibody to the toxic drug without interfering with the ability of the antibody or antibody fragment to bind to its target molecule.

The present invention also provides methods for preparing ADCs, which can further comprise: combining the antibody with a drug-linker compound (or linker-drug compound, LD, such as LD-1 to LD-17 shown in the present invention) under conditions sufficient to form the antibody conjugate (ADC).

In some embodiments, the method of the present invention comprises: combining the antibody with a linker compound under conditions sufficient to form an antibody-linker conjugate. In these embodiments, the method of the present invention further comprises: combining the antibody-linker conjugate with a drug moiety under conditions sufficient to covalently attach the drug moiety to the antibody via the linker.

Drug Loading, also known as the Drug-to-Antibody Ratio (DAR), refers to the average number of drug molecules conjugated per antibody in the ADC. It can range, for example, from about 1 to about 10 drugs per antibody, and in certain embodiments, from about 1 to about 8 drugs per antibody, preferably selected from the ranges of 2-8, 2-7, 2-6, 2-5, 2-4, 3-4, 3-5, 5-6, 5-7, 5-8, and 6-8. Exemplarily, the drug loading can be a mean value of 1, 2, 3, 4, 5, 6, 7, 8, 9, or 10. The general formula of the ADCs of this disclosure encompasses a collection of antibody-drug conjugates within the aforementioned ranges. In embodiments of this disclosure, the drug loading can be represented as n, which is a decimal or integer. Conventional methods such as UV/Vis spectroscopy, mass spectrometry, ELISA assays, and HPLC can be used to determine the drug loading.

In one embodiment of the present invention, the cytotoxic drug is conjugated to the antibody via a linker unit.

The loading of ligand drug conjugates can be controlled using the following non-limiting methods, including:
(1) controlling the molar ratio of the drug-linker fragment to the monoclonal antibody,
(2) controlling the reaction time and temperature,
(3) selecting different reaction reagents.

### Pharmaceutical Compositions and Administration Methods

Since the antibody-drug conjugates provided by the present invention can target specific cell populations and bind to specific proteins (antigens) on the cell surface, thereby releasing the drug in its active form into the cell through conjugate internalization or drug penetration, the antibody-drug conjugates of the present invention can be used to treat target diseases. The aforementioned antibody-drug conjugates can be administered to a subject (e.g., a human) in a therapeutically effective amount via suitable routes. Subjects in need of treatment may be patients at risk of or suspected of having a disorder related to the activity or expression level of a specific antigen. Such patients can be identified through routine physical examinations.

Conventional methods known to those of ordinary skill in the medical art can be used to administer the pharmaceutical composition to a subject, depending on the type of disease to be treated or the site of the disease. This composition can also be administered via other conventional routes, for example, orally, parenterally, by inhalation spray, topically, rectally, nasally, buccally, vaginally, or via an implanted reservoir. As used herein, the term "parenteral" includes subcutaneous, intradermal, intravenous, intramuscular, intra-articular, intra-arterial, intrasynovial, intrasternal, intrathecal, intralesional, and intracranial injection or infusion techniques. Furthermore, it can be administered via an injectable depot route, for example, using 1-, 3-, or 6-month depot injectable or biodegradable materials and methods.

Injectable compositions may contain various carriers such as vegetable oil, dimethylacetamide, dimethylformamide, ethyl lactate, ethyl carbonate, isopropyl myristate, ethanol, polyols (glycerol, propylene glycol, liquid polyethylene glycol, etc.). For intravenous injection, water-soluble antibodies can be administered by the drip method, whereby a pharmaceutical preparation containing the antibody and a physiologically acceptable excipient is infused. Physiologically acceptable excipients may include, for example, 5% dextrose, 0.9% saline, Ringer's solution, or other suitable excipients. Intramuscular preparations, for example, a sterile preparation of a suitable soluble salt form of the antibody, can be dissolved and administered in a pharmaceutical excipient such as Water for Injection, 0.9% saline, or 5% dextrose solution.

When treating with the antibody-drug conjugate of the present invention, delivery can be achieved by conventional methods in the art. For example, it can be introduced into cells using liposomes, hydrogels, cyclodextrins, biodegradable nanocapsules, or bioadhesive microspheres. Alternatively, the nucleic acid or vector can be delivered locally by direct injection or by using an infusion pump. Other methods include various transport and carrier systems using conjugates and biodegradable polymers.

The pharmaceutical composition of the present invention contains a safe and effective amount of the antibody-drug conjugate of the present invention and a pharmaceutically acceptable carrier. Such carriers include (but are not limited to): saline, buffers, dextrose, water, glycerol, ethanol, and combinations thereof. Generally, the pharmaceutical formulation should match the mode of administration. The pharmaceutical composition of the present invention can be prepared in the form of a solution, for example, prepared by conventional methods using physiological saline or an aqueous solution containing glucose and other adjuncts. The pharmaceutical composition is preferably manufactured under sterile conditions. The amount of the active ingredient administered is a therapeutically effective amount.

The effective amount of the antibody-drug conjugate of the present invention can vary depending on the mode of administration, the severity of the disease to be treated, etc. Selection of a preferred effective amount can be determined by one of ordinary skill in the art based on various factors (e.g., through clinical trials). These factors include, but are not limited to: the pharmacokinetic parameters of the antibody conjugate such as bioavailability, metabolism, half-life, etc.; the severity of the disease to be treated in the patient; the patient's body weight; the patient's immune status; the route of administration, etc. Generally, satisfactory results can be obtained when the antibody-drug conjugate of the present invention is administered daily at a dose of about 0.0001 mg to 50 mg/kg animal body weight (preferably 0.001 mg to 10 mg/kg animal body weight). For example, depending on the urgency of the treatment condition, several divided doses may be administered daily, or the dose may be proportionally reduced.

Dosage forms of the compound of the present invention for topical administration include ointments, powders, patches, sprays, and inhalants. The active ingredient is mixed under sterile conditions with a physiologically acceptable carrier and any preservatives, buffers, or propellants as may be required.

The compounds of the present invention may be administered alone or in combination with other pharmaceutically acceptable therapeutic agents.

When using the pharmaceutical composition, a safe and effective amount of the antibody conjugate of the present invention is applied to a mammal (e.g., a human) in need of treatment, wherein the dose for administration is a pharmaceutically recognized effective dose. For a 60 kg human, the daily dose is typically 1 to 2000 mg, preferably 5 to 500 mg. Of course, the specific dosage should also consider factors such as the route of administration and the patient's health status, which are within the purview of the skilled physician.

### Main Advantages of the Present Invention

(1) The monomer ratio of the conjugate detected by SEC-HPLC is within the normal range (purity>90%). The antibody-drug conjugate prepared by the novel linker of the present invention exhibits good solubility and druggability, and no precipitation occurs during the conjugation process.
(2) The linker of the antibody-drug conjugate of the present invention has good solubility, which can greatly improve the water solubility of existing small molecule compounds and enhance the stability and uniformity of the conjugate after conjugating with antibodies.
(3) This has a broad application scope and can be conjugated with drugs having different mechanisms of action to obtain various antibody-drug conjugates, which is expected to improve the therapeutic window of existing conjugated drugs.
(4) Compared with the prior art, the hydrophilic side chains of the linker of the antibody-drug conjugate of the present invention have higher hydrophilicity, resulting in better conjugation efficiency and applicability for conjugating with different carrier drugs, such as nanobodies.
(5) The conjugate prepared with the linker of the present invention has excellent therapeutic effects in various *in vitro* and *in vivo* pharmacological models, and is superior to the conjugate drugs prepared by existing technologies.
(6) The conjugate prepared with the linker of the present invention has good safety in preclinical toxicological assessments in cynomolgus monkeys.

The present invention will be further described in conjunction with specific examples. It should be understood that these examples are only used to illustrate the present invention and not to limit the scope of the present invention. Experimental methods without detailed conditions specified in the following examples are generally carried out under conventional conditions as described in Sambrook et al., Molecular Cloning: A Laboratory Manual (New York: Cold Spring Harbor Laboratory Press, 1989), or under conditions suggested by the manufacturer. Unless otherwise stated, percentages and parts are by weight.

### Example 1 Synthesis of Linker-STING Agonist Compound A1 (L1-AN014)

### Step 1: Preparation of Compound 3

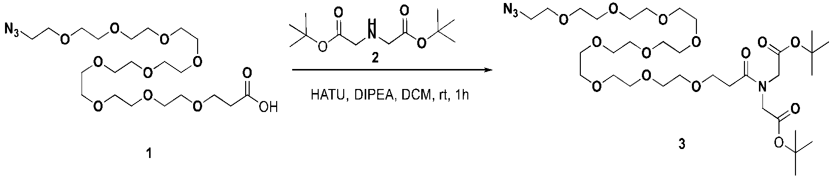

Compound **1** (1.00 g, 1.96 mmol, 1.0 eq) and Compound **2** (720 mg, 2.95 mmol, 1.5 eq) were dissolved in 20 mL of anhydrous dichloromethane. HATU (900 mg, 2.37 mmol, 1.2 eq) and N,N-Diisopropylethylamine (1.0 g, 7.83 mmol, 4.0 eq) were added, and the mixture was stirred at 25°C for 1 hour. The reaction was monitored by LCMS. After completion, the mixture was concentrated, and 40 mL of ethyl acetate/petroleum ether (1/2, v/v) was added for slurrying. The residue was filtered, and the filtrate was concentrated. The concentrate was purified by Prep-HPLC (0.1% formic acid solution/acetonitrile) and lyophilized to obtain Compound **3** (1.3 g, 90%) as a colorless liquid. LCMS (ESI) [M+H]⁺ = 739.4.

### Step 2: Preparation of Compound 4

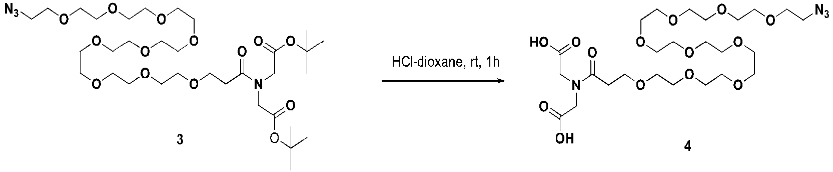

Hydrogen chlar-dioxane solution (6 M, 20 mL) was added to Compound **3** (1.3 g, 1.80 mmol), and the mixture was stirred at 25°C for 1 hour. The reaction was monitored by LCMS. After completion, the solvent was removed. The residue was diluted with dioxyhexyl (20 mL) and concentrated three times, then dried to obtain crude Compound **4** (1.1 g, 100%) as a yellow liquid. LCMS (ESI) [M+H]⁺ = 627.3.

### Step 3: Preparation of Compound 6

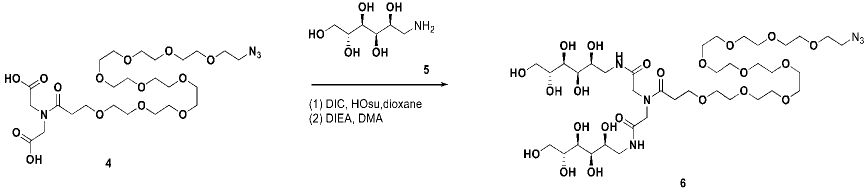

Compound **4** (900 mg, 1.44 mmol, 1.0 eq) was dissolved in 10 mL of anhydrous dioxane, DIC (544 mg, 4.32 mmol, 3.0 eq) and HOSu (497 mg, 4.32 mmol, 3.0 eq) were added, and the mixture was stirred at 25°C for 2 hours. Then N,N-Dimethylacetamide (5 mL), N,N-Diisopropylethylamine (928 mg, 7.2 mmol, 5.0 eq), and Compound **5** (912 mg, 5.04 mmol, 3.5 eq) were added, and the mixture was stirred at 25°C for 1 hour. The reaction was monitored by LCMS. After completion, the mixture was concentrated. The residue was purified by Prep-HPLC (0.1% formic acid in water/acetonitrile) and lyophilized to obtain Compound **6** (230 mg, 17%) as a white solid. LCMS (ESI) [M+H]⁺ = 953.4.

### Step 4: Preparation of Compound 9

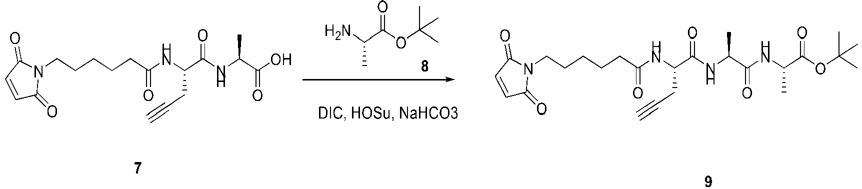

Compound **7** (300 mg, 0.79 mmol, 1.0 eq) was dissolved in 5 mL of anhydrous dioxane. DIC (200 mg, 1.58 mmol, 2.0 eq) and HOSu (183 mg, 1.58 mmol, 2.0 eq) were added, and the mixture was stirred at 20°C for 2 hours. Then a saturated NaHCO₃ solution (1 mL) and a solution of Compound **8** (173 mg, 1.19 mmol, 1.5 eq) in N,N-Dimethylacetamide (2 mL) were added. The mixture was stirred at 20°C for 1 hour. The reaction was monitored by LCMS. After completion, the mixture was concentrated. The residue was purified by Prep-HPLC (0.1% formic acid in water/acetonitrile) and lyophilized to obtain Compound **9** (250 mg, 62%) as a white solid. LCMS (ESI) [M+H-56]⁺ = 449.2.

### Step 5: Preparation of Compound 10

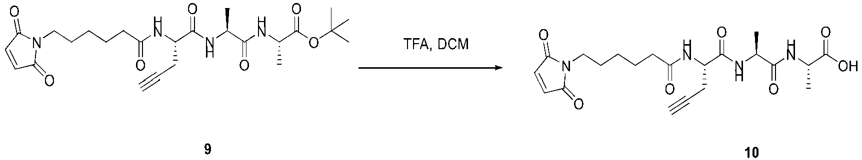

Compound **9** (200 mg, 0.40 mmol, 1.0 eq) was dissolved in 5 mL of dichloromethane, and then trifluoroacetic acid (2 mL) was added. The reaction mixture was stirred at 20°C for 1 hour. The reaction was monitored by LCMS. After completion, the mixture was concentrated. The residue was purified by Prep-HPLC (0.1% formic acid in water/acetonitrile) and lyophilized to obtain Compound **10** (120 mg, 68%) as a white solid. LCMS (ESI) [M+H]⁺ = 449.2.

### Step 6: Preparation of Compound 14

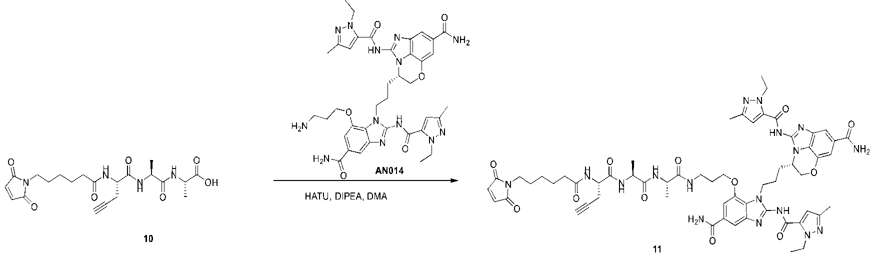

Compound **10** (76 mg, 0.17 mmol, 1.2 eq) and Compound **AN014** (110 mg, 0.14 mmol, 1.0 eq) were dissolved in 2 mL of N,N-Dimethylacetamide. Then HATU (64 mg, 0.17 mmol, 1.2 eq) and N,N-Diisopropylethylamine (73 mg, 0.68 mmol, 4.0 eq) were added. The reaction mixture was stirred at 20°C for 1 hour. The reaction was monitored by LCMS. After completion, the reaction mixture was purified by Prep-HPLC (0.1% formic acid in water/acetonitrile) and lyophilized to obtain Compound **11** (90 mg, 53%) as a white solid. LCMS (ESI) [M+H]⁺ = 1210.4.

### Step 7: Preparation of Compound A1 (L1-AN014)

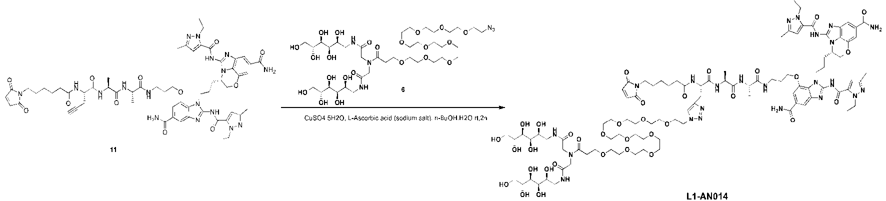

Compound **11** (80 mg, 0.066 mmol, 1.0 eq) and Compound **6** (94 mg, 0.099 mmol, 1.5 eq) were dissolved in a mixed solvent of n-butanol/water (10/1, v/v, 11 mL). Then, under nitrogen protection, sodium L-ascorbate (65 mg, 0.33 mmol, 5.0 eq) and copper sulfate pentahydrate (50 mg, 0.20 mmol, 3.0 eq) were added sequentially. The reaction mixture was stirred at 20°C under nitrogen protection for 2 hours. The reaction was monitored by LCMS. After completion, the mixture was concentrated. The residue was purified by Prep-HPLC (0.1% formic acid in water/acetonitrile) and lyophilized to obtain Compound **L1-AN014** (12.11 mg, 8%) as a white solid. LCMS (ESI) [1/2M+H]⁺ = 1082.4.

The LogS parameters of Compound 11 and Compound A1 were calculated and compared using PerkinElmer CHEMDRAW 22.2 software. The LogS value for Compound 11 was -10.02, while for Compound A1 it was -5.756, indicating an improvement in water solubility by nearly 4-5 orders of magnitude.

### Example 2 Synthesis of Linker-KRAS^{G12D} Inhibitor Compound A2 (TM-4)

### Step 1: Preparation of Compound 3

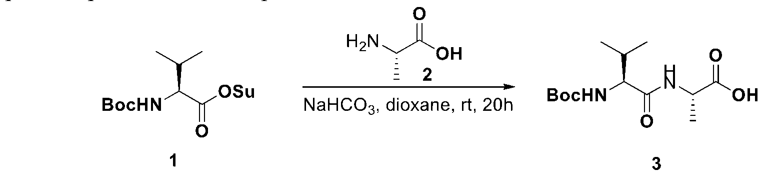

Compound **1** (3.00 g, 9.55 mmol, 1.0 eq) and Compound **2** (1.02 g, 11.47 mmol, 1.2 eq) were dissolved in 50 mL of dioxane, an aqueous solution (10 mL) of sodium bicarbonate (1.6 g, 19.1 mmol, 2.0 eq) was added, and the mixture was stirred at 20°C for 20 hours. The reaction was monitored by LCMS. After completion, the dioxane solvent was removed. The residue was diluted with ethyl acetate (50 mL) and washed with water (50 mL). The aqueous phase was acidified to an acidic pH with an aqueous citric acid solution, then extracted with ethyl acetate (50 mL × 3). The organic phase was washed with saturated brine, dried using anhydrous sodium sulfate, and concentrated under reduced pressure to obtain crude Compound **3** (3.0 g) as a colorless liquid. LCMS (ESI) [M+Na]⁺ = 311.1.

### Step 2: Preparation of Compound 5

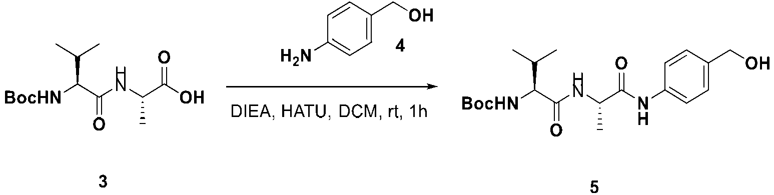

Compound **3** (3.00 g, 10.41 mmol, 1.0 eq) and Compound **4** (1.28 g, 10.41 mmol, 1.0 eq) were dissolved in 70 mL of dichloromethane. HATU (4.75 g, 12.49 mmol, 1.2 eq) and N,N-Diisopropylethylamine (5.38 g, 41.62 mmol, 4.0 eq) were added, and the mixture was stirred at 20°C for 1 hour. The reaction was monitored by LCMS. After completion, the solvent was removed, and the crude product was purified by column chromatography (dichloromethane:methanol = 15:1) to obtain crude Compound **5** (4.2 g, 100%) as a white solid. LCMS (ESI) [M+Na]⁺ = 416.3.

### Step 3: Preparation of Compound6

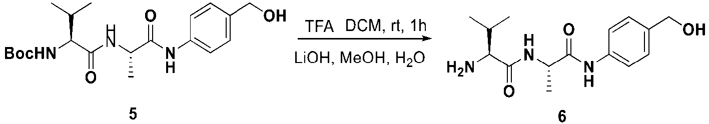

Compound **5** (4.2 g, 10.69 mmol, 1.0 eq) was dissolved in 60 mL of dichloromethane. Trifluoroacetic acid (20 mL) was added, and the mixture was stirred at 20°C for 1 hour. The mixture was then concentrated under reduced pressure. The residue was dissolved in 50 mL of methanol, and a saturated lithium hydroxide solution was added until the reaction mixture reached basic pH. The mixture was stirred at 20°C for 1 hour. The reaction was monitored by LCMS. After completion, the mixture was concentrated. The concentrate was purified by Prep-HPLC (0.1% ammonia solusion/acetonitrile) and lyophilized to obtain Compound 7 (2.2 g, 70%) as a yellow solid. LCMS (ESI) [M+H]⁺ = 294.1.

### Step 4: Preparation of Compound 16

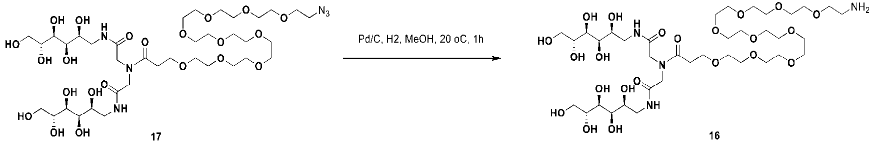

Compound **17** (100 mg, 0.11 mmol, 1.0 eq) was dissolved in 5 mL of methanol. Then, Pd/C (10%, 20 mg) was added, and the mixture was stirred under a hydrogen atmosphere at 20°C for 1 hour. The reaction was monitored by LCMS. After completion, the mixture was concentrated to obtain Compound **16** (90 mg, 93%) as a white solid.

### Step 5: Preparation of Compound 9

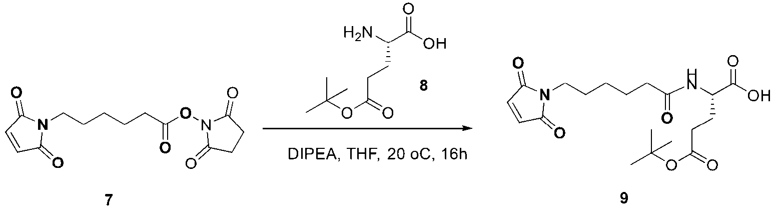

Compound **7** (1.00 g, 3.25 mmol, 1.0 eq) and Compound **8** (0.66 g, 3.25 mmol, 1.0 eq) were dissolved in 20 mL of tetrahydrofuran. Then N,N-Diisopropylethylamine (1.26 g, 9.75 mmol, 3.0 eq) was added, and the mixture was stirred at 20°C for 16 hours. The reaction was monitored by LCMS. After completion, the mixture was concentrated. The concentrate was purified by Prep-HPLC (0.1% formic acid in water/acetonitrile) and lyophilized to obtain Compound **9** (1.2 g, 93%) as a white solid. LCMS (ESI) [M+H-56]⁺ = 341.1.

### Step 6: Preparation of Compound 10

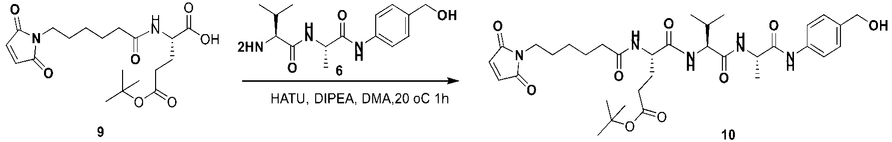

Compound **9** (500 mg, 1.26 mmol, 1.0 eq) and Compound **6** (370 mg, 1.26 mmol, 1.0 eq) were dissolved in 5 mL of N,N-Dimethylacetamide. Then HATU (575 mg, 1.51 mmol, 1.2 eq) and N,N-Diisopropylethylamine (489 mg, 3.78 mmol, 3.0 eq) were added. The reaction mixture was stirred at 20°C for 1 hour. The reaction was monitored by LCMS. After completion, the reaction mixture was purified by Prep-HPLC (0.1% formic acid in water/acetonitrile) and lyophilized to obtain Compound **10** (420 mg, 50%) as a white solid. LCMS (ESI) [M+H-18]⁺ = 654.3.

### Step 7: Preparation of Compound 12

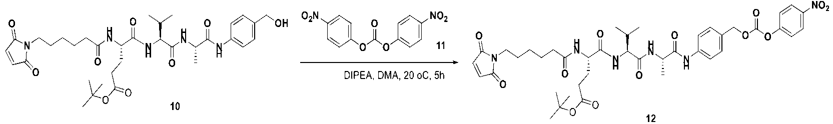

Compound **10** (150 mg, 0.22 mmol, 1.0 eq) and Compound **11** (136 mg, 0.44 mmol, 2.0 eq) were dissolved in 3 mL of N,N-Dimethylacetamide. Then N,N-Diisopropylethylamine (87 mg, 0.67 mmol, 5.0 eq) was added. The reaction mixture was stirred at 20°C for 5 hours. The reaction was monitored by LCMS. After completion, the mixture was extracted with ethyl acetate, washed with water and saturated saline. The organic phase was dried using anhydrous sodium sulfate, filtered, and concentrated. The residue was purified by silica gel column chromatography (dichloromethane/methanol = 15/1) to obtain Compound **12** (95 mg, 51%) as an orange-red solid. LCMS (ESI) [M+H-56]⁺ = 781.3.

### Step 8: Preparation of Compound 14

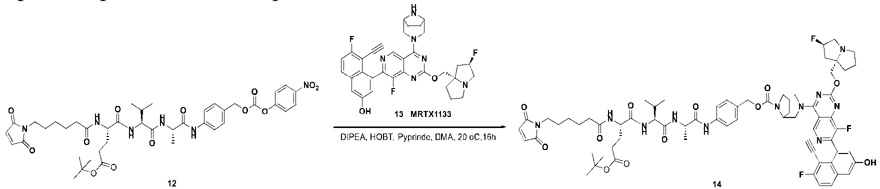

Compound **12** (95 mg, 0.11 mmol, 1.0 eq) and Compound **13** (68 mg, 0.11 mmol, 1.0 eq) were dissolved in 2 mL of N,N-Dimethylacetamide and 0.2 mL of pyridine. 1-Hydroxybenzotriazole (15 mg, 0.11 mmol, 1.0 eq) and N,N-Diisopropylethylamine (71 mg, 0.67 mmol, 5.0 eq) were added. The reaction mixture was stirred at 20°C for 16 hours. The reaction was monitored by LCMS. After completion, the reaction mixture was purified by Prep-HPLC (0.1% formic acid in water/acetonitrile) and lyophilized to obtain Compound **6** (90 mg, 61%) as a yellow solid. LCMS (ESI) [M+H]⁺ = 1298.5.

### Step 9: Preparation of Compound 15

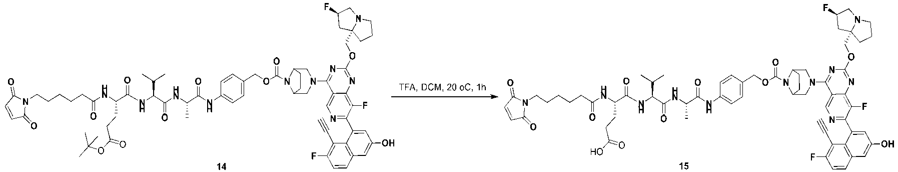

Compound **14** (90 mg, 0.07 mmol) was dissolved in 5 mL of dichloromethane, and trifluoroacetic acid (1 mL) was added. The reaction mixture was stirred at 20°C for 1 hour. The reaction was monitored by LCMS. After completion, the solvent was removed. The residue was purified by Prep-HPLC (0.1% formic acid in water/acetonitrile) and lyophilized to obtain Compound **15** (45 mg, 52%) as a yellow solid. LCMS (ESI) [M+H]⁺ = 1243.4.

### Step 10: Preparation of Compound A2 (TM-4)

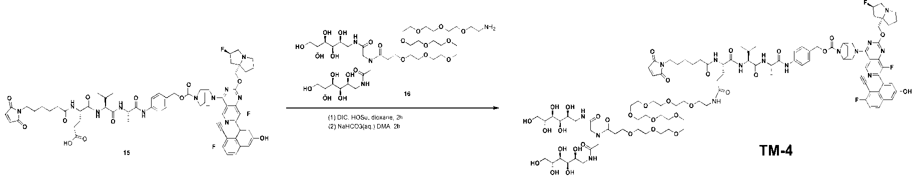

Compound **15** (45 mg, 0.036 mmol, 1.0 eq) was dissolved in 2 mL of anhydrous dioxane. DIC (23 mg, 0.181 mmol, 5.0 eq) and HOSu (21 mg, 0.181 mmol, 5.0 eq) were added, and the mixture was stirred at 20°C for 2 hours. Then, a saturated NaHCO₃ solution (0.2 mL) and a solution of Compound **16** (173 mg, 1.19 mmol, 1.5 eq) in N,N-Dimethylacetamide (1 mL) were added. The mixture was stirred at 20°C for 2 hours. The reaction was monitored by LCMS. After completion, the mixture was concentrated. The residue was purified by Prep-HPLC (0.1% formic acid in water/acetonitrile) and lyophilized to obtain Compound **TM-4** (11.02 mg, 14%) as a yellow solid. LCMS (ESI) [1/2M+H]⁺ = 1076.4.

The LogS parameters of Compound 15 and Compound A2 were calculated and compared using PerkinElmer CHEMDRAW 22.2 software. The LogS value for Compound 15 was -11.14, while for Compound A2 it was -6.926, indicating an improvement solusion solubility by nearly 4 orders of magnitude.

### Example 3 Synthesis of Linker-Topo1 Inhibitor Compound A4 (FD-LP1)

### Step 1: Synthesis of compound 5

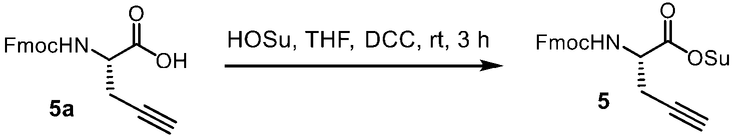

Compound **5a** (200 mg, 0.596 mmol, 1.0 eq), HOSu (103.0 mg, 0.895 mmol, 1.5 eq), and DCC (246.1 mg, 1.193 mmol, 2.0 eq) were dissolved in tetrahydrofuran (5 mL). The reaction mixture was stirred at room temperature for 3 hours. After the reaction was complete monitored by LC-MS, the mixture was directly concentrated. The residue was purified by silica gel column chromatography (DCM/MeOH = 93/7) to obtain Compound **5** (160 mg, 37%) as a white solid. LCMS: [M+Na]⁺ = 455.1.

### Step 2: Synthesis of Compound 3

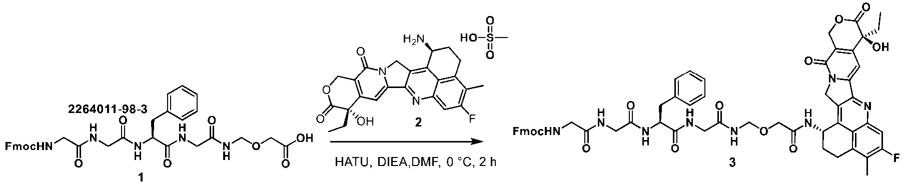

Compound **2** (247.5 mg, 0.466 mmol, 1.0 eq) and diisopropylethylamine (180.5 mg, 1.397 mmol, 3.0 eq) were dissolved in DMF (5 mL). After stirred until clear, Compound **1** (300 mg, 0.466 mmol, 1.0 eq) and N,N,N',N' -tetramethyl-O -(7-aza-benzotriazol-1-yl) hexafluoride phosphate (283.3 mg, 0.745 mmol, 1.6 eq) were added. After addition, the reaction mixture was stirred at 0°C for 2 hours. After the reaction was complete monitored by LC-MS, the reaction mixture was directly subjected to reverse-phase purification (0.1% FA in water/acetonitrile = 2/3) to obtain Compound 3 (420 mg, 81%) as a yellow solid. LCMS: [M+H]⁺ = 1063.3.

### Step 3: Synthesis of Compound 4

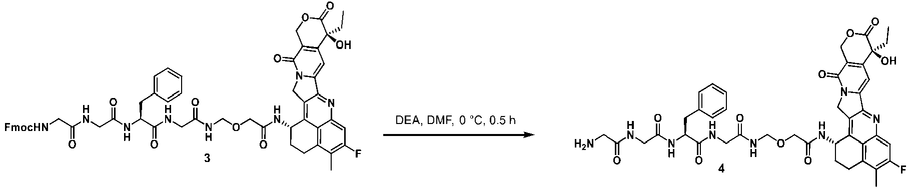

Compound **3** (420 mg, 0.395 mmol, 1.0 eq) was dissolved in DMF (4 mL). The temperature was lowered to 0°C, and diethylamine (0.4 mL) was added. After addition, the reaction mixture was stirred at 0°C for half an hour. After the reaction was complete monitored by LC-MS, the organic phase was concentrated to remove diethylamine. Acetic acid (420 mg) dissolved in a small amount of DMF was added to the reaction system and shaken to mix. The reaction system was directly subjected to reverse-phase purification (0.1% FA in water/acetonitrile = 7/3) to obtain Compound **4** (290 mg, 83%) as a yellow solid. LCMS: [M+H]⁺ = 841.3.

### Step 4: Synthesis of Compound 6

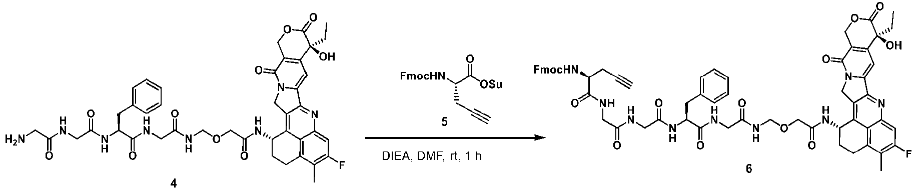

Compound **4** (170 mg, 0.202 mmol, 1.0 eq), Compound **5** (104.9 mg, 0.243 mmol, 1.2 eq), and diisopropylethylamine (52.3 mg, 0.404 mmol, 2.0 eq) were dissolved in DMF (5 mL). After addition, the reaction mixture was stirred at room temperature for 1 hour. After the reaction was complete monitored by LC-MS, the reaction mixture was directly subjected to reverse-phase purification (0.1% HCOOH in water/acetonitrile = 1/1) to obtain Compound **6** (190 mg, 80%) as a white solid. LCMS: [M+H]⁺ = 1158.4.

### Step 5: Synthesis of Compound 7

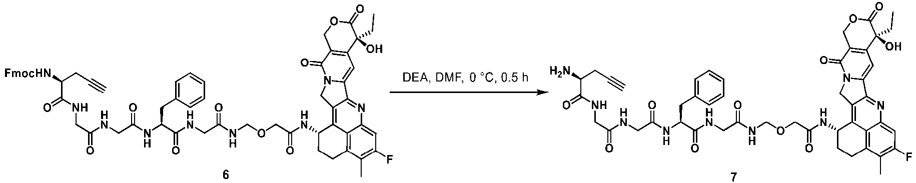

Compound **6** (180 mg, 0.156 mmol, 1.0 eq) was dissolved in DMF (3 mL). The temperature was lowered to 0°C, and diethylamine (0.3 mL) was added. After addition, the reaction mixture was stirred at 0°C for half an hour. After the reaction was complete monitored by LC-MS, the organic phase was concentrated to remove diethylamine. Acetic acid (420 mg) dissolved in a small amount of DMF was added to the reaction system and shaken to mix. The reaction system was directly subjected to reverse-phase purification (0.1% HCOOH in water/acetonitrile = 7/3) to obtain Compound **7** (120 mg, 81%) as a white solid. LCMS: [M+H]⁺ = 936.2.

### Step 6: Synthesis of Compound 9

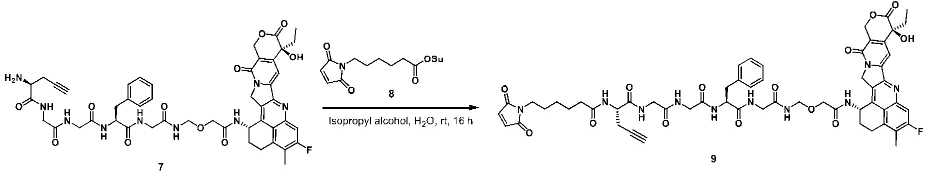

Compound **7** (230 mg, 0.2458 mmol, 1.0 eq) and Compound **8** (113.6 mg, 0.3690 mmol, 1.5 eq) were dissolved in isopropanol:water = 3:2 (10 mL). After addition, the reaction mixture was stirred at room temperature for 16 hours. After the reaction was complete monitored by LC-MS, the reaction mixture was purified by Prep-HPLC (0.1% FA in water/acetonitrile = 7/3) to obtain Compound **9** (85 mg, 31%) as a white solid. LCMS: [M+H]⁺ = 1129.3.

### Step 7: Synthesis of compound FD-LP1

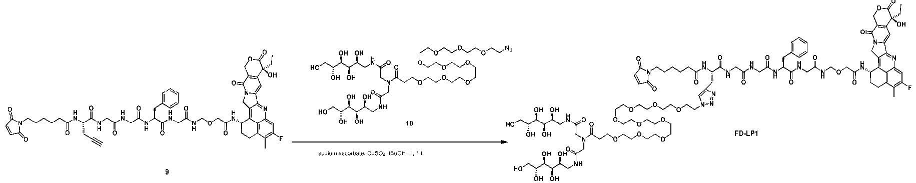

Compound **9** (85 mg, 0.0753 mmol, 1.0 eq) and Compound **10** (107 mg, 0.1129 mmol, 1.5 eq) were dissolved in tert-butanol:water = 1:1 (10 mL). Sodium L-ascorbate (74 mg, 0.3764 mmol, 5.0 eq) and copper sulfate pentahydrate (56 mg, 0.2258 mmol, 3.0 eq) were added. After addition, the reaction mixture was stirred under a nitrogen atmosphere at room temperature for 1 hour. After the reaction was complete monitored by LC-MS, the mixture was directly purified by Prep-HPLC (0.1% FA in water/acetonitrile = 7/3) to obtain Compound **FD-LP1** (100 mg, 64%) as a light yellow solid. LCMS: [M/2+H]⁺ = 1041.5 (half peak).

### Example 4 Synthesis of Linker-Topo1 Inhibitor Compound A9 (FD-LP5)

### Step 1: Synthesis of Compound 3

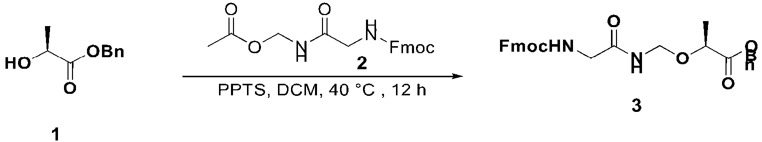

At 25°C, Compound **1** (1 g, 2.70 mmol) was dissolved in anhydrous dichloromethane (50 mL). Compound **2** (1.46 g, 8.10 mmol) was added, followed by **PPTS** (0.34 g, 1.35 mmol). The mixture was stirred at 45°C for 16 hours, then concentrated under reduced pressure to obtain the crude product. The crude product was purified by normal phase silica gel column (ethyl acetate:petroleum ether = 0% - 100%) to obtain Compound 3 (1 g, 66.67%) as a white solid. MS m/z (ESI): 511.1 (M+Na)⁺.

### Step 2: Synthesis of Compound 4

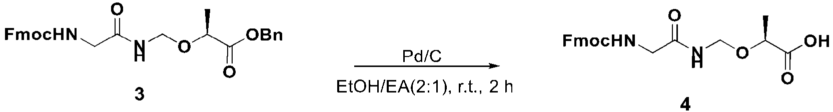

At 25°C, Compound **3** (1 g, 2.04 mmol) was dissolved in ethanol (20 mL) and ethyl acetate (10 mL). Palladium on carbon (1.18 g, 10% loading) was added. The atmosphere was replaced with hydrogen three times, and the reaction was maintained under a hydrogen atmosphere at room temperature for 2 hours. The reaction mixture was filtered and concentrated. The concentrate was purified by Prep-HPLC (water/acetonitrile) and lyophilized to obtain Compound **4** (0.55 g, 62.16%) as a white solid. MS m/z (ESI): 421.1 (M+23)⁺.

### Step 3: Synthesis of Compound 6

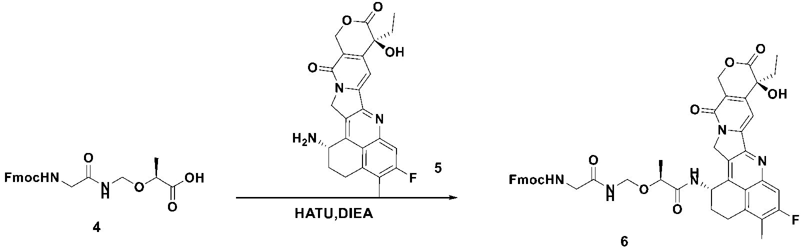

At 25°C, Compound **4** (550 mg, 1.38 mmol) was dissolved in DMF (10 mL). Under an ice bath, Compound **5** (462 mg, 1.79 mmol), HATU (605 mg, 2.07 mmol), and N,N-Diisopropylethylamine (411 mg, 4.14 mmol) were added. The resulting reaction mixture was stirred under the ice bath for 1 hour. The organic phase was concentrated under reduced pressure to obtain the crude product. The crude product was purified by normal phase silica gel column (ethyl acetate:petroleum ether = 0% - 100%) to obtain Compound **6** (277 mg, 28.83%) as a white solid. MS m/z (ESI): 816.2 (M+1)⁺.

### Step 4: Synthesis of Compound 7

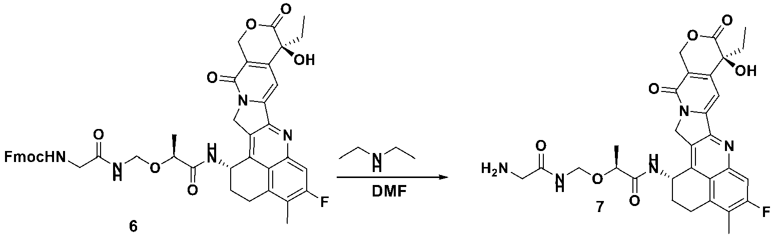

At 25°C, Compound **6** (277 mg, 0.34 mmol) was dissolved in DMF (20 mL). Diethylamine (2 mL) was added at room temperature, and the reaction was stirred at room temperature for 0.5 hours. The mixture was concentrated under reduced pressure. The concentrate was purified by Prep-HPLC (water/acetonitrile) and lyophilized to obtain Compound **7** (138 mg, 62.03%) as a white solid. MS m/z (ESI): 594.2 (M+1)⁺.

### Step 5: Synthesis of Compound 9

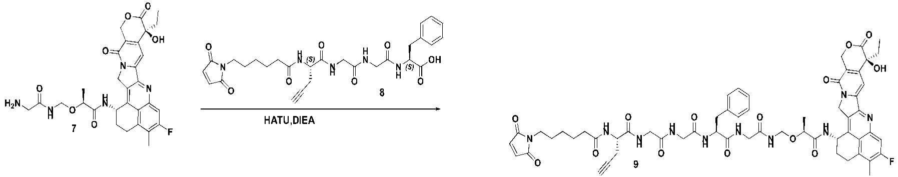

At 25°C, Compound **7** (120 mg, 0.15 mmol) was dissolved in DMF (10 mL). Under an ice bath, Compound **8** (116 mg, 0.15 mmol), HATU (92 mg, 0.18 mmol), and N,N-Diisopropylethylamine (80 mg, 0.45 mmol) were added. The resulting reaction mixture was stirred under the ice bath for 1 hour. The reaction mixture was purified by Prep-HPLC (1% FA in water/acetonitrile) and lyophilized to obtain Compound **9** (80 mg, 31.09%) as a white solid. MS m/z (ESI): 1143.2 (M+1)⁺.

### Step 6: Synthesis of compound FD-LP5

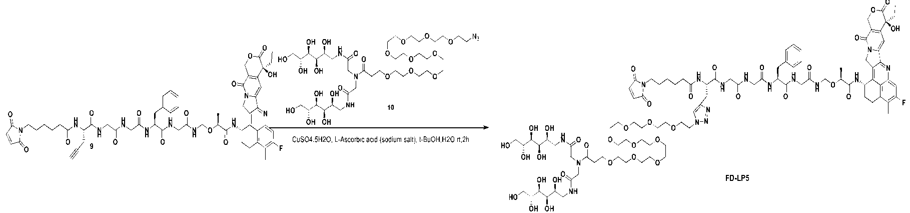

Compound **9** (80 mg, 0.066 mmol) and Compound **10** (100 mg, 0.10 mmol) were dissolved in a mixed solvent of tert-butanol/water (10/1, 11 mL). Then under nitrogen protection, sodium L-ascorbate (69 mg, 0.34 mmol) and copper sulfate pentahydrate (52 mg, 0.21 mmol) were added sequentially. The reaction mixture was stirred at 20°C under nitrogen protection for 0.5 hours. The reaction was monitored by LCMS. After completion, the reaction mixture was purified by Prep-HPLC (0.1% formic acid in water/acetonitrile) and lyophilized to obtain Compound **FD-LP5** (10 mg, 7%) as a white solid. LCMS (ESI) [1/2M+H]⁺ = 1048.1.

### Example 5: Synthesis of Linker-Topo1 Inhibitor Compound A10 (FD-LP6)

### Step 1: Synthesis of Compound 2

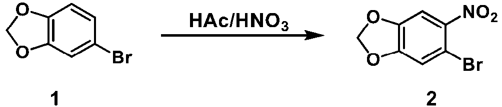

In an ice bath, Compound **1** (10.0 g, 0.0497 mol, 1.0 eq) was dissolved in acetic acid (60 mL). Concentrated nitric acid (20 mL) was slowly added. The reaction mixture was stirred at 0°C for 1 hour. After the reaction was complete monitored by LC-MS, the mixture was poured into ice water. A yellow solid precipitated, which was filtered, and the filter cake was dried to obtain Compound **2** (12.0 g, 90% purity, 88%) as a yellow solid. LCMS: [M+H]⁺ = 245.9.

### Step 2: Synthesis of Compound 4

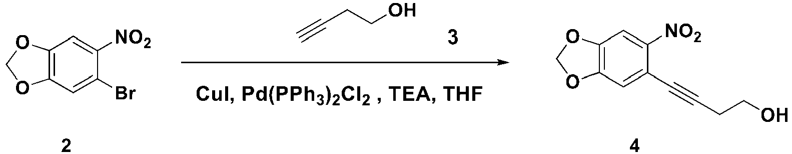

Compound **2** (5.0 g, 0.0203 mol, 1.0 eq) and Compound **3** (1.7 g, 0.0244 mol, 1.2 eq) were dissolved in DMF (60 mL). CuI (0.8 g, 0.0041 mol, 0.2 eq), bis(triphenylphosphine)palladium chloride (2.1 g, 0.0030 mol, 0.15 eq), and triethylamine (10.3 g, 0.1015 mol, 5.0 eq) were added. The mixture was reacted at 50°C for 4 hours. After the reaction was complete monitored by LC-MS, the mixture was extracted with ethyl acetate. The organic phase was collected and dried using anhydrous sodium sulfate. After concentration, the product was purified by column chromatography (PE:EA = 1:1) to obtain Compound **4** (4 g, 85% purity, 71%) as a brown solid. LCMS: [M+H]⁺ = 236.1.

### Step 3: Synthesis of compound 5

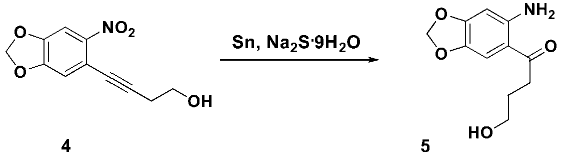

In an ice bath, Compound **4** (4.0 g, 0.0170 mol, 1.0 eq) was dissolved in EtOH:H₂O = 7:1 (80 mL). Sodium sulfide hydrate (1.2 g, 0.0051 mol, 0.3 eq) and tin powder (4.0 g, 0.0340 mol, 2.0 eq) were added. Concentrated hydrochloric acid (7 mL, 0.0850 mol, 5 eq) was slowly added dropwise. The mixture was reacted at 55°C for 1 hour. After the reaction was complete monitored by LC-MS, the hot reaction mixture was filtered through diatomaceous earth and washed with a small amount of ethanol. The filtrates were combined and concentrated to dry. The residue was dissolved in ethyl acetate and slowly diluted into an aqueous sodium carbonate solution. The pH was adjusted to 8. The mixture was filtered, and the filtrate was separated. The organic phase was concentrated and purified by column chromatography (DCM:MeOH = 20:1) to obtain Compound 5 (1.6 g, 70% purity, 29%) as a brown solid. LCMS: [M+H]⁺ = 224.2.

### Step 4: Synthesis of Compound 7

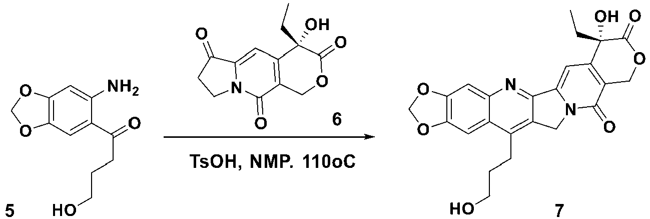

Compound **5** (1.1 g, 0.0049 mol, 1.0 eq) and Compound **6** (0.9 g, 0.0034 mol, 0.7 eq) were dissolved in NMP (10 mL). p-toluenesulfonic acid monohydrate (0.93 g, 0.0049 mol, 1 eq) was added. After addition, the reaction mixture was stirred at 110°C for 2 hours. After the reaction was complete monitored by LC-MS, the mixture was directly concentrated and purified by column chromatography (DCM:MeOH = 1:20) to obtain Compound 7 (0.6 g, 90% purity, 24%) as a brown oily solid. LCMS: [M+H]⁺ = 451.1.

### Step 5: Synthesis of Compound 9

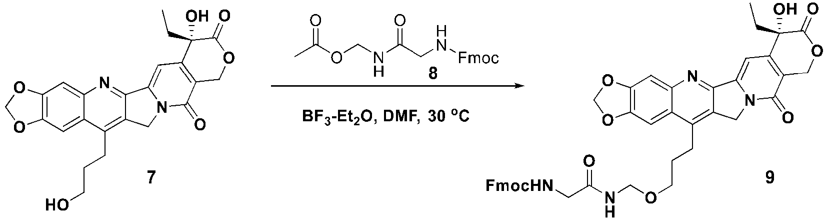

In an ice bath, Compound **7** (600.0 mg, 1.332 mmol, 1.0 eq) and Compound **8** (2.5 g, 6.660 mmol, 5 eq) were dissolved in DMF (10 mL). Boron trifluoride diethyl etherate (378.1 mg, 2.664 mmol, 2 eq) was added. After addition, the reaction mixture was stirred at 30°C for 1 hour. After the reaction was complete monitored by LC-MS, a small amount of ice water was added to quench the reaction. The mixture was directly concentrated and purified by column chromatography (DCM:MeOH = 1:20) to obtain the crude product, which was further purified by reverse-phase column (0.1% FA in water/acetonitrile = 1/1) to obtain Compound **9** (400 mg, 90% purity, 35%) as a brown solid. LCMS: [M+H]⁺ = 759.2.

### Step 6: Synthesis of Compound 11

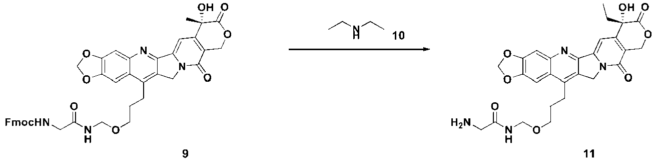

Compound **9** (400.0 mg, 0.5272 mmol, 1.0 eq) was dissolved in DMF (5 mL). Diethylamine (462.7 mg, 6.3264 mmol, 12.0 eq) was added at room temperature, and the reaction mixture was stirred for 0.5 hours. After the reaction was complete monitored by LC-MS, the mixture was passed through a reverse-phase column (0.1% FA in water/acetonitrile = 9/1) to remove excess diethylamine, then eluted with (water/acetonitrile = 7/3) to obtain Compound **11** (307 mg, 90% purity, 97%) as a brown solid. LCMS: [M+H]⁺ = 537.3.

### Step 7: Synthesis of compound 13

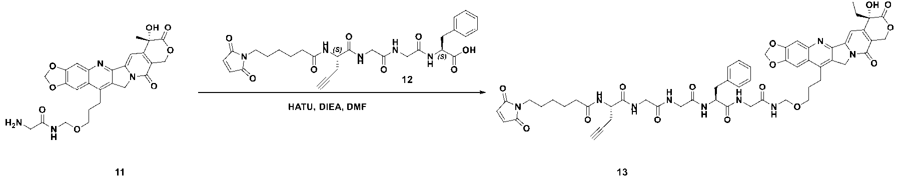

Compound **11** (240.0 mg, 0.4473 mmol, 1.0 eq) and Compound **12** (330.0 g, 0.5815 mmol, 1.3 eq) were dissolved in DMF (30 mL). *N,N,N',N'*-tetramethyl-O -(7-aza-benzotriazol-1-yl) hexafluoride phosphate (204.1 mg, 0.5368 mmol, 1.2 eq) and N,N-Diisopropylethylamine (173.4 mg, 1.3419 mmol, 3 eq) were added. After addition, the reaction mixture was stirred at room temperature for 0.5 hours. After the reaction was complete monitored by LC-MS, the reaction mixture was purified by Prep-HPLC (0.1% FA in water/acetonitrile = 7/3) to obtain Compound 13 (60 mg, 11%) as a yellow solid. LCMS: [M+H]⁺ = 1086.3.

### Step 8: Synthesis of compound FD-LP6

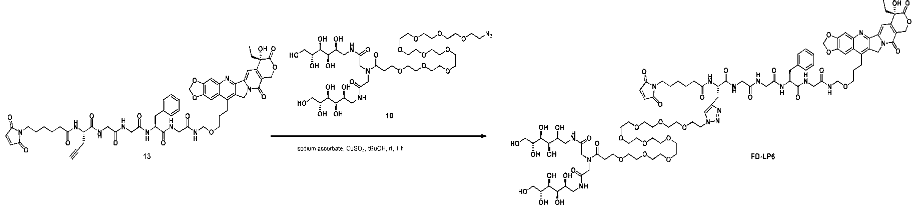

Compound 13 (60.0 mg, 0.0552 mmol, 1.0 eq) and Compound 10 (78.9 mg, 0.0828 mmol, 1.5 eq) were dissolved in tert-butanol:water = 1:1 (10 mL). Sodium L-ascorbate (54.7 mg, 0.2760 mmol, 5.0 eq) and copper sulfate pentahydrate (41.4 mg, 0.1656 mmol, 3.0 eq) were added. After addition, the reaction mixture was stirred under a nitrogen atmosphere at room temperature for 1 hour. After the reaction was complete monitored by LC-MS, the mixture was directly purified by Prep-HPLC (0.1% FA in water/acetonitrile = 7/3) to obtain Compound **FD-LP6** (13 mg, 8%) as a yellow solid. LCMS: [M/2+H]⁺ = 1020.2 (half peak).

### Example 6 Preparation of TF Antibody, TF Nanobody Conjugates with L1-AN014 and TM-4

The heavy chain of the TF-targeting monoclonal antibody HuSC1-39 (from WO2018/036117 A1) is shown in SEQ ID NO: 10, and the light chain is shown in SEQ ID NO: 11.

The TF-targeting nanobody-Fc fusion protein is 4A02-FCWT (wherein the CDR sequences are shown in SEQ ID NOs: 1-3, respectively, the VHH sequence is shown in SEQ ID NO: 4, and the 4A02-FCWT fusion protein sequence is shown in SEQ ID NO: 5).

The antibody stock solution was exchanged into 50 mM PB/1.0 mM EDTA buffer (pH 7.0) using a G25 desalting column. 8 equivalents of TECP were added, and the mixture was stirred at 37°C for 2 hours to fully reduce the interchain disulfide bonds of the antibody. Subsequently, the pH of the reduced antibody solution was adjusted to 6.0 using phosphoric acid, and the water bath temperature was lowered to 25°C in preparation for the conjugation reaction. The linker-drug conjugates prepared according to the methods of Example 1 and Example 2 above were dissolved in DMA. 12 equivalents of the linker-drug conjugate solution were taken and added dropwise to the reduced antibody solution. DMA was added to achieve a final concentration of 10% (V/V). The reaction was stirred at 25°C for 0.5 hours. After completion, the sample was filtered through a 0.22 µm membrane. Excess unconjugated small molecules were removed using a tangential flow filtration system for purification, with 50 mM PB/1.0 mM EDTA solution (pH=6.0) as the buffer. After purification, sucrose was added to a final concentration of 6%, and the solution was stored at -20°C. The absorbance was measured at 280 nm and 370 nm using UV spectroscopy to calculate the DAR value.

For most linker-drug conjugates in this technical solution, no precipitation occurred during the conjugation process. The DAR values for the HuSC1-39 conjugates ADCs were between 7-8, and the DAR values for the 4A02-FCWT conjugates NDCs were between 3.9-4. The DAR values were determined by HIC-HPLC, RP-HPLC, or LCMS. The aggregate ratio of the conjugates detected by SEC-HPLC was within the normal range (purity >90%), indicating that the antibody-drug conjugates of the present invention have good solubility and druggability, with no precipitation occurring during the conjugation process. **Table 1** summarizes the preparation results of the four TF-ADC and TF-NDC conjugates.

**Table 1: Preparation Results of TF-STING ADC/NDC and TF-KRAS^{G12D}-Inhibitor ADC/NDC**

| TF-ADC/NDC | LC0d | LC1d | HC0D | HC1d | HC2d | HC3d | LC | HC | RP-DAR |
|---|---|---|---|---|---|---|---|---|---|
| HuSC1-39-L1-AN014 | 0.89 | 26.74 | 0 | 0 | 4.16 | 66.21 | 27.63 | 70.37 | 7.82 |
| 4A02-FCWT-L1-AN014 | NA | NA | 0 | 1.55 | 98.45 | NA | NA | 100 | 3.97 |
| HuSC1-39-TM-4 | 0.55 | 27.94 | 0.24 | 1.42 | 10.6 | 58.91 | 28.49 | 71.17 | 7.56 |
| 4A02-FCWT-TM-4 | NA | NA | 0 | 1.19 | 98.64 | NA | NA | 99.83 | 3.98 |

### Example 7 Preparation of TF Humanized Nanobody, HER2 Nanobody Conjugates with Linker-Topo1 Inhibitors FD-LP1, FD-LP5, FD-LP6

Using the TF-targeting humanized nanobody-Fc fusion protein 4A02-HM8-FCWT (code name FD40, sequence shown in SEQ ID NO: 6), conjugation reactions with FD-LP1, FD-LP5, and FD-LP6 were performed using the method described in **Example 6.** The conjugates were purified, and the DAR values were analyzed.

For most linker-drug conjugates in this technical solution, no precipitation occurred during the conjugation process. The DAR values for the 4A02-HM8-FCWT (FD40) conjugates (NDCs) were between 3.6-3.9. The DAR values were determined by HIC-HPLC, RP-HPLC, or LCMS. The aggregate ratio of the conjugates detected by SEC-HPLC was within the normal range (purity >90%), indicating that the antibody-drug conjugates of the present invention have good solubility and druggability, with no precipitation occurring during the conjugation process. **Table 2** summarizes the preparation results of the three TF-NDCs.

**Table 2: Preparation Results of TF-Topo1 Inhibitor NDCs**

| **Lot #1** | HC0d | HCld | HC2d | HC | RP-DAR |
|---|---|---|---|---|---|
| FD40-LP1 | 0.34 | 1.73 | 97.93 | 100 | 3.9 |
| FD40-LP5 | 0.85 | 1.75 | 97.39 | 100 | 3.9 |
| FD40-LP6 | 2.23 | 7.6 | 90.16 | 100 | 3.8 |

| **Lot #2** | HC0d | HC1d | HC2d | HC | RP-DAR |
|---|---|---|---|---|---|
| FD40-LP1 | 0.79 | 3.56 | 95.65 | 100 | 3.9 |
| FD40-LP5 | 0.62 | 1.99 | 97.39 | 100 | 3.9 |
| FD40-LP6 | 3.71 | 10.59 | 85.7 | 100 | 3.6 |

Similarly, using the HER2-targeting nanobody-Fc fusion protein 1-G07-FCWT (sequence shown in SEQ ID NO: 7), or the humanized HER2 nanobody-Fc fusion proteins 1-G07-HM1-FCWT and 1-G07-HM3-FCWT (sequences shown in SEQ ID NO: 8 and SEQ ID NO: 9, respectively), conjugation reactions with FD-LP5 were performed using the method described in **Example 6.** The conjugates were purified, and the DAR values were analyzed. **Table 3** shows the preparation results of the HER2-Topol Inhibitor NDCs.

**Table 3: Preparation Results of HER2-Topo1 Inhibitor NDCs**

| HER2-NDC | HC0d | HCld | HC2d | HC | RP-DAR |
|---|---|---|---|---|---|
| 1-G07-LP5 (Lot 1) | 4.49 | 19.26 | 76.25 | 100.0 | 3.4 |
| 1-G07-LP5 (Lot 2) | 4.7 | 19.34 | 75.97 | 100.0 | 3.4 |

### Example 8: Activity Assay of TF Antibody/Nanobody-L1-AN014 Conjugates (TF-STING ADC, NDC)

The cell lines used in this example included triple-negative breast cancer HCC1806 and MDA-MB-231, and pancreatic cancer cell lines BxPC-3 and HPAF-II, purchased from the American Type Culture Collection (ATCC) and the Cell Bank of the Chinese Academy of Sciences, and cultured according to the respective instructions. Cryopreserved vials of human peripheral blood mononuclear cells (PBMCs) were provided by Jiangsu Cidier Biotechnology Co., Ltd.

1x10⁴ tumor cells (using complete medium for PBMCs) were seeded into 96-well plates and cultured overnight. PBMCs were thawed separately in complete medium (RPMI1640 + 10% inactivated FBS + 1% penicillin/streptomycin + 1% sodium pyruvate + 1% GlutaMAX). The next day, 1x10⁵ PBMCs (PBMC:tumor cell = 10:1) were added to the 96-well plates for incubation. Simultaneously, the test drugs were serially diluted 5-fold in the complete medium used for PBMC culture to different concentrations. HuSC1-39, 4A02-FCWT, HuSC1-39-L1-AN014, and 4A02-FCWT-L1-AN014 all started at 150 µg/mL; AN014 and diABZI started at 1000 nM. The gradient-diluted test drugs were then added to the aforementioned 96-well plates. After incubation in a 37°C incubator for an appropriate time, the cell supernatant was collected. The survival ratio of tumor cells was detected using a firefly luciferase reporter gene detection kit or MTS reagent (MTS powder purchased from Promega, Cat# G1111; PMS powder purchased from Sigma, Cat# P9625).

Additionally, CXCL10 and Interferon-γ (IFN-γ) are classic markers of STING pathway activation, so the expression levels of CXCL10 (Cat#EK168, Lianke Biotech) and Interferon-γ (CAt#EK180, Lianke Biotech) in the co-culture supernatant were detected using ELISA kits to evaluate the activation effect of TF-STING ADC/NDC on the STING pathway.

As shown in **Figure 1****,** in the co-culture of triple-negative breast cancer MDA-MB-231 and human PBMCs for 48 hours, the naked antibodies HuSC1-39 and 4A02-FCWT slightly increased cytokine secretion compared to hIgG1, while the TF-STING ADC/NDCs HuSC1-39-L1-AN014 and 4A02-FCWT-L1-AN014 strongly induced the secretion of the chemokine CXCL10 **(****Figure 1A****)** and Interferon-γ **(****Figure 1B****),** with EC50 values of 0.01~0.05 nM. This indicates their activation of the STING pathway is far superior to 1000 nM diABZI.

As shown in **Figure 2****,** after the co-culture of pancreatic cancer BxPC-3 and human PBMCs for 48 hours, TF-STING ADC/NDCs also showed extremely potent anti-tumor effects. **Figure 2A** shows the killing EC₅₀ against BxPC-3 cells was <0.0002 nM and <0.0004 nM; **Figure 2B** shows that TF-STING ADC/NDCs strongly induced Interferon-γ secretion activity, with EC₅₀ values of approximately 1.12 nM and 1.21 nM.

Furthermore, as shown in **Figures 3 to 5****,** in the co-culture system of TF-high expressing tumor cells and PBMCs, TF-STING ADC/NDCs showed stronger and more effective tumor-killing activity compared to naked antibodies. The killing EC₅₀ against HCC1806 cells at 24 hours was 0.014~0.066 µg/mL **(****Figure 3****),** against MDA-MB-231 cells at 48 hours was approximately 0.0015 µg/mL **(****Figure 4****),** and against HPAF-II cells at 72 hours was approximately 0.01 µg/mL **(****Figure 5****).**

In the *in vivo* experiment, 5x10⁶ HPAF-II cells in the logarithmic growth phase were inoculated onto the backs of 6-week-old female Balb/c nude mice (purchased from Shanghai Sippr-BK Lab Animal Co., Ltd.). When the tumor volume reached 150-200 mm³, the animals were randomly grouped for treatment, with 8 tumors per group. As shown in **Figure 6****,** compared to the vehicle group or the small molecule payload alone treatment group, administration of 3 mg/kg 4A02-FCWT-L1-AN014 (once weekly for 2 doses) significantly inhibited tumor growth.

### Example 9 Activity Assay of TF Antibody/Nanobody-TM-4 Conjugates (TF-KRAS^{G12D} Inhibitor ADC/NDC)

The cell line HPAF-II used in this example was purchased from the Cell Bank of the Chinese Academy of Sciences. Cells in the logarithmic growth phase were seeded into 96-well cell culture plates at a density of 1000-2000 cells per well, 150 µL/well. After incubation at 37°C, 5% CO₂ for about 5 hours, different concentrations of TF-ADC/NDCs (15 µg/mL - 0.00019 µg/mL) were added. Each drug concentration was set up with 2-4 replicate wells, along with corresponding vehicle control and blank control wells. After culturing for several days (ensuring sufficient cell divisions based on cell growth rate), the culture medium was discarded, and MTS reagent (purchased from Promega, Cat# G3581) was added, 100 µL/well. The plates were incubated at 37°C until the expected color was reached. The cell viability (OD490nm) of each group was measured, and the cell survival rate was calculated according to the following formula: Survival rate = (ODdrug - ODblank) / (ODvehicle - ODblank) × 100%. The above data were analyzed using GraphPad Prism 8 software, and the IC₅₀ values of the above TF-NDCs on different cell lines were calculated.

The KRAS^{G12D} mutant pancreatic cancer cell line HPAF-II was used to validate the *in vitro* efficacy of TF-KRAS^{G12D} inhibitor ADC/NDC. Cells in the logarithmic growth phase were seeded into 96-well cell culture plates. Gradient-diluted HuSC1-39-TM-4 and 4A02-FCWT-TM-4 (15 µg/mL - 0.00019 µg/mL, n=3) were added separately. After 6 days of culture, the culture medium was discarded, and MTS reagent (purchased from Promega, Cat# G3581) was added to measure the cell viability (OD490nm) of each group.

As shown in **Figure 7****,** HuSC1-39-TM-4 (DAR=7.56) and 4A02-FCWT-TM-4 (DAR=3.98) inhibited the proliferation of HPAF-II cells in a dose-dependent manner, with IC₅₀ values of 0.0795 µg/mL and 0.0594 µg/mL, respectively.

In the *in vivo* experiment, 5x10⁶ HPAF-II cells were inoculated onto the backs of 6-week-old female Balb/c nude mice. On day 6, when the tumor volume reached 150-200 mm³, the animals were randomly grouped for treatment, with 8 tumors per group. As shown in **Figure 8****,** compared to the vehicle group or the naked antibody HuSC1-39 treatment group, administration of 10 mg/kg HuSC1-39-TM-4 (on days 6, 13, and 16) significantly inhibited tumor growth.

### Example 10 In Vitro Anti-tumor Activity of TF Nanobody-Topo1 Inhibitor Conjugates (TF-Topo1 Inhibitor NDC)

Refer to the *in vitro* anti-tumor activity assay method described in **Example 9.** MDA-453, HPAF-II, BxPC3, HCC1806, MDA-231, and NCI-H1373 cells in the logarithmic growth phase were seeded into 96-well cell culture plates at a density of 1000-3000 cells per well, 150 µL/well. After incubation at 37°C, 5% CO₂ for about 5 hours, different concentrations of TF-NDCs FD40-GGFG-Dxd, FD40-LP1, FD40-LP5, FD40-LP6 (15 µg/mL - 0.00019 µg/mL) were added. After 6 days, the culture medium was discarded, and MTS reagent (purchased from Promega, Cat# G3581) was added to measure the cell viability (OD490nm) of each group.

As shown in **Figure 9****,** FD40-GGFG-Dxd (DAR=4.0), FD40-LP1 (DAR=3.9), FD40-LP5 (DAR=3.9), and FD40-LP6 (DAR=3.8) inhibited the proliferation of TF-high expressing cell lines HPAF-II, BxPC3, HCC1806, MDA-231, and NCI-H1373 in a dose-dependent manner, with IC₅₀ values ranging from 0.0004 µg/mL to 0.0176 µg/mL. In contrast, the IC₅₀ values for the TF-negative cell line MDA-453 range from 4.5 µg/mL to 5.1 µg/mL. Therefore, in the *in vitro* proliferation assay, the TF-Topo1 inhibitor NDCs prepared in the present invention all demonstrated tumor cell killing effects dependent on the TF target.

### Example 11: In Vivo Efficacy of TF Nanobody-Topo1 Inhibitor Conjugates (TF-Topo1 Inhibitor NDC)

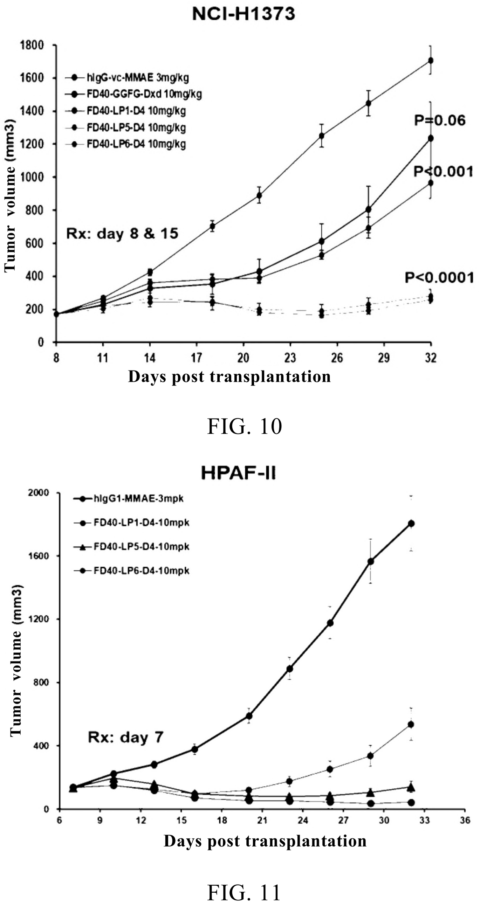

Lung cancer NCI-H1373 *in vivo* model: 5x10⁶ NCI-H1373 cells were inoculated onto the backs of 6-week-old female Balb/c nude mice. On day 8, when the tumor volume reached ~200 mm³, the animals were randomly grouped, with 10 tumors per group. Dosing was performed once weekly for a total of 2 doses (on days 8 and 15). As shown in **Figure 10****,** compared to the hIgG1-MMAE control group, administration of 10 mg/kg FD40-GGFG-Dxd (DAR=4.0), FD40-LP1 (DAR=3.9), FD40-LP5 (DAR=3.9), and FD40-LP6 (DAR=3.8) significantly inhibited tumor growth. On day 32 (17 days after the last dose) of the experiment, FD40-LP5 and FD40-LP6 maintained excellent tumor suppression effects. The therapeutic effect of FD40-LP1 (P<0.001) was also improved compared to FD40-GGFG-Dxd (P=0.06).

Pancreatic cancer HPAF-II *in vivo* model: 5x10⁶ HPAF-II cells were inoculated onto the backs of 6-week-old female Balb/c nude mice. On day 7, when the tumor volume reached ~150 mm³, the animals were randomly grouped for treatment, with 10 tumors per group, receiving a single dose. As shown in **Figure 11****,** compared to the hIgG1-MMAE control group, administration of 10 mg/kg FD40-LP1, FD40-LP5, and FD40-LP6 significantly inhibited tumor growth. The therapeutic effects of FD40-LP5 and FD40-LP6 were superior to that of FD40-LP1.

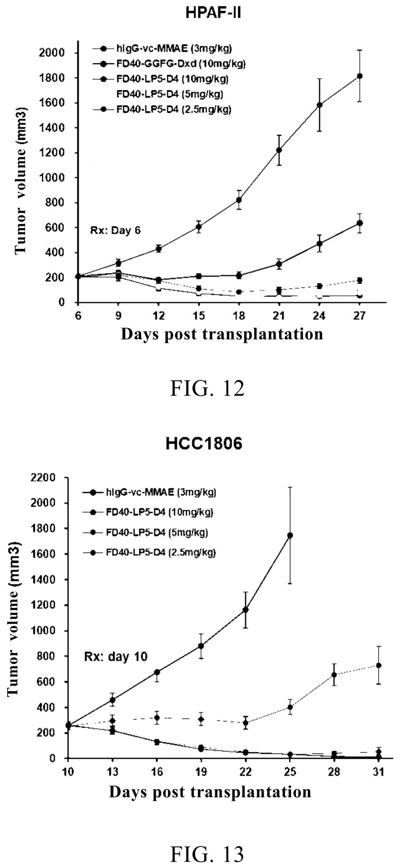

Similar pancreatic cancer HPAF-II in vivo model: on day 6, when the tumor volume reached ~200 mm³, the animals were randomly grouped for treatment, with 10 tumors per group, receiving a single dose. As shown in **Figure 12****,** compared to administration of 10 mg/kg FD40-GGFG-Dxd, FD40-LP5 (10 mg/kg, 5 mg/kg, 2.5 mg/kg) demonstrated superior anti-tumor therapeutic effects.

Triple-negative breast cancer (TNBC) HCC1806 model: HCC1806 cells in the logarithmic growth phase were inoculated into the mammary fat pads of 6-week-old female Balb/c nude mice at a density of 3×10⁶ cells per 200 µL of serum-free medium. When the tumor volume reached 200 mm³, the animals were randomly grouped, with 10 tumors per group, receiving a single dose. As shown in **Figure 13****,** the treatment groups administered with 10 mg/kg, 5 mg/kg, and 2.5 mg/kg FD40-LP5 all showed excellent or good tumor therapeutic effects.

### Example 12: In Vitro and In Vivo Efficacy of HER2 Nanobody-Topo1 Inhibitor Conjugate (1-G07-LPS) and Tumor Tissue Gene Transcriptome Analysis

Referring to the *in vitro* anti-tumor activity assay method in **Example 9,** NCI-N87 (2000 cells/well) and HCC1954 cells (1000 cells/well) in the logarithmic growth phase were seeded into 96-well cell culture plates, 150 µL/well. After incubation at 37°C, 5% CO₂ for about 5 hours, gradient diluted DS-8201/T-Dxd (trastuzumab-GGFG-Dxd, DAR=8), 1-G07-GGFG-Dxd (DAR=3.9), and 1-G07-LP5 (DAR=3.4) were added separately. After 6 days, the culture medium was discarded, and MTS reagent (purchased from Promega, Cat# G3581) was added to measure the cell viability (OD490nm) of each group. The results are shown in **Figure 14A** (NCI-N87) and **Figure 14B** (HCC1954). In the NCI-N87 cell assay, the IC₅₀ values for 1-G07-LP5 and 1-G07-GGFG-Dxd were 1.064 µg/mL and ~10 µg/mL, respectively. In the HCC1954 cell assay, the IC₅₀ values were 0.58 µg/mL and 1.39 µg/mL, respectively, indicating that the *in vitro* anti-tumor activity of 1-G07-LP5 is higher than that of 1-G07-GGFG-Dxd.

Referring to the *in vivo* efficacy assay method in **Example 11,** 200 µL of a suspension containing 5×10⁶ NCI-N87 cells in PBS and Matrigel (PBS:standard concentration Matrigel = 1:1) was inoculated subcutaneously into the flank of female nude mice (Balb/c nude, 5-6 weeks old). When the tumor volume reached approximately 400 mm³ (day 16), the mice were randomly grouped (n=8) based on tumor volume and body weight. Doses of 5 mg/kg T-Dxd (DAR=8) and 5 mg/kg 1-G07-LP5 (DAR=3.4) were administered via tail vein injection for a total of two doses (on day 16 and day 23), with hIgG1-vc-MMAE set as the negative control. The results are shown in **Figure 15****.** Compared to the control group, 5 mg/kg T-Dxd (DAR=8) inhibited tumor growth, while 5 mg/kg 1-G07-LP5 (DAR=3.4) lead to significant tumor shrinkage. The difference in efficacy between the two groups was statistically significant (p < 0.05).

### Example 13 Therapeutic Effects of HER2-NDC 1-G07-LP5 and TF-NDC FD40-LP5 in Nude Mouse Intracranial Tumor Model

An NCI-N87 intracranial model was established to evaluate 1-G07-LP5. NCI-N87-luc cells were resuspended in PBS and adjusted to a concentration of 1×10⁸/mL. A microsyringe was used to draw 5 µL of the cell suspension (5×10⁵ cells) for inoculation. Female Balb/c nude mice aged 6-7 weeks were anesthetized with Avertin and fixed on a brain stereotaxic instrument. Using the mouse's bregma as the center, the microsyringe needle was moved 2 mm to the right and 0.6 mm upward. After drilling through the skull with a triangular needle, the needle was inserted 4 mm downward, then withdrawn 1 mm, and the tumor cells were injected at a constant speed. The burr hole was sealed with bone wax, and finally the scalp was sutured. 9 days later, D-luciferin (150 mg/kg) was injected intraperitoneally, and fluorescence images were acquired using a small animal fluorescence/CT *in vivo* imaging system to quantify the brain fluorescence signal intensity. Grouping was based on fluorescence signal intensity (Radiance (p/sec/cm²/sr)) and mouse body weight. Vehicle control, 5 mg/kg 1-G07-LP5, and 5 mg/kg T-Dxd groups were set up. Drugs were administered via tail vein injection, with a second dose given one week later, for a total of 2 doses. Fluorescence images were acquired every 1-2 weeks using the small animal fluorescence/CT *in vivo* imaging system to monitor brain fluorescence signal intensity (Radiance (p/sec/cm²/sr)) and mouse body weight, and tumor growth curves were plotted. The results are shown in **Figure 16****.** Compared to the vehicle group, the T-Dxd group completely inhibited intracranial tumor growth, while the 1-G07-LP5 group led to almost complete tumor regression, with a therapeutic effect significantly superior to T-Dxd.

An HCC1806 intracranial model was established to evaluate FD40-LP5. Following the modeling and experimental methods described above, 5 µL of HCC1806-luc cells (5×10⁵ cells) were injected intracranially at a constant speed using a microsyringe. 7 days later, the mice were randomly grouped (n=4) based on fluorescence signal intensity and body weight. The hIgG1-MMAE group and the 5 mg/kg FD40-LP5 (DAR=3.9) group were set up. Drugs were administered via tail vein injection, with a single dose. Fluorescence images were acquired weekly using the small animal fluorescence/CT *in vivo* imaging system to monitor brain fluorescence signal intensity and mouse body weight. The results are shown in **Figure 17****.** Compared to the hIgG1-MMAE group, 5 mg/kg FD40-LP5 completely inhibited tumor growth or caused partial regression.

### Example 14 Pharmacokinetic Detection of HER2-NDC and TF-NDC in Mice

Serum Sample Collection: HER2-NDC 1 mg/kg 1-G07-GGFG-Dxd and 1 mg/kg 1-G07-LP5 were administered via tail vein injection to 8-week-old female Balb/c mice. Approximately 100 µL of blood was collected from the orbit at time points 0 min, 5 min, 30 min, 4 h, 8 h, 24 h, 48 h, 72 h, 96 h, 120 h, 168 h, and 192 h post-adminsteration. The blood was left at room temperature for 30 min, then placed at 4°C for 3-4 h, and centrifuged at 1500 rpm for 15 min to collect the upper serum. ELISA detection of antibody conjugate concentration: The anti-Dxd antibody (Abmax Biotechnology, Cat#05-0191-L, whose affinity for LP5 is similar to that for Dxd) was diluted to 2.5 µg/mL with coating buffer and coated onto ELISA plates, 100 µL/well. The plates were sealed and incubated at 4°C overnight. Unbound antigen was removed, and blocking buffer (3% BSA in PBS) was added, 200 µL/well, for blocking at room temperature for 2 h. After the blocking solution was removed, the 3-fold serially diluted test serum samples and 3-fold serially diluted standard samples (starting from 333.33 ng/mL) were added, 100 µL/well, and incubated at room temperature for 2 h. Unbound antibodies were removed, and HRP-labeled secondary antibody diluted 1:8000 was added, 100 µL/well, and incubated at room temperature for 1 h. After removing unbound secondary antibody, TMB substrate solution was added, 150 µL/well, for color development at room temperature in the dark for about 5 min. ELISA stop solution was added, 50 µL/well, to terminate the color reaction. The OD value at 450 nm was measured, and the drug concentration in the serum was analyzed and calculated. The drug concentrations in the serum at different time points were input into Phoenix software, and a non-compartmental model was used to analyze the drug concentration-time related parameters. The results are shown in **Figure 18****.** After 1 mg/kg intravenous administration, the mean Cₘₐₓ for 1-G07-GGFG and 1-G07-LP5 were 18.6 µg/mL and 17.5 µg/mL, respectively; the mean T_{1/2} were 34.6 h and 35.9 h, respectively; the mean AUC₀₋ₜ were 380.5 h*µg/mL and 456.3 h*µg/mL, respectively; and the mean MRTlast were 33.9 h and 43.1 h, respectively. These results indicate that LP5 has improved stability in the systemic circulation compared to GGFG-Dxd.

Similarly, TF-NDC 1 mg/kg FD40-GGFG-Dxd and 1 mg/kg FD40-LP5 were administered via tail vein injection to 8-week-old female Balb/c mice. Approximately 100 µL of blood was collected from the orbit at time points 0 min, 5 min, 30 min, 4 h, 8 h, 24 h, 48 h, 72 h, 96 h, 120 h, 168 h, and 192 h post-administeration, processed for detection and analysis. The results are shown in **Figure 19****.** After 1 mg/kg intravenous administration, the mean Cₘₐₓ for FD40-GGFG-Dxd and FD40-LP5 were 16.5 µg/mL and 16.75 µg/mL, respectively; the mean T_{1/2} were 45.56 h and 52.29 h, respectively; the mean AUC₀₋ₜ were 272.88 h*µg/mL and 531.75 h*µg/mL, respectively; and the mean MRTlast were 34.92 h and 49.12 h, respectively. These results indicate that LP5 has significantly improved stability in the systemic circulation compared to GGFG-Dxd.

### Example 15 Permeability of TF-NDC FD40-LP5 and TF-ADC in an In Vitro Blood-Brain Barrier (BBB) Model

The cells used in this example were sourced from Wuhan Procell Life Science & Technology Co., Ltd., and cultured according to the corresponding instructions, including C8-D1A and b.End3 cells. Establishment of the *in vitro* BBB model: 6.5 mm diameter, 3 µm pore size transwell inserts (6.5 mm, Corning, Cat#3415) were coated with 100 µg/mL rat tail type I collagen at 37°C for 1 h. Mouse brain astrocyte cells C8-D1A were resuspended in DMEM/F12 complete medium. A 24-well plate and the inserts were inverted, and 50 µL of the C8-D1A cell suspension was added to the bottom of each insert, with a final cell density of 1×10⁵ cells/cm². The bottom of the 24-well plate was used as a lid to cover the inserts, then placed in an incubator for 3 h. The inserts and the 24-well plate were righted, the medium was replenished, and incubation continued for 48 h. Mouse microvascular endothelial cells b.End3 were resuspended using DMEM/F12 complete medium. 100 µL of the b.End3 cell suspension was added to the upper chamber of the inserts, with a final cell density of 2×10⁵ cells/cm². After incubation for 5 h, the medium was replenished, and incubation continued for 96 h. 0.2% crystal violet stain was used to confirm that the inserts were fully covered with cells.

Permeability Assay: The culture medium was removed, and 700 µL of fresh medium was added below the inserts. Different drugs at 100 µg/mL were added to the upper chamber of the inserts, which were then placed in the incubator. At 6 h and 24 h, 120 µL of medium from below the inserts was collected and promptly replaced with 120 µL of fresh medium. For TF-NDC and TF-ADC drugs, antibody concentrations were detected by ELISA.

The results are shown in **Figure 20****.** At both the 6h and 24h time points, the BBB penetration rate of FD40-LP5 was approximately 2 times that of the TF-ADC HuSC1-39-MMAE.

### Example 16: Preparation and Stability Testing of FD40-LP5 Conjugate with Scaled-up Batch

Based on **Example 7,** scaled-up batch preparation of FD40-LP5 was performed by adjusting and optimizing the conjugation reaction and conjugate purification conditions. 860 mg of FD40 antibody was used for the conjugation reaction, the TECP/antibody molar ratio was 2.8, the LP5/antibody molar ratio was 7.0, the organic solvent DMA was 10%, the reduction condition was 22°C for 18 h, and the conjugation condition was 22°C for 0.5 h. The overall conjugate yield was 81%, DAR=4.0, monomer ratio was 97.39%, and residual small molecule ratio was <0.06%. **Figure 21** shows the SEC and LC-MS detection data for the scaled-up batch of the FD40-LP5 conjugate.

This batch of FD40-LP5 conjugate was used for freeze-thaw stability and thermal stability tests. The results in **Table 4** show that the FD40-LP5 conjugate has excellent freeze-thaw stability and excellent thermal stability.

**Table 4: Stability Test Results of FD40-LPS Conjugate**

| Sample ID | BCA Conc. (mg/ml) | Reduced MS-DAR | Monomer (%) | Appearance |
|---|---|---|---|---|
| FD40-FD-LP5-0x freeze thaw | 11. 14 | 4 | 97. 39 | Clear liquid |
| FD40-FD-LP5-1x freeze thaw | 11. 54 | 4. 03 | 96. 8 | Clear liquid |
| FD40-FD-LP5-3x freeze thaw | 11. 5 | 4. 03 | 96. 7 | Clear liquid |
| FD40-FD-LP5-5x freeze thaw | 11. 49 | 4. 03 | 96. 94 | Clear liquid |

| Sample ID | BCA Conc. (mg/ml) | Reduced MS-DAR | Monomer (%) | Appearance |
|---|---|---|---|---|
| FD40-FD-LP5-0 week | 11. 14 | 4. 02 | 96. 81 | Clear liquid |
| FD40-FD-LP5-25 °C 1 week | 10. 61 | 4 | 96. 79 | Clear liquid |
| FD40-FD-LP5-25 °C 2 weeks | 11. 42 | 3. 99 | 96. 67 | Clear liquid |

### Example 17 Exploratory Safety Assessment of FD40-LP5 in Cynomolgus Monkeys

Two cynomolgus monkeys (one male and one female) were administered a single intravenous infusion of 10 mg/kg FD40-LP5, followed by continuous observation for 21 days. On day 22, a second intravenous infusion of 30 mg/kg FD40-LP5 was administered, followed by another 21 days of continuous observation (total 42 days). The results showed no significant drug-related changes in the clinical status of the animals at either dose level. Slight and reversible fluctuations were observed in feeding and body weight indicators.

As shown in **Figure 22****,** during the experiment period, especially at the high dose, the animals showed slight and reversible body weight loss (5-10%).

As shown in **Figure 23****,** no significant drug-related changes were observed in the blood coagulation parameters of the animals at either dose level.

As shown in **Figure 24****,** no significant drug-related changes were observed in the blood biochemical parameters of the animals at either dose level.

As shown in **Figure 25****,** no significant drug-related changes were observed in the hematological parameters of the animals at either dose level.

### Example 18 Toxicokinetic (TK) Detection of FD40-GGFG-Dxd and FD40-LP5 in Cynomolgus Monkeys

K2477 experiment: 2 cynomolgus monkeys (one male and one female) were administered a single intravenous infusion of 10 mg/kg FD40-GGFG-Dxd (designated as the 10 mg/kg dose group). On day 22, a second intravenous infusion of 30 mg/kg FD40-GGFG-Dxd was administered (designated as the 30 mg/kg dose group). Blood was collected from the forelimb or hindlimb veins before each dose and at 5 min, 1 h, 8 h, 24 h, 48 h, 72 h, 96 h, 120 h, 168 h, 240 h, 336 h, and 504 h after each dose to prepare serum. Pharmacokinetic detection was performed using the method described in **Example 14.**

K2504 experiment: 2 cynomolgus monkeys (one male and one female) were administered a single intravenous infusion of 10 mg/kg FD40-LP5 (designated as the 10 mg/kg dose group). On day 22, a second intravenous infusion of 30 mg/kg FD40-LP5 was administered (designated as the 30 mg/kg dose group). Blood was collected from the forelimb or hindlimb veins before each dose and at 5 min, 1 h, 8 h, 24 h, 48 h, 72 h, 96 h, 120 h, 168 h, 240 h, 336 h, and 504 h after each dose to prepare serum.

Pharmacokinetic detection was performed using the method described in **Example 14.** The results are shown in **Figure 26****.** After the 10 mg/kg intravenous administration, the mean AUC₀₋ₜ for FD40-GGFG-Dxd and FD40-LP5 were 4725.19 h*µg/mL and 7527.15 h*µg/mL, respectively. After the 30 mg/kg intravenous administration, the mean AUC₀₋ₜ were 14822.3 h*µg/mL and 24615.3 h*µg/mL, respectively. These results indicate that LP5 has significantly improved stability in the systemic circulation of cynomolgus monkeys compared to GGFG-Dxd.

In summary, the experiments in the relevant examples clearly demonstrate:
1. TF-STING ADC/NDCs prepared with the novel linker A of the present invention exert excellent anti-tumor effects in triple-negative breast cancer and pancreatic cancer models by activating the STING signaling pathway, promoting tumor-immune cell interactions, and inducing the secretion of CXCL10 and IFNγ.
2. TF-KRASG12D-I ADC/NDCs prepared with the novel linker A of the present invention have good anti-tumor effects in pancreatic cancer models with KRASG12D mutation.
3. TF-Topo1 inhibitor NDCs prepared with the novel linker A of the present invention have good anti-tumor effects in lung cancer models with KRASG12C mutation, pancreatic cancer models with KRASG12D mutation, and triple-negative breast cancer models.
4. HER2-Topo1 inhibitor NDCs prepared with the novel linker A of the present invention have good anti-tumor effects in gastric cancer models and are superior to trastuzumab deruxtecan (T-Dxd).
5. In intracranial tumor models, TF-NDCs and HER2-NDCs prepared with the novel linker A have good BBB penetration and exhibit good anti-tumor effects against intracranial tumors, superior to trastuzumab deruxtecan (T-Dxd).
6. In head-to-head comparative mouse pharmacokinetic experiments, HER2-NDCs and TF-NDCs prepared with the novel linker A have higher in vivo half-life and plasma exposure compared to those prepared with the known linker (GGFG-Dxd). Similarly, in the toxicokinetic (TK) detection in cynomolgus monkeys, the mean plasma exposure of FD40-LP5 was significantly higher than that of FD40-GGFG-Dxd.
7. The novel linker A of the present invention has broad applicability and is suitable for conjugating antibodies and nanobodies targeting different antigens, as well as payload compounds with different mechanisms of action.
8. The conjugation and purification technology for preparing NDCs/ADCs with the novel linker A of the present invention is stable and conducive to industrial-scale processes.
9. FD40-LP5 prepared with the novel linker A of the present invention has excellent druggability, showing outstanding water solubility, homogeneity, freeze-thaw stability, and thermal stability.
10. FD40-LP5 prepared with the novel linker A of the present invention showed good safety in the exploratory toxicological assessment study in cynomolgus monkeys.

**The sequences of TF nanobody, HER2 nanobody, TF monoclonal antibody of the present invention**

| | | |
|---|---|---|
| SEQ ID NO.1: | 4-A02 CDR1 | ETISSTYI |
| SEQ ID NO.2: | 4-A02 CDR2 | ISGDGVTH |
| SEQ ID NO.3: | 4-A02 CDR3 | YAAGRWNH |
| SEQ ID NO.4: | 4A02 VHH | |
| QVQLVESGGGLVQPGGSLSLSCTASETISSTYIMGWHRRSPGKERELVAVISGDGVTHYAD FVKGRFVISRDNAKNAVYLKMNFLTPEDTAVYYCYAAGRWNHWGQGTQVTVSS | | |
| SEQ ID NO.5: | 4A02-FCWT | |
| QVQLVESGGGLVQPGGSLSLSCTASETISSTYIMGWHRRSPGKERELVAVISGDGVTHYAD FVKGRFVISRDNAKNAVYLKMNFLTPEDTAVYYCYAAGRWNHWGQGTQVTVSSEPKSSDKTH TCPPCPAPELLGGPSVFLFPPKPKDTLMISRTPEVTCVVVDVSHEDPEVKFNWYVDGVEVHNAK TKPREEQYNSTYRVVSVLTVLHQDWLNGKEYKCKVSNKALPAPIEKTISKAKGQPREPQVYTLP PSREEMTKNQVSLTCLVKGFYPSDIAVEWESNGQPENNYKTTPPVLDSDGSFFLYSKLTVDKSR WQQGNVFSCSVMHEALHNHYTQKSLSLSPGK | | |
| SEQ ID NO.6: | 4A02-HM8-FCWT | |
| QVQLVESGGGLVKPGGSLRLSCTASETISSTYIMGWHRQAPGKGRELVAVISGDGVTHYA DFVKGRFTISRDNAKNTVYLQMNFLRAEDTAVYYCYAAGRWNHWGQGTMVTVSSEPKSSDK THTCPPCPAPELLGGPSVFLFPPKPKDTLMISRTPEVTCVVVDVSHEDPEVKFNWYVDGVEVHN AKTKPREEQYNSTYRVVSVLTVLHQDWLNGKEYKCKVSNKALPAPIEKTISKAKGQPREPQVY TLPPSREEMTKNQVSLTCLVKGFYPSDIAVEWESNGQPENNYKTTPPVLDSDGSFFLYSKLTVD KSRWQQGNVFSCSVMHEALHNHYTQKSLSLSPGK | | |
| SEQ ID NO.7: | 1-G07-FCWT | |
| QVQLVESGGGKAQPGGSLRLSCVASRITFRHYDLSWYRQAPGQERELVATVTNGGVITYA DSVKGRFTISRDNAKNTVQLQMNNLKPEDTAVYYCNAVWVYLQRLTQNKKENDYWGPGTQV TVSSEPKSSDKTHTCPPCPAPELLGGPSVFLFPPKPKDTLMISRTPEVTCVVVDVSHEDPEVKFN WYVDGVEVHNAKTKPREEQYNSTYRVVSVLTVLHQDWLNGKEYKCKVSNKALPAPIEKTISK | | |
| AKGQPREPQVYTLPPSREEMTKNQVSLTCLVKGFYPSDIAVEWESNGQPENNYKTTPPVLDSDG SFFLYSKLTVDKSRWQQGNVFSCSVMHEALHNHYTQKSLSLSPGK | | |
| SEQ ID NO.8: | 1-G07-HM1-FCWT | |
| QVQLVESGGGLVQPGGSLRLSCSASRITFRHYDLSWYRQAPGKGRELVATVTNGGVITY ADSVKGRFTISRDNAKNTVYLQMNNLRAEDTAVYYCNAVWVYLQRLTQNKKENDYWGQGTL VTVSSEPKSSDKTHTCPPCPAPELLGGPSVFLFPPKPKDTLMISRTPEVTCVVVDVSHEDPEVKFN WYVDGVEVHNAKTKPREEQYNSTYRVVSVLTVLHQDWLNGKEYKCKVSNKALPAPIEKTISK AKGQPREPQVYTLPPSREEMTKNQVSLTCLVKGFYPSDIAVEWESNGQPENNYKTTPPVLDSDG SFFLYSKLTVDKSRWQQGNVFSCSVMHEALHNHYTQKSLSLSPGK | | |
| SEQ ID NO.9: | 1-G07-HM3-FCWT | |
| QVQLVESGGGLVKPGGSLRLSCAASRITFRHYDLSWYRQAPGKGRELVATVTNGGVITY ADSVKGRFTISRDNAKNTVYLQMNNLRAEDTAVYYCNAVWVYLQRLTQNKKENDYWGQGTL VTVSSEPKSSDKTHTCPPCPAPELLGGPSVFLFPPKPKDTLMISRTPEVTCVVVDVSHEDPEVKFN WYVDGVEVHNAKTKPREEQYNSTYRVVSVLTVLHQDWLNGKEYKCKVSNKALPAPIEKTISK AKGQPREPQVYTLPPSREEMTKNQVSLTCLVKGFYPSDIAVEWESNGQPENNYKTTPPVLDSDG SFFLYSKLTVDKSRWQQGNVFSCSVMHEALHNHYTQKSLSLSPGK | | |
| SEQ ID NO.10: | HuSC1-39 VH | |
| EVQLVQSGAEVKKPGESLKISCKGSGYSFTSYWMNWVRQMPGKGLEWMGMIYPADSET RLNQKFKDQATLSVDKSISTAYLQWSSLKASDTAMYYCAREDYGSSDYWGQGTTVTVSS | | |
| SEQ ID NO.11: | HuSC1-39 VL | |
| DIQLTQSPSSLSASVGDRVTITCSASSSVSYMNWYQQKPGKSPKIWIYGISNLASGVPSRFS GSGSGTDFTLTISSLQPEDFATYYCQQKSSFPWTFGGGTKVEIK | | |

All literatures mentioned in the present application are incorporated herein by reference, as each one is individually incorporated by reference. In addition, it should also be understood that, after reading the above teachings of the present invention, those skilled in the art can make various changes or modifications, and these equivalents also fall in the scope of claims as defined in the appended claims.

## Claims

1. A compound, or stereoisomer thereof, or pharmaceutically acceptable salt thereof, which has a structure as shown in Formula A: 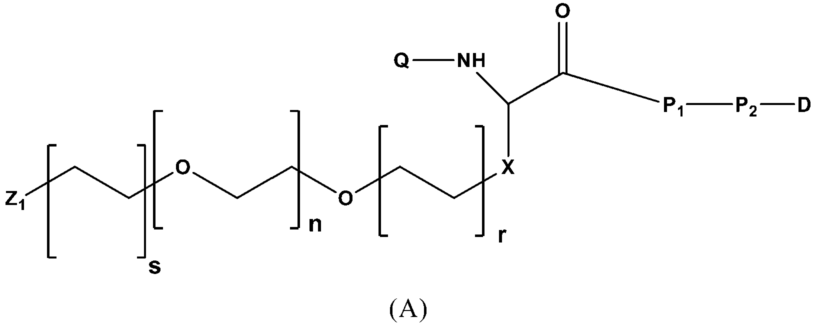 wherein,
Q is a linker group for connecting to an antibody;
Z1 comprises a glucosyl group containing Y hydroxyl groups;
s is an integer from 0 to 10;
n is an integer from 1 to 24;
r is an integer from 0 to 10;
X is a linking group;
P1 is a polypeptide residue;
P2 is a chemical bond or AA-PAB structure; wherein AA is a dipeptide, tripeptide, or tetrapeptide fragment (i.e. a fragment formed by 2-4 amino acids linked through peptide bonds), and PAB is p-aminobenzylcarbamoyl;
D is drug.

2. The compound or stereoisomer thereof or pharmaceutically acceptable salt thereof according to claim 1, wherein the linker group Q is selected from:
| | | |
|---|---|---|
| 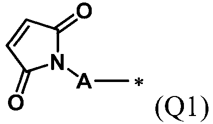 | 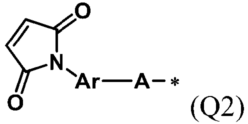 | 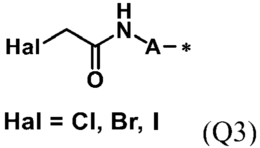 |
| 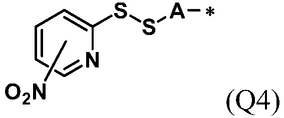 wherein NO2 is optionally substituted | 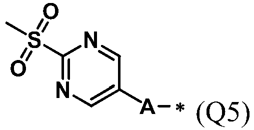 | 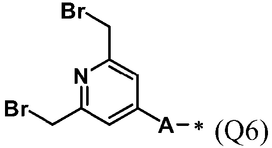 |
| 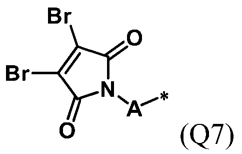 | 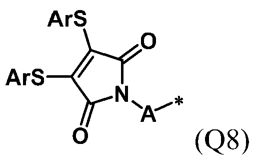 | 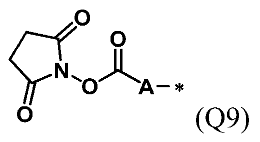 |
wherein A represents an optionally substituted C3-C8 alkylene, C3-C8 alkene, C3-C8 alkynyl, C3-C6 cycloalkene, C3-C8 cycloalkyl, and optionally substituted diglycolyl to octaglycolyl group; Ar represents an optionally substituted C5-6 aryl or heteroaryl group; and "*" represents -C=O- forming an amide bond with an amino group.

3. The compound or stereoisomer thereof or pharmaceutically acceptable salt thereof according to claim 1, wherein P1 is selected from the group consisting of:
^{NH} -Val-Cit-^{C=O}, ^{NH}-Val-Ala-^{C=O}, ^{NH}-Ala-Ala-Ala-^{C=O}, ^{NH}-Ala-Ala-^{C=O}, ^{NH}-Gly-Gly-Phe-Gly-^{C=O}, ^{NH} -Val-Lys-^{C=O}.

4. The compound or stereoisomer thereof or pharmaceutically acceptable salt thereof according to claim 1, wherein X is selected from

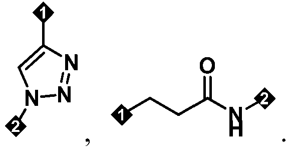

5. The compound or stereoisomer thereof or pharmaceutically acceptable salt thereof according to claim 1, wherein the structure of Z1 is selected from the group consisting of:

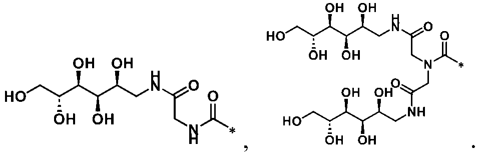

6. The compound or stereoisomer thereof or pharmaceutically acceptable salt thereof according to claim 1, wherein D is a cytotoxic small molecule drug selected from the group consisting of: a STING agonist, KRAS-G12D inhibitor, tubulin inhibitor, topoisomerase inhibitor, and DNA binding agent.

7. The compound or stereoisomer thereof or pharmaceutically acceptable salt thereof according to claim 1, wherein the structure of Formula A is selected from the group consisting of:
| | |
|---|---|
| 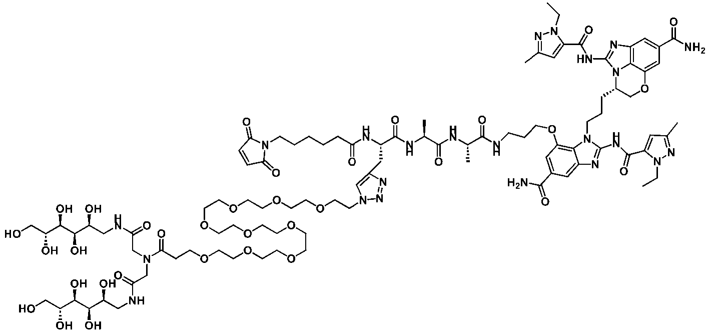 | A1 |
| 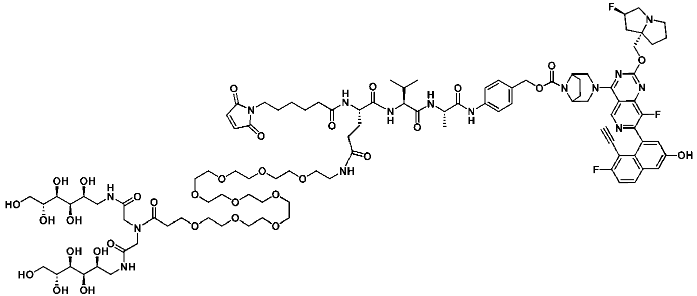 | A2 |
| 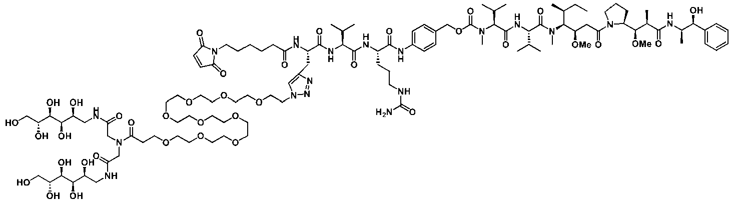 | A3 |
| 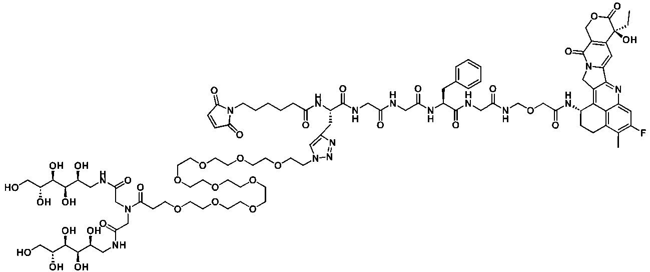 | A4 |
| 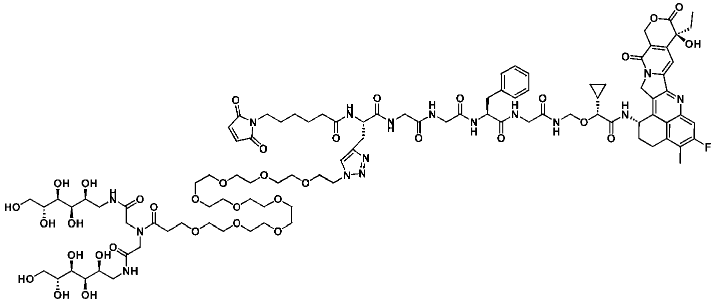 | A5 |
| 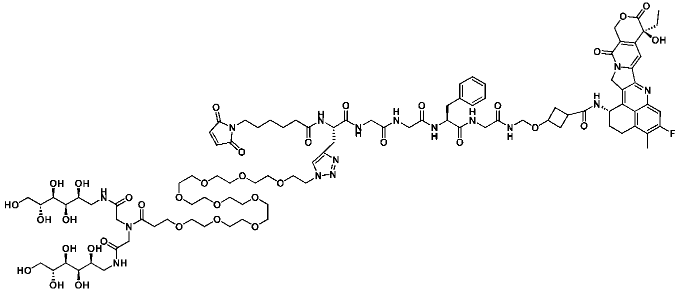 | A6 |
| 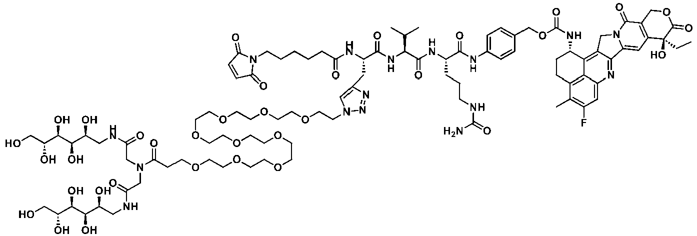 | A7 |
| 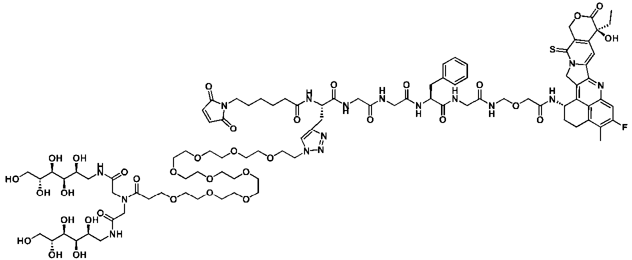 | A8 |
| 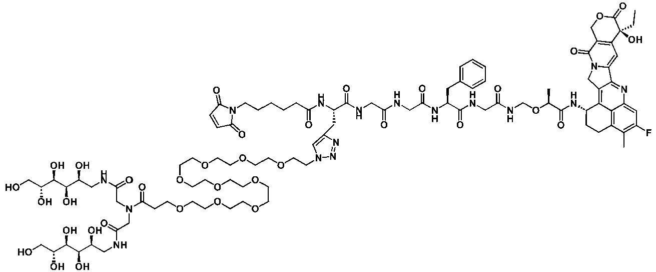 | A9 |
| 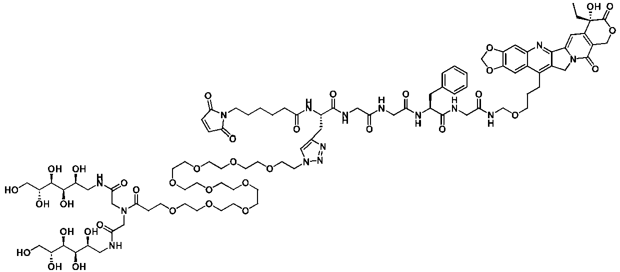 | A10 |

8. An antibody-drug conjugate (ADC), which is formed by conjugating the compound of Formula A according to claim 1 with an antibody.

9. The antibody-drug conjugate according to claim 8, wherein the conjugate is shown as Formula B: 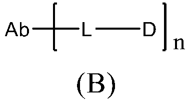 wherein:
Ab is an antibody;
L is a connector;
D is a drug;
n is an integer or decimal from 1 to 10.

10. The antibody-drug conjugate according to claim 8, wherein the antibody comprises an antigen binding fragment, a nanobody, a chimeric antibody, a bivalent antibody, and/or a multivalent antibody.

11. The antibody-drug conjugate according to claim 8, wherein the antibody or nanobody or fusion protein thereof targets a target selected from the group consisting of: TF, HER2, EGFR, HER3, BCMA, B7-H3, CD73, AXL, DLL3, CD38, CD123, CD19, CD20, CD22, B7-H6, GPC3, PMSA, CD28, 4-1BB, OX40, CD40, CD27, CD3, CTLA4, PD1, PDL1, BCMA, Trop2, TIGIT, LAG-3, TLR7, or a combination thereof.

12. The antibody-drug conjugate according to claim 8, wherein the antibody is an antibody or nanobody or fusion protein thereof targeting TF.

13. The antibody-drug conjugate according to claim 12, wherein the antigen binding fragment of the antibody or nanobody targeting TF has CDR1 as shown in SEQ ID NO. 1, CDR2 as shown in SEQ ID NO. 2, and CDR3 as shown in SEQ ID NO. 3.

14. An antibody-drug conjugate, which comprises the structure shown in formula (B): 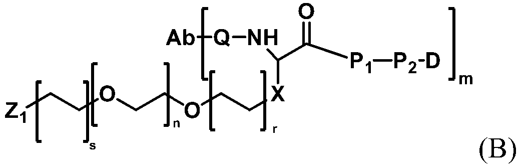 wherein: Q is a linker group the nanobody targeting HER2; Z1 is a hydrophilic group, including a hydroxyl and a glucosyl group containing an amino group; X is a linking group; P1 is a polypeptide residue; P2 is a direct bond or a p-aminobenzyloxycarbonyl (PABC) group; D is an anti-tumor drug; n is an integer from 1 to 24; Ab is an antibody or nanobody fusion protein; m=1-8.

15. A pharmaceutical composition, comprising (a) the antibody-drug conjugate or pharmaceutically acceptable salt thereof according to claim 8 or 14, and (b) a pharmaceutically acceptable carrier or excipient.

16. Use of the antibody-drug conjugate or pharmaceutically acceptable salt thereof according to claim 8 or 14, or the pharmaceutical composition comprising the conjugate or pharmaceutically acceptable salt thereof, in the manufacture of a medicament for treating a tumor or cancer.

17. The use according to claim 16, wherein the cancer is selected from the group consisting of: lung cancer, liver cancer, breast cancer (triple-negative breast cancer), ovarian cancer, non-Hodgkin lymphoma, Hodgkin lymphoma, acute lymphoblastic leukemia, anaplastic large cell lymphoma, multiple myeloma, prostate cancer, non-small cell lung cancer, small cell lung cancer, malignant melanoma, squamous cell cancer, glioblastoma, renal cell cancer, gastrointestinal cancer, pancreatic cancer, colorectal cancer, gastric cancer, glioma, mesothelioma.

18. A method for preparing the antibody-drug conjugate according to claim 8 or 14, which comprises the steps of:
(1) reacting an antibody with a reducing reagent in a buffer to obtain a reduced antibody;
(2) crosslinking (conjugating) the compound shown in Formula A with the reduced antibody obtained in step (1) in a mixture of buffer and organic solvent to obtain the antibody-drug conjugate B.
